(19) **Europäisches Patentamt / European Patent Office / Office européen des brevets**

(11) **EP 1 763 374 B1**

(12) **EUROPEAN PATENT SPECIFICATION**

(45) Date of publication and mention of the grant of the patent:
**20.06.2012 Bulletin 2012/25**

(21) Application number: **05751734.4**

(22) Date of filing: **09.06.2005**

(51) Int Cl.:
*A61F 2/28* (2006.01)     *A61L 27/12* (2006.01)
*A61L 27/46* (2006.01)     *A61L 27/54* (2006.01)
*A61L 27/56* (2006.01)     *A61L 27/50* (2006.01)
*A61L 27/58* (2006.01)     *A61L 27/44* (2006.01)
*A61K 6/00* (2006.01)     *A61K 9/00* (2006.01)
*A61L 27/48* (2006.01)

(86) International application number:
**PCT/EP2005/006204**

(87) International publication number:
**WO 2005/120595 (22.12.2005 Gazette 2005/51)**

(54) **IN SITU HARDENING PASTE, ITS MANUFACTURING AND USE**

IN SITU HÄRTENDE PASTE, IHRE HERSTELLUNG UND VERWENDUNG

PATE A DURCISSEMENT IN SITU, SA FABRICATION ET SON UTILISATION

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV YU**

(30) Priority: **09.06.2004 EP 04013668**

(43) Date of publication of application:
**21.03.2007 Bulletin 2007/12**

(60) Divisional application:
**10012828.9 / 2 322 234**

(73) Proprietor: **Scil Technology GmbH**
**85152 Martinsried (DE)**

(72) Inventors:
• **HELLERBRAND, Klaus**
**82269 Geltendorf (DE)**

• **SIEDLER, Michael**
**80538 München (DE)**
• **SCHÜTZ, Andreas**
**82131 Stockdorf (DE)**
• **POMPE, Cornelius**
**80636 München (DE)**
• **FRIESS, Wolfgang**
**82393 Iffeldorf (DE)**

(74) Representative: **Grünecker, Kinkeldey, Stockmair & Schwanhäusser**
**Leopoldstrasse 4**
**80802 München (DE)**

(56) References cited:
**EP-A- 0 470 305**     **WO-A-96/10374**
**WO-A-2004/073563**     **US-A1- 2003 158 302**
**US-A1- 2004 034 434**     **US-B1- 6 277 151**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

An *in situ* hardening paste, containing a water soluble or miscible plasticizer which is an organic solvent, an organic or inorganic water insoluble filling material, and a water-insoluble polymer was developed as a stable injectable and moldable formulation which exhibits - once hardened *in situ* - scaffold properties. The paste in addition can be used as a delivery system for an active agent in the filed of tissue regeneration.

The addition of a water soluble pore building filler as an anti-skin forming agent increases the formation of pores with pore sizes of diameters sufficient for cell infiltration.

The hardened paste is sufficient mechanical stable to be used as bone and cartilage replacement matrix as well as regeneration of ligament, tension or treatment of periodontal diseases. All components are full biocompatible, preferably bioresorbable and certified for parenteral application. The sustained release of peptides and proteins can be modulated by the composition and process design.

The invention encompasses a pharmaceutical composition comprising the paste of the invention and relates to the use of said paste for the preparation of a pharmaceutical composition to be used for bone augmentation, for treating bone defects, for treating degenerative and traumatic disc disease, for treating bone dehiscence or to be used for sinus floor elevation, ligament and tension regeneration including periodontal repair. Finally, the invention relates to a kit comprising the paste of the invention.

Background Technology

In the field of medicinal technology many different materials have already been evaluated and/or are still in the process of being further improved for use as a bone replacement material (bone graft), because of the wide range of requirements the materials should fulfill. Dependent on the indication an ideal bone graft substitute should have the following properties, initially moldable and easy to shape and administer, but mechanically stable over time similar to endogenous bone for bridging bone defects, filling cavities or bone augmentation, preferably, the material should be capable for *in situ* hardening to be applicable by minimal invasive applications. The material must be biocompatible, preferably biodegradable and bioresorbable and promote cell adhesion and proliferation. It should have an interconnected porosity to allow cell ingrowth to allow a binding to the surrounding bone tissue (osteoconductivity). Furthermore the material should be capable to act as a carrier for bone growth factors (BMPs) for the controlled release of these proteins to introduce bone formation (osteoinductivity). Ideally the protein within the material is protected against washout and proteolytic degradation at the site of implantation. Finally the material should be of synthetic origin to avoid infections and immunological reactions, should be available in access and of reproducible quality. Ideally synthetic bone substitute materials should be clearly visible on radiographic examinations to survey the healing process and determine the amount and mass of new bone formation.

Nevertheless up to now there exists no material, which is capable to fulfill all of these requirements of a preferable material.

Due to the medical need for artificial bone grafts and to the limited availability of autologous bone, different materials are commonly in use. The most prominent are calcium phosphates and bioresorbable polymers of the PLGA type.

**Calcium phosphates based material**

Various calcium phosphates such as beta-tricalcium phosphate ($Ca_3(PO_4)_2$) (beta-TCP), alpha-tricalcium phosphate (alpha-TCP) and hydroxyapatite ($Ca_{10}(PO_4)_6(OH)_2$) (HA) have been shown to be effective as bone replacement materials. Beta-TCP, for example, is suitable both as various granulates and in pieces (blocks) for the treatment of bone defects. The bone replacements materials containing calcium phosphate are usually used when the regeneration of the bone is not possible any more or is possible with difficulties only. In addition, bone replacement materials are used when the formation of additional bone is a prerequisite for a subsequent setting of an implant. The calcium phosphates exhibit an osteoconductive effect, i.e. they represent an inert structure facilitating the migration of cells from the neighboring bone. The presence of bones or different mesenchymal cells, however, is a precondition for the new formation of bones.

Adding autologous bone chips can significantly increase the affect of calcium phosphates. These bones chips are not only osteoconductive, but also osteoinductive, i.e. they cause the transformation of undifferentiated mesenchymal stem cells into osteoblasts and chondrocytes. For reasons of safety, autogenic bone chips are preferred to the allogenic or xenogenic preparations. The use of autogenic bones, however, always involves a second surgical procedure, which is uncomfortable for the patient and is limited in access. In - addition, biopsies of autologous bonegraft material have several- disadvantages including post surgery pain and graft harvest complications.

Beside the above-mentioned solid blocks and granules, calcium phosphate cements (CPC) represent a further huge class of calcium phosphate based materials. They are delivered via injection or scraper into the bone defect as a moldable paste, can be adapted to the defect site and become solid after a period of time (Driessens et al., 2002).

One of the first self-hardening CPC consists of tetracalcium phosphate (TTCP) and dicalcium phosphate anhydrous (DCPA) (Chow et al., 2000). These components react in the presence of water to form hydroxyapatite as the final product of the cement setting reaction. The reactions are either endothermic or exothermic. The exothermic reactions generate sufficient heat to degenerate osteogenic proteins, whereas endothermic setting reactions prevent thermal injury to surrounding tissue.

Because the hydroxyapatite is formed in an aqueous environment, it is more similar to biological apatites rather than hydroxyapatite formed by high temperature processes. As a consequence, CPC is osteoconductive and readily osteointegrates (Chemg et al., 1997).

While CPC appears to have several advantages over presently used calcium phosphate biomaterials, an apparent limitation is its relatively long hardening time coupled with the washout effect explained below (Chemg et al., 1997). In recent years, additional CPC that contains alpha-tricalcium phosphate (alpha-TCP) and calcium carbonate ($CaCO_3$), mono calcium phosphate (MCPA), mono calcium phosphate monohydrate (MCPM), dicalcium phosphate dehydrate (DCPD) or DCPA and $Ca(OH)_2$ have been developed (Takagi et al., 2003). However, most of the commercial available CPC developed in the last years have alpha-TCP as the main reactant with different particle sizes of the initial powder phase. Commercial available products include Blobon® ($\alpha$-BSM), Biocement D and H, Biofill®, Bonesource® Calcibon®, Cementek®, Mimics Biopex® and Norian® SRS® (Driessens et al., 2002).

Alpha-BSM (ETEX Corporation, Cambridge, MA) is primarily composed of two calcium phosphates, the first an amorphous calcium phosphate (ACP) with a Ca/P ratio of 1,54, the second a dicalcium phosphate dehydrate (DCPD, or brushite). The material is an injectable bone substitute that hardens at body temperature (37°C) within approximately 20 minutes after being mixed with water or a saline solution, forming a poorly crystalline hydroxyapatite phase.

Another CPC paste is Norian® SRS® (Skeletal Repair System), which is an injectable, fast hardening carbonated apatite cement used to fill defects in areas of compromised cancellous bone during restoration or augmentation of the skeleton. Norian SRS hardens to form dahlite, which closely replicates the mineral phase of bone and gradually remodels to bone In the body via osteoclastic resorption and new bone formation. This CPC needs to be premixed In a special adopted apparatus before application.

Most of these available CPC formulations are composed of two components that react and harden when mixed (Seeherman et al. 2002). The powder components are mixed with an aqueous solution some including an accelerator or promoter (e.g. disodium hydrogen phosphate, $N_{32}HPO_4$) immediately before application. This premixture procedure yields to deviations in the quality of the final product, depending on the handling prior to use e.g., inhomogeneous suspension. Therefore this premixing procedure can lead to decreased mechanical stability of the implant and therefore difficulties regarding reproducibility.

Another limitation of these formulation is if the powder is mixed with the aqueous component, the mixture starts to solidify, therefore the timeframe were the cement can be administered is limited to a few minutes only. In the clinical situation, the surgeon has to properly mix the cement and than place the cement paste in the defect within the prescribed time, which is a crucial factor in achieving optimum results.

A premixed CPC paste, which is stable in the syringe and hardens only after being exposed to water contains TTCP plus DCPA powders and glycerol as a cement liquid. However, the CPC-glycerol paste did not have a good washout resistance when it was applied to a wet opened field (Takagi et al., 2003).

This washout-effect arise when the CPC paste comes into contact with physiologic fluids or when bleeding occurs due to its difficultly in some cases to achieve hemostasis. It has also been found that such pastes often partially or completely disintegrate or exhibit inhomogeneous behavior soon as they come into contact with body fluids or other aqueous solutions (US 6,206,957 and references therein). Furthermore, such pastes readily separate during extrusion from syringes, the more liquid part being forced out of the syringe while the more solid parts remain in the syringe and cannot been removed from it, even by means of higher pressure. As a result of a separation, a material, which is no longer suitable for the intended purpose may thus be obtained.

Several attempts have been made in the past to improve the features of CPC. Chemg et al. addeted hydroxypropylmethylcellulose (HPMC), carboxymethyleellulose (CMC), and chitosan to the cement liquids (Chemg et al., 1997) to improve the handling properties of CPC with. However, hardening time was retarded by addition of HPMC and CMC, and mechanical strength was weakened by the addition of either chitosan lactate or chitosan acetate.

Other attempts have been made to improve injectability of CPC by for example an increase of the liquid-to-powder ratio, the addition of citrate ions, and the addition of polymeric drugs to the liquid component (Bohner and Baroud, 2004). However, none of these improvements resulted in a stable premixed CPC paste.

Another major problem of CPC is that they exhibit of only micropores with pore sizes of submicrometer to a few micrometers. Previous studies with hydroxyapatite implants have shown that a pore size of about 100 $\mu$m to several hundred $\mu$m (macropores) is required for bone ingrowth (del Real et al., 2002). Macropores have been shown to be beneficial in facilitating cell infiltration and tissue ingrowth. However, macroporosity always results into a significant decrease in mechanical strength (Chow. 2000).

Other groups tried to improve the resorption behaviour of CPC e.g. by increasing the porosity of the material (del Real et al., 2002). Most extensive experiments have been done by mixing the CPC with crystals of the right dimensions of highly soluble and non-toxic compounds such as mannitol or sucrose. The disadvantage is that the porosity cannot be created during hardening of the cement in the *in vivo* environment. Addition of sucrose or mannitol requires dissolution of these components after application and hardening of the cement in the bone defect in order to get macroporosity. Another method consists in adding $NaHCO_3$ to the starting cement powder and using two different liquids to create macropores (del Real et al., 2002).

An increase in the strength of CPC while forming macropores was attempted by using for example mannitol crystals or aramid fibers (Xu et al., 2001). The incorporation of chitosan, a natural bipolymer, was also found to increase the CPC strength, however, no macropores were built into the chitosan containing CPC (Takagi et al., 2003; Xu et al., 2002).

In general CPC suffer from a relatively low mechanical stability (e.g. compression strength, brittleness) and lack of macroporosity e.g., osteoconductivity, limiting its applicability in orthopaedics to only non load-bearing applications. The different cement reactions cause hydroxyapatite to form varying states of crystallinity which result in altered resorption time. Due to the lack of macroporosity and therefore osteoconductivity many of the cement formulations are poor carriers for osteogenic growth factors.

Further attempts have been made to improve CPC by adding biodegradable polymers (e.g. poly(DL-lactid-co-glycolic acid, PLGA) microparticles as delivery vehicles for bioactive molecules (Ruhe et al., 2003). Protein PLGA microparticles were added to the CPC powder and an aqueous solution of $Na_2HPO_4$ was used as a liquid, which was added to the composition shortly before application.

It is generally accepted that CPC needs further improvement to broaden their potential clinical applications. However, further improvement on material properties should keep in mind the way surgeons apply bone cements through minimal invasive surgery techniques (MIST cited in Bohner, 2001). In this sense, further material improvement should be considered at the compromise between what is considered, for a certain clinical application, the optimum cement injectability, fast-setting behavior, macroporosity and mechanical strength.

Recently these CPC systems become interesting especially in combination with bone morphogenetic proteins (Seeherman et al., 2003).

**Synthetic PLGA based material**

Another important biomaterial class, which plays a predominant role in the field of bone tissue engineering, are bioresorbable polymers (Vert, 1989). Especially the compound class of poly(hydroxy acids) has interesting application prospects due to their intrinsic biodegradability. These materials of which poly(glycolic acid) (PGA) and poly(lactic acid) (PLA) are the most prominent undergo hydrolytic chain cleavage (degradation) in a moist environment. Sustained degradation finally leads to the corresponding hydroxy acid units. Most of these hydrolytic end products occur as metabolites of many bacteria and cell phenotypes.

The degradation potential and their mechanical properties offer applications for the use as substrates for temporary implants in medical technology. Studies for various polymers in different tissues document the biocompatibility of these compounds *in vivo* forming the bases for the development of commercial implants as medical devices (Middleton et al, 2000).

In clinical surgery, polyesters presumably play the most important role in connection with the fixation, augmentation and replacement of bone. Devices like screws, plate, anchors or pins serve for positioning and fixation of bone fragments after bone loss or damage. The major feature of these absorbable polymers in application is the lacking necessity for a removal operation. Another important point is in favor of polymeric fixation devices: the mechanical integration of the implant in the bone tissue.

An important drawback in totally polyester based implants is the possible accumulation of degradation products reaching cytotoxic levels and the accompanying acidification at the implant site due to the pH lowering release of acid monomers, especially when solid none porous implants were used and the degradation proceeds according to a bulk degradation mechanism (Li et al., 1990).

To avoid such negative consequences caused by local pH decrease the implant should exhibit porosity e.g., by salt leaching process to avoid bulk degradation and therefore an accumulation of acidic monomers and to reduce the net amount of the polymer. Furthermore it has been suggested to incorporate basic salts within PLA/ PGA implants (Agrawal et al., 1997). Other approaches employ basic ceramics like calcium phosphates as fillers for polyesters to balance the local pH value and increase the mechanical stability (Schiller et al., 2003). Also composites of hydroxyapatite and PLA/PLGA (polylactide-co-glycolide) have been produced (Durucan et al., 2000). The processing of such composites requires thermal treatment and the use of chloroform solutions of the polymeric component (Ignjatovic et al., 1999). As an alternative one can use polymers with higher hydrolytic stability like poly(hydroxybutyric acid).

For the use of these polymers as bone substitutes the common strategy is to design an implant, which temporarily fulfills the function to allow a healing process and to retain strength during the early stages at the implantation site after operation. Afterwards the loss of strength and modulus of the implant should be in harmony with the increasing strength of the injured tissue (Tormälä et al., 1995). Proceeding degradation creates space for restoring processes to fill the gap with ingrown of vital host tissue. Presently, no filling material is available that fits this requirements satisfactorily to form new homogeneous bone in large defects (Rueger et al., 1996).

**Biomaterials and osteoinductive proteins**

To achieve an osteoinductive effect an alternative to the use of autogenic bones is the user of specific bone growth and differentiation factors such as GDF-5 or different bone morphogenetic proteins (BMPs). Numerous animal studies clearly show that this osteoinductive effect can be greatly increased if these protein factors are combined with a carrier which decelerated the protein release and therefore increased the effective residence time of the protein at the defect site and finally to accelerate bone-healing compared to liquid formulation buffers. (Seeherman et al., 2003). In the literature, calcium phosphates, collagen, mineralised collagen (collagen-containing calcium phosphate) and bioresorbable polymers, hydroxyapatite and beta-TCP (Hotz et al., 1994), hydroxylic apatite from algae extracts (Gao et al., 1996), bone extracts (Gombotz et al., 1996), collagen (Friess et al., 1999) and poly(alpha-hydroxy) acids (Hollinger et al., 1996) are described as carriers.

The analyses of the potency of the coated carriers, which are described in the literature, do not present a uniform picture but exhibit significant variations which are a consequence of either the carrier type selected or the coating method (Terheyden et al., 1997). Various methods are described.

In WO 98/21972 first dissolving GDF-5 in an organic solvent and then precipitating it by adding water achieve coating by rapid precipitation of GDF-5 onto beta-TCP. Due to the toxicity of many solvents, however, such a process is not preferred for the production of pharmaceutical compositions. Lind et al. (1996) carry out the coating of various calcium phosphate ceramics in the presence of gelatine (usually obtained from bovine or pig bones) as protection protein. Due to the increased risk of infection and immunogenic reactions, however, the use of animal substances should be avoided for the production of pharmaceutical compositions and medicinal products. Friess et al. (1999) and Gao et al. (1996) describe the coating of collagens with BMP-2. Due to the low compressive strength of collagens, such carriers, however, are not suitable for many indications. This particularly applies to indications with which the newly-formed bone has to sustain a later pressure load. Furthermore, pharmaceutical qualities of collagen are so far available from animal sources only. Finally, according to the fast degradation rate and release of the growth factors in the state of the art products (e.g. rhBMP-2 and collagen sponge) the drug substance content is often dramatically above the physiological level in the bone tissue.

Advantageously, as disclosed in WO 03/043673, it has been found by the present inventors that improved and reliable osteoinductive and osteoconductive properties *in vivo* after implantation into a subject, preferably a human, is achieved in a device, wherein a homogenous distribution of the composite carrier, such as beta-TCP or other calcium phosphates, with biologically active, non-aggregated osteoinductive protein can be realized. Such aggregation causes micro-precipitation, which is the reason for an inhomogenous distribution resulting in at least significantly decreased osteoinductive properties as described for other devices in the prior art, e.g., in WO98/21972. Moreover, it has been found that undesirable side effects, such as inflammation and toxic reactions of the subject after implantation, can be avoided by the device of WO 03/043673, which is free of toxic impurities or infectious contaminants. In particular, the use of protecting proteins (such as e.g. gelatine) as solubility mediator is totally unnecessary for the device of WO 03/043673. However, such devices are not suitable for applications requiring a retarded release of the active agent.

In the field of bone augmentation retarded release systems are especially required in view of short half-life of proteins or peptides in the human body with respect to bone induction, either due to dispersion from the implant site or through degradation. In first attempts to achieve a retarded release of bone morphogenic proteins, devices have been disclosed, wherein such proteins have been combined with bioresorbable polymers. Hollinger et al (1996) published the use of poly (alpha-hydroxy acids) as carriers for BMP-2. In combination with osteogenic proteins or peptides these polymers are of special interests with regards to achieve a controlled release of the active agent. Wang et al (2000) disclose an emulsion freeze-drying process starting with a PLA solution in methylene chloride for the fabrication of a biodegradable scaffold capable of incorporating and delivering bioactive macromolecules for bone regeneration. Schmidmaier et al (2000) disclose the use of a chloroform solution of PLA together with the osteoinductive factors IGF-I and TGF-beta1 in the coating implants.

WO02/070029 discloses a porous beta-TCP matrix which is optionally admixed with PLGA microspheres encapsulated with OP-1 (osteogenic protein 1, a bone morphogenic protein) to form a heterogeneous material. In contrast to WO 03/043673 the beta-TCP matrix in WO02/070029 exhibits single separate voids instead of interconnected pores. The pores of this matrix are not capable to be equipped with a homogeneous coating of the polymer and or active agent component. The microspheres are produced by Alkmeres, Inc and exhibit a 20 to 500 $\mu$m diameter permitting microaggregation of the encapsulated active agent For the production of such microspheres methylene chloride solutions of the polymeric component together with the protein are sprayed and frozen in a deeply cold ethanol (Herbert et al., 1998 and see e.g. US Patent Application No 6,726,860), both steps in combination with two different organic solvents imparting chemical and mechanical stress to the protein.

**Flowable biodegradable polymer based delivery systems**

Placing medical devices such as implants and other solid articles in a body frequently involves a surgical procedure. For some applications e.g. drug delivery or minimally invasive however, it has been described that biodegradable polymer based delivery systems can be introduced in a body as flowable formulations similar to calcium phosphate cements (see e.g. EP0436667, EP0537559, EP0539751, EP0754032, EP0862416, EP1126822. EP1147781, EP1404294, US 6,461,631, US 5,780,044 and US 5,278,202, as well as foreign counterparts, assigned to Atrix Laboratories, Inc.).

In contrast to the also flowable CPC, were the protein is within or onto the carrier in direct contact with the surrounding medium, a protein within a polymer containing carrier (e.g., poly(alpha-hydroxy acids) can be protected and/or stabilized. Furthermore, the protein or peptide is released only by diffusion from the calcium phosphate cement whereas the protein or peptide within the polymer matrix is released with the increasing degradation of the polymer and/or by diffusion from the polymer matrix. Therefore the release kinetic can be fine-tuned more easily than it's the case for the pure calcium phosphate cement.

These compositions comprise of a water insoluble biodegradable polymer in a biocompatible water miscible organic solvent for forming a biodegradable solid implant *in situ* within the body by exposure to body fluids or aqueous fluid and are administered as liquids using a syringe to form *in situ* a solid matrix by dissipation or dispersion of the organic solvent within the body. During contact with water a scaffold with a high porous inner core structure surrounded by a nearly none porous surface is formed.

This none porous surface inhibits cell migration into the inner core therefore these material exhibits no osteoconductive properties. These implants are used as prosthetic devices and/or controlled delivery system for biological active agents.

Another drawback of this type of material class is the prolonged hardening time until the material shows a sufficient mechanical stability. However, the subsequent *in vivo* degradation of the polymer causes similar problems as described above for conventional polymer based scaffolds. They exhibit degradation, leading to a loss in mechanical properties, and a lowering of the local pH to a cytotoxic level. As a consequence this can lead to an Inflammatory foreign body response.

US 2004034434 (A1) discloses an implantable material for deployment in select locations or select tissue for tissue regeneration is disclosed. The implant comprises collagen, ceramics, and or other bio-resorbable materials or additives, where the implant may also be used for therapy delivery.

WO 2004073563 (A2) forms prior art under Article 54(3) EPC and discloses an orthopedic device for implanting between adjacent vertebrae comprising an arcuate balloon and a hardenable material within said balloon.

In addition, they do not possess the same bioactive and osteoconductive properties of calcium phosphate systems described above.

Therefore, there exists a need for an improved injectable- and biocompatible and biodegradable composition, which provides *in vivo* a interconnective porous scaffold for cells infiltration and migration with an accelerated hardening behavior to replace the biomaterial by bony structures while reducing the burden (polymer content) for the organism.

Another object underlying the present invention is to have a premixed *in situ* hardening paste with a good washout resistance that is stable in its package and hardens only after being placed into the defect with sufficient time to adjust the shape of the material to the defect size if necessary.

Another object of the present invention is the provision of an *in situ* hardening paste suitable for implantation into a subject in the need of bone augmentation by a composition being able to form a macroporous scaffold throughout the surface and the interior of the scaffold after being placed into the defect.

Another object underlying the present invention is the provision of an *in situ* hardening paste suitable for implantation into a subject in the need of bone augmentation with a sufficient mechanical strength and/or avoiding the problems associated with a local pH decrease induced by polymer degradation.

Another object underlying the present invention is the provision of an *in situ* hardening paste suitable for implantation into a subject in the need of bone augmentation by injection, molding, filling or pressing. *In situ* the paste with an active agent shall allow the retarded release of an active agent attached to the matrix, preferably with an optimized local activity of said active agent

Another object underlying the present invention is the provision of an *in situ* hardening paste suitable for implantation into a subject in the need of bone augmentation by injection allowing retarded release of an attached active agent and avoiding the problems associated with a local pH decrease induced by polymer degradation.

Another object underlying the present invention is the provision of an *in situ* hardening paste suitable for implantation into a subject in the need of bone augmentation by injection allowing retarded release of an attached active agent and avoiding toxic side effects and / or inflammatory responses.

Another object underlying the present invention is the provision of an *in situ* hardening paste suitable for implantation into a subject in the need of bone augmentation by injection allowing retarded release of an attached active agent and allowing lower doses of the active agent compared to conventional devices.

Another object underlying the present invention is the provision of an *in situ* hardening paste suitable for implantation into a subject in the need of bone allowing retarded release of an attached active agent by injection which paste is capable of forming a mechanically stable and porous scaffold for bone and cartilage defect filling.

Another object underlying the present invention is the provision of an *in situ* hardening paste suitable for implantation into a subject in the need of bone allowing retarded release of an attached active agent by injection which paste hardens under physiological conditions and is capable of replacing conventional devices implanted via surgery.

Summary of the Invention

Surprisingly, the present Inventors were able to provide an *in situ* hardening paste solving these objects and corresponding methods for the production of said paste.

Therewith, the present inventors provide an *in situ* hardening paste of embodiment (1) comprising a plasticizer, a water insoluble polymer and a water Insoluble solid filler which paste is a stable premixed paste that hardens after contact with an aqueous liquid such as water, a physiological solution, cell culture medium (e.g. FCS) or body fluid and exhibits improved mechanical strength and resistance to washout at the Implant site, even if implanted into a wet open field, while reducing the burden of high polymer content for the organism such as humans or animals. Furthermore the inventors provide an *in situ* hardening paste comprising a plasticizer, a water insoluble polymer, water insoluble solid filler and water soluble pore building filler with improved macroporosity.

The high mechanical strength as well as porosity, preferably macroporosity with pore size of about 100 μm and more and the formation of an interconnecting porous scaffold which is sufficient for ingrowth of living cell, support new bone formation in the void filled with the composition.

The embodiments of the invention are:

(1) An *in situ* hardening paste comprising:

(a) a plasticizer, which is a water soluble or water miscible biocompatible organic liquid,
(b) a water insoluble polymer, which is biocompatible, biodegradable, and/or bioresorbable and soluble in the plasticizer,
(c) a water insoluble solid filler, which is insoluble in the plasticizer,

wherein the paste, which is injectable and stable in its package, is capable of hardening *in situ* to form a solid implant upon contact with the aqueous medium or body fluid, wherein the plasticizes is as defined in (1a).

(2) The *in situ hardening paste* according to (1), further comprising an effective amount of a water soluble pore building filler.

(3) The *in situ hardening paste* according to any of (1) or (2), wherein the implant has interconnecting pores with a diameter of equal or more than 100 μm.

(4) The *in situ hardening paste* according to any of (1) to (3), further comprising an active agent.

(5) The *in situ hardening paste* according to any of (1) to (4), comprising an active agent coated onto a water insoluble solid filler or dissolved or suspended in the plasticizer, preferably homogenous coated onto a water insoluble solid filler.

(6) The *in situ hardening paste* according to any (1) to (5), wherein the water insoluble solid filler is

a) an inorganic compound,
b) an organic compound.

In a preferred embodiment (6a) said inorganic compound is magnesiumoxide; magnesium hydroxide, magnesium carbonate, or silicium dioxide.

Alternatively (6b) said inorganic compound is a calcium compound, preferably calcium sulfate or calcium carbonate, more preferably calcium phosphate; and most preferably tricalcium phosphate, beta-tricalcium phosphate, alpha-tricalcium phosphate, apatite, calcium phosphate containing cement or tetracalcium phosphate.

In a further embodiment (6c) said organic compound is chitosan, collagen, calcium alginate, poly(2-hydroxyethyl methacrylate), hyaluronic acid or derivatives thereof, cellulose or derivatives thereof, or starch or derivatives thereof.

Combinations of one or more compounds of (6a), (6b) and (6c) are also encompassed (6d).

(7) The *in situ hardening paste* according to (6), wherein said inorganic compound is alpha-tricalcium phosphate having a particle size which is equal to or greater than 300 μm.

(8) The *in situ hardening paste* according to any of (1) to (7) with at least one further calcium containing water insoluble solid filler, preferably selected from the group of calcium carbonate, calciumhydrogenphosphate or hydroxyapatite.

(9) The *in situ hardening paste* according to any of (1) to (8), wherein the ratio (i.e. weight ratio) of the water insoluble solid filler and the water Insoluble polymer is between 1 : = 1 and 5 : 1, preferably between 1 : 1 and 3 : 1, more preferably approximately 1,5 :1 as in a mixture containing less than 50 wt%, preferably 30 to 36 wt% water insoluble solid filler and less than 40 wt%, preferably 20 - 25 wt% water insoluble polymer.

(10) The *in situ hardening paste* according to any of (1) to (9), wherein said water insoluble polymer is poly(alpha-hydroxy acids), poly(ortho esters), poly(anhydrides), poly(aminoacids), polyglycoild acid (PGA), polylactic acid (PLLA). poly(D,L)-lactic add (PDLLA), poly(lactic-co-glycolic and) (PLGA), poly(lactic-co-glycolic acid) polyethylene glycol (PLGA-PEG) copolymers, poly(3-hydroxybutyrtcacid) (P(3-HB)), poly(3-hydroxy valeric acid) P(3-HV), poly(p-dioxonone) (PDS), poly(epsilon-caprolactone) (PCL), polyanhydride (PA) polyorthoester, polyethylene (PE), polypropylene (PP), polyethylonerephtalate (PET), polyglactine, polyamide (PA), polymethylmethacrylate (PMMA), polyhydroxymethylmethacrylate (PHEMA), polyvinylchloride (PVC), polyvinylalcohole (PVA), polyetetrafluorethylene (PTFE), polyetheretherketone (PEEK), polysulfon (PSU), polyvinylpyrrolidone, polyurethane, polysiloxane, or copolymers, terpolymers, blockcopolymers, combinations, mixtures thereof. These polymers are biocompatible.

(11) The *in situ hardening paste* according to any of (1) to (10), wherein the water insoluble polymer is PLGA.

(12) The *in situ hardening paste* according to (11), wherein the water Insoluble polymer is an end-capped polymer.

(13) The *in situ hardening paste* according to *any* of (1) to (12), wherein the water insoluble polymer content of the composition is equal or smaller than 40 wt%.

(14) The *in situ hardening paste* according to any of (1) to (13), wherein the density of the paste composition is equal to or greater than 1.21 g/ml, preferable between 1,3 g/ml *and* 1,5 g/ml.

(19) The *in situ hardening paste* according to (1), wherein said plasticizer is polyethylene glycol (PEG) 400, PEG 200, PEG 300, PEG 600, 1,3-butandiole, castor oil, N-methyl-2-pyrrolidone, 2-pyrrolidone, C2 to C6 alkanols, propylene glycol, solketal, acetone, methyl acetate, ethyl acetate, ethyl lactate, methyl ethyl ketone, dimethylformamide, dimethyl sulfone, tetrahydrofuran, caprolactam, decylmethylsulfoxide, oleic acid, propylene carbonate, triacetin, N,N-diethyl-m-toluamide; 1-dodecylazacycloheptan-2-one or mixtures thereof.

(15) An *in situ hardening paste* according to any of (1) to (14), wherein the plasticizer content is 40 - 95 wt%, preferably 40 - 55 wt %.

(16) The *in situ hardening paste* according to any of (1) to (15), wherein said water soluble solid pore building filler comprises one or more of a

(a) swelling agent, preferably cellulose derivatives;
(b) surfactant, preferably block copolymers of ethylene oxide and propylene oxide such as Pluronics® or Tween® 80; or
(c) porogenic agent such as trehalose, manniol, sucrose, sorbitol, physiological amino acids, e.g. glycine, glutamine, arginine, sodium citrate, sodium succinate and sodium phosphates, sodium chloride, polyvinylpyrrolidon (PVP), solid PEGs such as PEG 4000, PEG 10000, sodium hydrogen carbonate, calcium sulfate or chitosan; or
(d) gas or gas forming agent such as calcium carbonate or sodium hydrogencarbonate.

(17) The *in situ hardening paste* according to (16), wherein the water soluble solid pore building filler is carboxymethylcellulose salt, preferably a carboxymethylcellulose sodium salt

(18) The *in situ hardening paste* according to any of (16) and (17), wherein the water soluble solid pore building filler content of the composition is less than 10 wt%, preferably less than 5 wt%, more preferably less than 2,5 wt%, most preferably equal to or less than 1 wt%.

(19) The *in situ hardening paste* according to any of (16) to (18), wherein the water soluble solid pore building filter has a particle size of less than 1000, preferably between 25 to 1000 μm, preferably 50 to 500 μm, most preferably 100 to 300 μm.

(20) The *in situ hardening paste* according to any of (1) to (19), comprising:

(a) PEG 400
(b) PLGA
(c) calcium phosphate selected from the group of calcium phosphate containing cement, calcium carbonate, hydroxyapatite, calcium hydrogenphosphate and alpha-tricalcium phosphate or a mixture thereof; and
(d) swelling agent, preferably a carboxymethylcellulose sodium salt.

(21) The *in situ hardening paste* according to any of (1) to (20), comprising:

(a) PEG 400: 40 to 50 wt%, preferably 40 to 45%;
(b) PLGA: 20 to 25 wt%, preferably 22 to 25%;
(c) calcium phosphate selected from the group of calcium phosphate containing cement and aipha-tricaicium phosphate: 25 to 40 wt%, preferably 30 to 35%; and
(d) carboxymethylcellulose sodium salt: equal to or less than 10 wt%. Preferably equal to or less than 5 wt%, more preferably less than 2,5 wt%. most preferably equal to or less than 1 wt.

(22) The *in situ hardening paste* according to any of (1) to (21), wherein said *in situ hardening paste* has osteoinductive and/or osteoconductive, cartilage or periodontal ligament regenerating properties *in vivo.*

(23) The *in situ hardening paste* according to any of (4) to (22), wherein said active agent is selected from the group consisting of hormones, cytokines, growth factors, antibiotics and small molecules.

(24) The *in situ hardening paste* of any of (4) to (23), wherein said active agent is parathyroid hormone (PTH) and/or PTH 1-34 peptide.

(25) *The in situ hardening paste* of any of (4) to (24), wherein said active agent is an osteoinductive or cartilage inductive protein.

(26) The *in situ hardening paste* of (25), wherein said active agent is a member of the TGF-beta family or a member of the BMP family.

(27) *The in situ hardening paste* of (25), wherein said active agent is BMP-1, BMP-2, BMP-3. BMP-4, BMP-5, BMP-6, BMP-7, BMP-8. BMP-9, BMP-10, BMP-11, BMP-12, BMP-13, BMP-14, BMP-15 or BMP-16.

(28) The *in situ hardening paste* of (25) wherein said active agent is GDF-1, GDF-2, GDF-3, GDF-4, GDF-5. GDF-6, GDF-7, GDF-8. GDF-9. GDF-10 or GDF-11.

(29) The *in situ hardening paste* of (25), wherein said active agent is the cartilage regenerating cartilage derived-retinoic acid-sensitive protein (CD-RAP).

(30) A method for the production of the *in situ hardening paste* of any of (1) to (3) or (6) to (24), comprising the steps of:

(a) mixing a component A comprising the plasticizer and a component B comprising the water insoluble polymer by dissolving component B in component A to provide a viscous liquid; and
(b) mixing said viscous liquid obtained in (a) with the water insoluble solid filler to prepare the paste.

(31) The method for the production of the *in situ hardening paste* of any of (4) to (29) comprising the steps of:

(a) mixing a component A comprising the plasticizer and a component B comprising the water insoluble polymer by dissolving component B in component A to provide a viscous liquid;
(b) dissolving the active agent in said viscous liquid; and
(c) mixing said viscous liquid obtained in (b) with the water insoluble solid filler to prepare the paste.

This method is suitable for an active agent soluble in the above viscous liquid.

(32) The method for the production of the *in situ hardening paste* of any of (4) to (29) comprising the steps of:

(a) mixing a component A comprising the plasticizer and a component B comprising the water insoluble polymer by dissolving component B in component A to provide a viscous liquid;
(b) mixing said viscous liquid obtained in (a) with the water insoluble solid filler to prepare the paste, wherein said water insoluble solid filler comprises the active agent which is coated on said solid filler preferably which is homogeneously coated on said filler.

This method is suitable also for an active agent insoluble in the above viscous liquid.

(33) The method of any of (30) to (32), further comprising the step of adding the water soluble pore building filler.

(34) A pharmaceutical composition comprising the *in situ hardening paste* of any of (1) to (29).

(35) Use of the *in situ hardening paste* of any of (1) to (29) for the preparation of a pharmaceutical compositton to be used for bone augmentation.

In a preferred embodiment (35a) said bone augmentation follows traumatic, malignant or artificial defects or is a prerequisite for the subsequent setting of an Implant. Further embodiments related to bone augmentation are listed below.

(36) Use of the *in situ hardening paste* of any of (1) to (24) for the preparation of a pharmaceutical composition for treating bone defects.

In a preferred embodiment (37a) said bone defects are long bone defects, defects In the maxillofacial area or bone defects following apicoectomy, extirpation of cysts or tumors, tooth extraction, or surgical removal of retained teeth.

(37) Use of the in situ hardening paste of any of (1) to (29) for the preparation of a pharmaceutical composition for treating degenerative, traumatic disc disease, spinal fusion, vertebral body fracture, vertebroplastie and kyphoplastie.

(38) Use of the *in situ hardening paste* of any of (1) to (29) for the preparation of a (39) pharmaceutical composition for treating bone dehiscence.

(39) Use of the *in situ hardening paste* of any of (1) to (29) for the preparation of a pharmaceutical composition to be used for sinus floor elevation or augmentation of the atrophied maxillary or mandibular ridge.

(40) Use of the *in situ hardening paste* of any of (1) to (29) for the preparation of a pharmaceutical composition for filling cavities and/or support guided tissue regeneration in periodontology.

(41) Use of the *in situ hardening paste* of any of (1) to (29) for the Preparation of a pharmaceutical composition for promoting chondrogenesis.

(42) Use of the *in situ hardening paste* of any of (1) to (29) for the preparation of a pharmaceutical composition to be used for the treatment of at least one bone disease.

Preferably said bone disease is selected from the following diseases in which chondrogenic differentiation is involved: osteoarthritis; rheumatoid arthritis, injury of articular cartilage due to trauma, maintenance of chondrocyte phenotypes In autologous chondrocyte transplantation, reconstruction of cartilage in the ear, trachea or nose, osteochondritis dissecans, regeneration of intervertebral disk or meniscus, bone fracture and/or osteogenesis from cartilage.

Brief description of the Figures

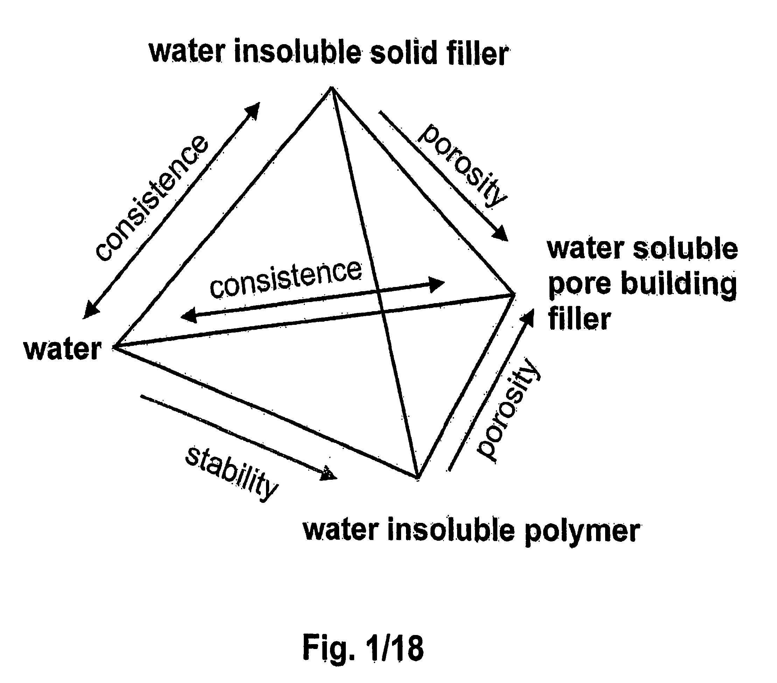

**Figure** 1 shows the influence of the ingredients of the *in situ* hardening paste referring to its consistency, porosity and hardening characteristics depending on their ratio.

**Figure 2** shows various *in situ* forming scaffold (IFS)-compositions or candidate compositions for the *in situ* hardening paste of the present invention with a variation of polymer and calcium phosphate cement ratio and the hardness [%] of the different compositions at three different time points (after 1,9 and 24 days).

black: polyethylene glycol 400 (44.5 wt%), calcium phosphate clement (55.5 wt%)
dark grey: PLGA RG 503H (11.0 wt%), polyethylene glycol 400 (44.5 wt%), calcium phosphate cement (44.5 wt%).
bright grey: PLGA RG 503H (22.2 wt%), polyethylene glycol 400 (44.5 wt%), calcium phosphate cement (33.3 wt%)
white: PLGA RG 503H (38.8 wt%), polyethylene glycol 400 (44.5 wt%), calcium phosphate cement (16.7 wt%)
white with grey points: PLGA RG 503H (38.9 wt%), polyethylene glycol 400 (61.2 wt%) grey with white points: calcium phosphate cement (control)

The calcium phosphate cement is composed of $\alpha$-TCP (59.0 wt%), $CaHP0_4$ (24.0 wt%), $CaCO_3$ (8.5 wt%), hydroxyapatite (8.5 wt%)

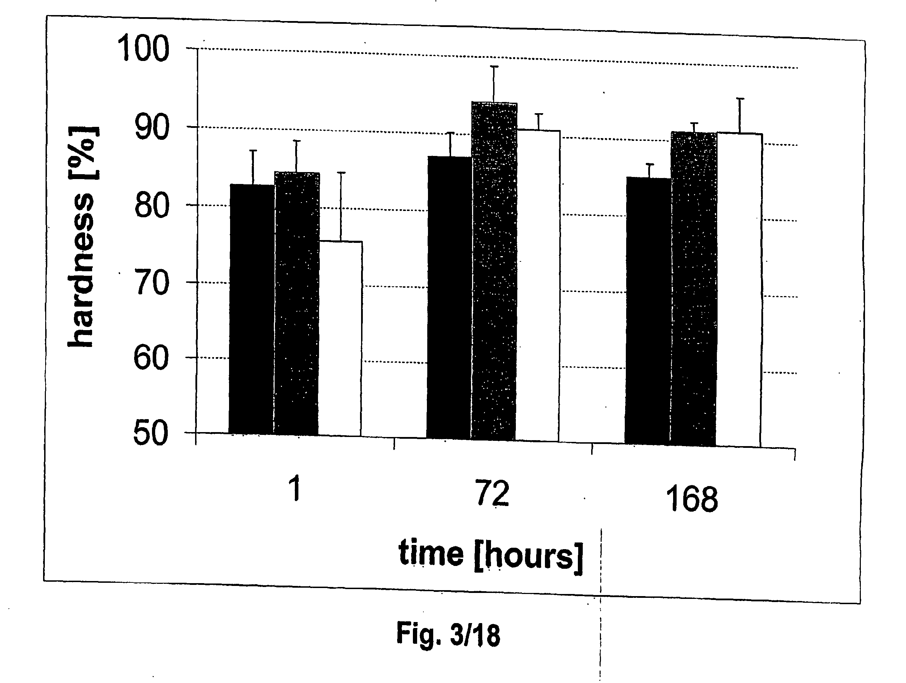

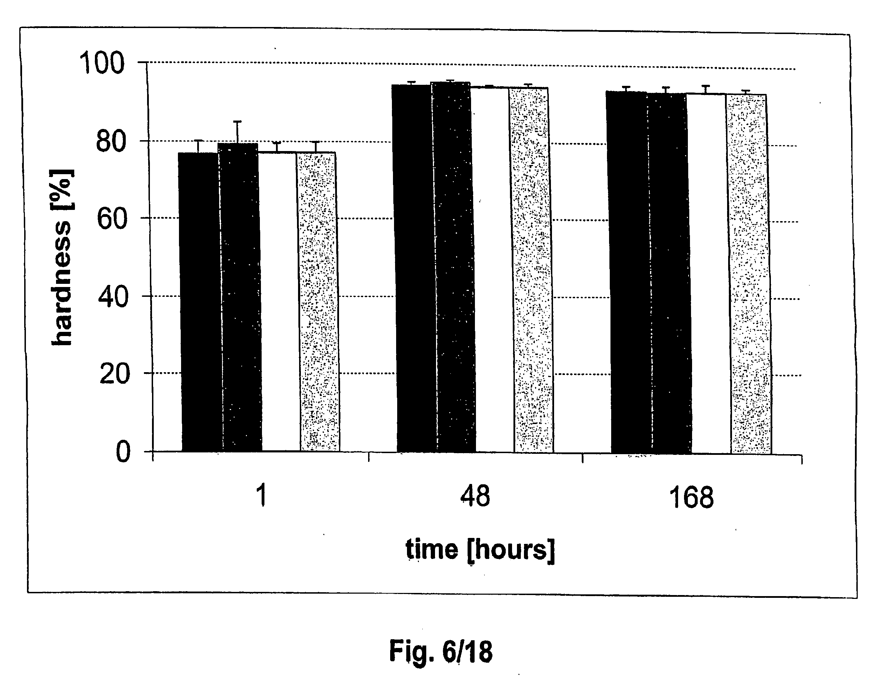

**Figure 3** shows the hardness [%] of various IFS composition with a different polymer to calcium phosphate ratio as a function of time (after 1, 72, and 168 h), wherein 0 is the time point where the IFS specimen was transferred into a PBS-buffer at 37°C and the hardness was determined according to the examples described infra. The composition of the calcium phosphate cement used is described under Fig. 6.

Black: PLGA RG 503H (19.4 wt%), polyethylene glycol 400 (44.5 wt%), calcium phosphate cement (36.1 wt%)
grey: PLGA RG 503H (22.2 wt%), polyethylene glycol 400 (44.5 wt%), calcium phosphate cement (33.3 wt%)
white: PLGA RG 503H (25.0 wt%), polyethylene glycol 400 (44.5 wt%), calcium phosphate cement (30.5 wt%)

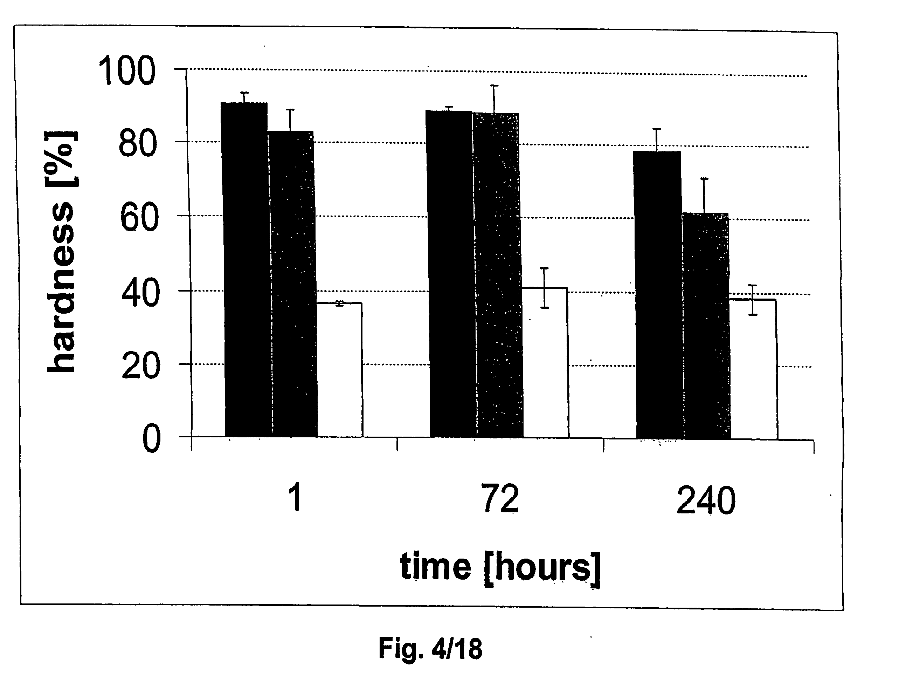

**Figure 4** shows the hardness [%] for IFS compositions with different contents of polyethylene glycol 400 (PEG 400) as a plasticizer. The composition of the calcium phosphate cement used is described under Fig. 6.

black: PLGA RG 503H (22.2 wt%), polyethylene glycol 400 (44.5 wt%), calcium phosphate cement (33.3 wt%)
grey: PLGA RG 503H (20.0 wt%), polyethylene glycol 400 (50.0 wt%), calcium phosphate cement (30.0 wt%)
white PLGA RG 503H (16.0 wt%), polyethylene glycol 400 (60.0 wt%), calcium phosphate cement (24.0 wt%)

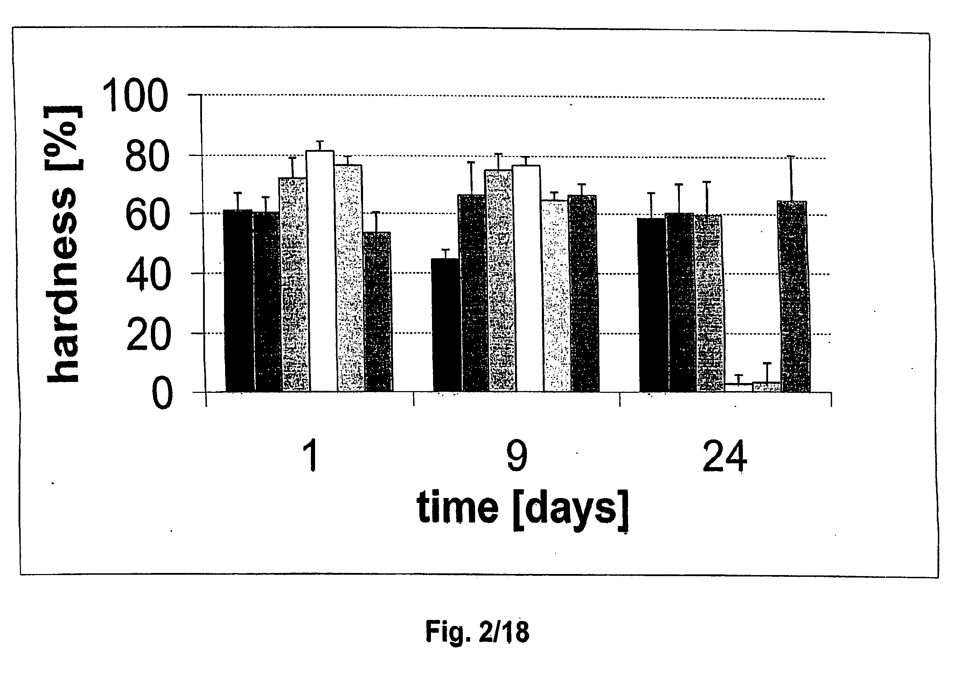

Figure 2 to 4 shows how the composition e.g. polymer / inorganic filler ratio and organic solvent content affect the mechanical properties of the *in situ* hardening paste of the invention. The highest mechanical stability within these examples was observed for the inorganic filler / polymer ratio of 1,5 : 1. Preferred embodiments fulfilling the stability requirements of the present invention are described above. Further details are described below.

Experimental results to evaluate the optimal plasticizer content of the composition to achieve maximum mechanical stability are shown in Fig. 4. Within the range between 44.5 % and lower than 60 % an improved mechanical stability could be achieved in theses analyzed samples.

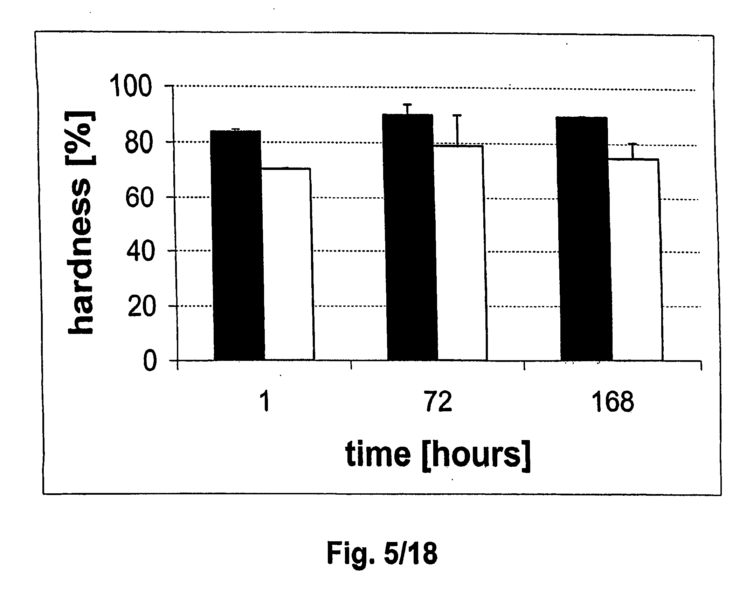

**Figure 5** shows the impact of calcium phosphate cement (CPC, black) versus ß-TCP (white) as an inorganic phase on the hardness [%] of the IFS as a function of time. The composition of the calcium phosphate cement used is described under Fig. 6.

black: PLGA RG 503H (22.2 wt%), polyethylene glycol 400 (44.5 wt%), calcium phosphate cement (33.3 wt%)
white PLGA RG 503H (22.2 wt%), polyethylene glycol 400 (44.5 wt%), ß-tricalcium phosphate (33.3 wt%)

**Figure 6** shows the hardness [%] for various calcium phosphate containing compositions as a function of time (1, 48, 168 h).

Each formulation comprises PLGA RG 503H (22.2 wt%), polyethylene glycol 400 (44.5 wt%) and an inorganic component (33.3 wt%). The inorganic component of the different formulations were used as follows:

black: calcium phosphate cement consisting of $\alpha$-TCP (62.5 wt%), $CaHPO_4$ (26.8 wt%), $CaCO_3$ (8.9 wt%), hydroxyapatite (1.8 wt%)
grey: $\alpha$-TCP
white: $\alpha$-TCP (99.0 wt%), hydroxyapatite (1.0 wt%)
white with grey points: $\alpha$-TCP (98.0 wt%), hydroxyapatite (2.0 wt%)

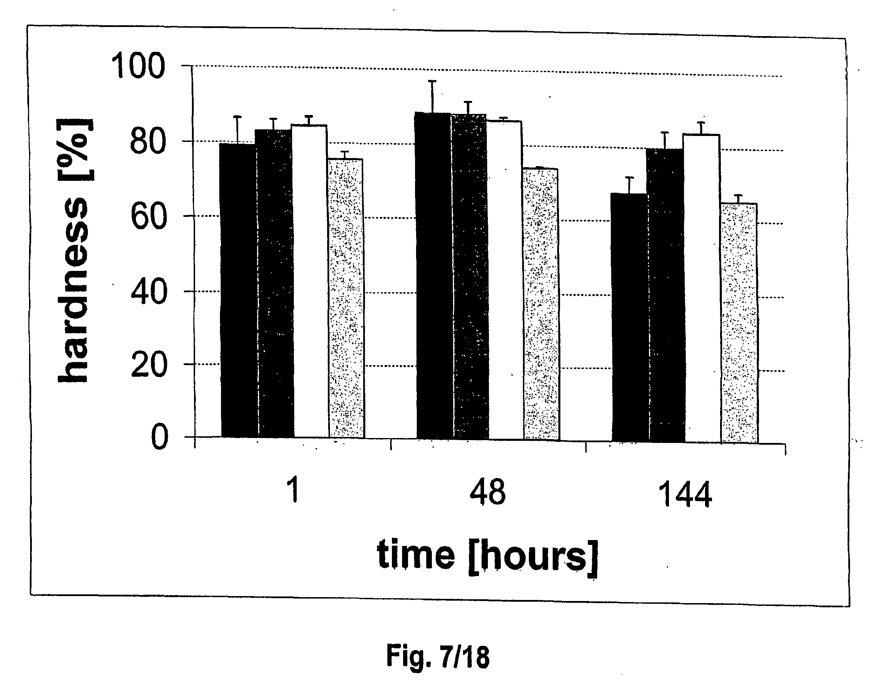

**Figure 7** shows the hardness [%] of the IFS dependent on the particle size of $\alpha$-TCP as a function of time (1, 48 and 144 h).

Each formulation comprises PLGA RG 503H (22.2 wt%), polyethylene glycol 400 (44.5 wt%) and a calcium phosphate cement (33.3 wt%)

The calcium phosphate cement consists of $\alpha$-TCP (62.5 wt%), $CaHPO_4$ (26.8 wt%), $CaCO_3$ (8.9 wt%), hydroxyapatite (1.8 wt%).

The particle size of $\alpha$-TCP is varied:

black: particle size of $\alpha$-TCP granules $\rightarrow$ 300 - 500 $\mu$m
grey: particle size of $\alpha$-TCP granules $\rightarrow$ 500 - 700 $\mu$m
white: particle size of $\alpha$-TCP granules $\rightarrow$ 700 -1000 $\mu$m
white with grey points: particle size of $\alpha$-TCP granules $\rightarrow$ < 300 $\mu$m

In Fig. 7 the inventors show the influence of the particle size of the inorganic filler analyzed on the mechanical properties of the *in situ* hardening paste with improved mechanical stability when using a filler with a particle size of 300 $\mu$m or larger.

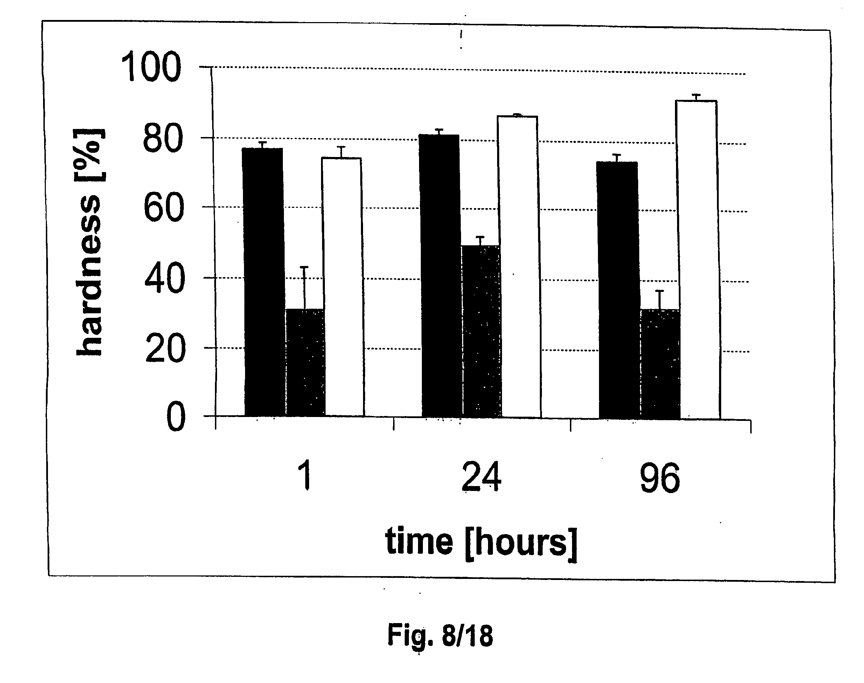

**Figure 8** shows the impact of different organic solvents on the hardness of the IFS compositions over time.

black: PLGA RG 503H (22.2 wt%), calcium phosphate cement (33.3 wt%), polyethylene glycol 400 (44.5 wt%). The composition of the calcium phosphate cement used is described under Fig. 6.
grey: PLGA (22.2 wt%), calcium phosphate cement (33.3 wt%), N-methylpyrrolidone (NMP) (44.5 wt%)
white: calcium phosphate cement (42.8 wt%). N-methylpyrrolidone (57.2 wt%)

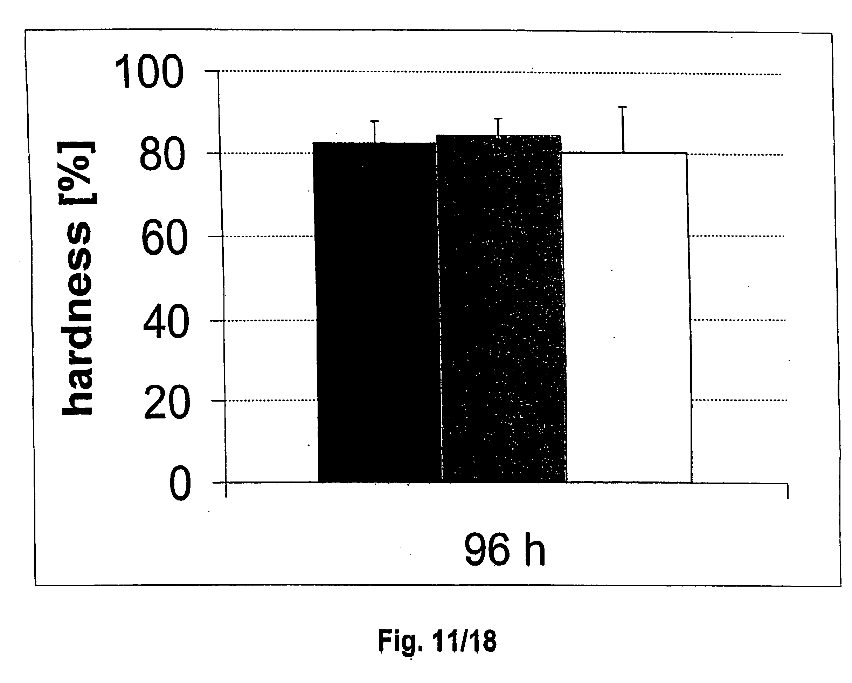

The preferred plasticizers of the present invention are intoxic substances such as PEGs or DMSO (see fig 11). As shown in Figure 8 for PLGA (50/50) PEGs such as PEG 400 yield to an increase in mechanical stability over time (1 to 98 h) compared to NMP, which is frequently used in prior art pharmaceutical polymer liquids, although it is classified in the "deutschen Gefahrstoffstoffverordnung" as "Xi" and as "Giftidasse (CH)5".

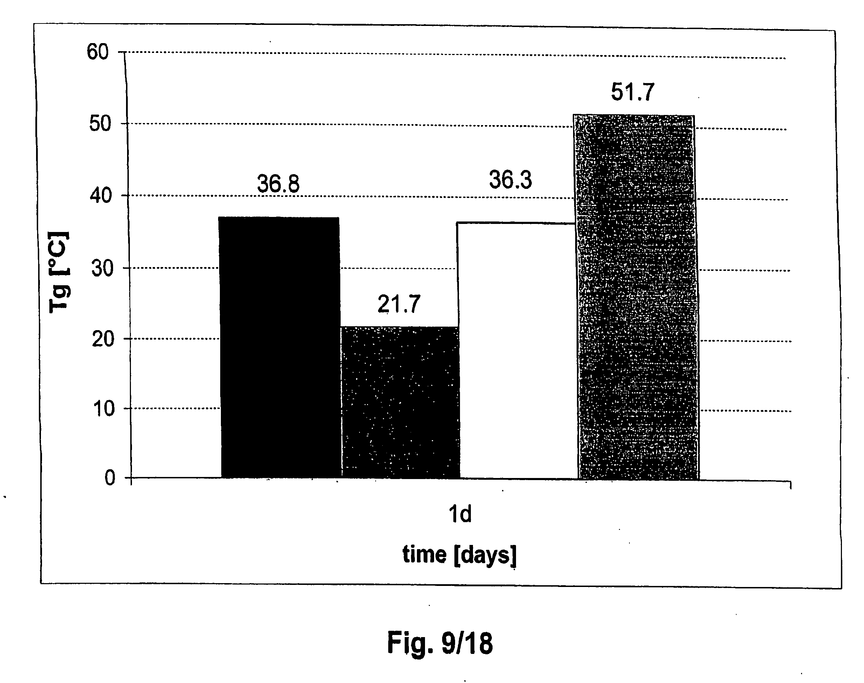

**Figure 9** shows the plasticizing effect of different organic solvents in IFS mixtures with PLGA RG 503H (determination by measuring the decrease of the glass transition temperature (Tg) by DSC according to Example 21). Dependent on the organic solvent used the Tg of the polymer is altered, whereas a reduction in Tg negatively influences the mechanical properties of the IFS.

black: PLGA RG 503H/ polyethylene glycol 400 (1:2)
grey: PLGA RG 503H/ N-methylpyrrolidone (1:2)
white: PLGA RG 503H/ dimethyl sulfoxide (1:2)
grey with white points: PLGA RG 503H

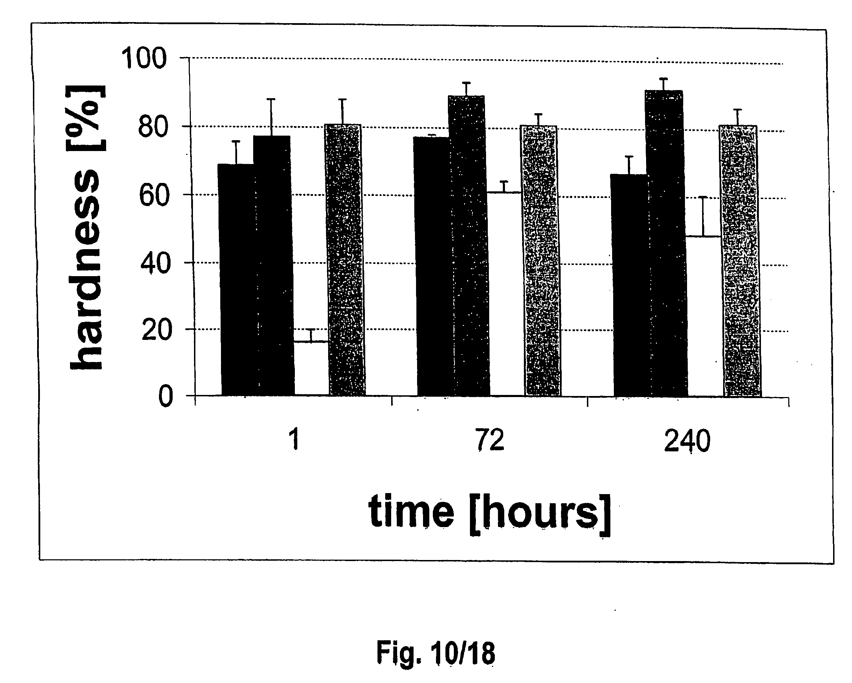

**Figure 10** shows the hardness [%] dependent on different PLGA types. The results reveal that several features have to be taken into account for the selection of a polymer, which is suitable as a component of the IFS. Both the ratio of lactic-/ and glycolic acid with the polymer chain and the inherent viscosity influence the mechanical properties of the hardened IFS. The composition of the calcium phosphate cement used is described under Fig. 6.

black: PLGA RG 756 (22.2 wt%), calcium phosphate cement (33.3 wt%), polyethylene glycol 400 (44.5 wt%),
grey. PLGA RG 503 (22.2 wt%), calcium phosphate cement (33.3 wt%), polyethylene glycol 400 (44.5 wt%),
white: PLGA RG 502 (22.2 wt%), calcium phosphate cement (33.3 wt%), polyethylene glycol 400 (44.5 wt%),
grey with white points: PLGA RG 503H (22.2 wt%), calcium phosphate cement (33.3%), polyethylene glycol 400 (44.5 wt%),

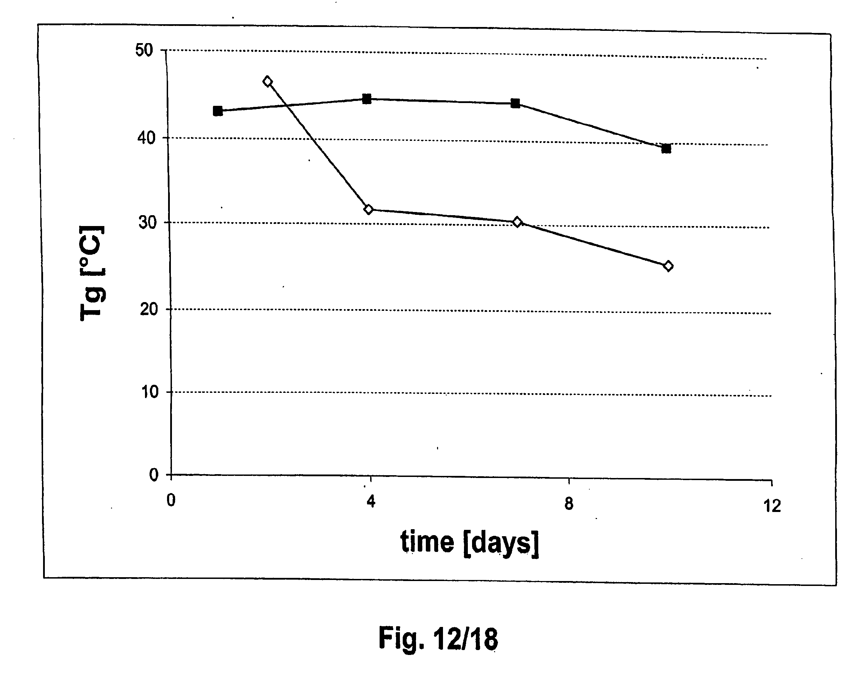

According to the application of the *in situ* hardening paste of the present invention the mechanical properties of the paste can be adjusted by selection of the polymer type (Fig. 10). Thanks to the present invention, the inventors found that the long time mechanical stability of the implant in aqueous media can be significantly enhanced if polymers with a lactic acid / glycolic acid ratio of less than 75:25, preferably 50:50 (RG 503H) are used compared to 75:25 (RG 756H). A further improvement of mechanical stability can be achieved if using end-capped polymers as shown for RG 503 compared to RG 503H (not end-capped) shown in Fig. 10 and 12. Furthermore the mechanical properties are improved using polymers with increased molecular weight (RG 503 compared to RG 502).

**Figure 11** shows the impact of PLGA-PEG diblock-copolymers on the hardness [%] of the IFS

black: diblock-copolymer (11.4 wt%), dimethyl sulfoxide (44.5 wt%), calcium phosphate cement (44.1 wt%)

grey: diblock-copolymer (7.6 wt%), dimethyl sulfoxide (44.5 wt%), calcium phosphate cement (47.9 wt%)
white: diblock-copolymer (3.8 wt%), dimethyl sulfoxide (44.5 wt%), calcium phosphate cement (51.7 wt%)

Not only PLGA can be used as a suitable polymer for manufacturing the *in situ* hardening paste of the present invention, also PLGA-PEG diblock-copolymers result into a paste with a hardness of grater than 80 % of polymer concentrations between 3 and 12 wt%, if used with a plasticizer in which the polymer is soluble such as dimethyl sulfoxide.

**Figure 12** shows a sustained degradation of the IFS by using an end-capped PLGA - copolymer as determined by measuring the glass transition temperature (Tg) as a function of time by DSC as described in example 21. The composition of the calcium phosphate cement used is described under Fig. 6.

black curve: PLGA RG 503 (22.2 wt%, end-capped), PEG 400 (44.5 wt%), calcium phosphate cement (33.3 wt%)
white curve: PLGA RG 503H (22.2 wt%, not end-capped), PEG 400 (44.5 wt%), calcium phosphate cement (33.3 wt%)

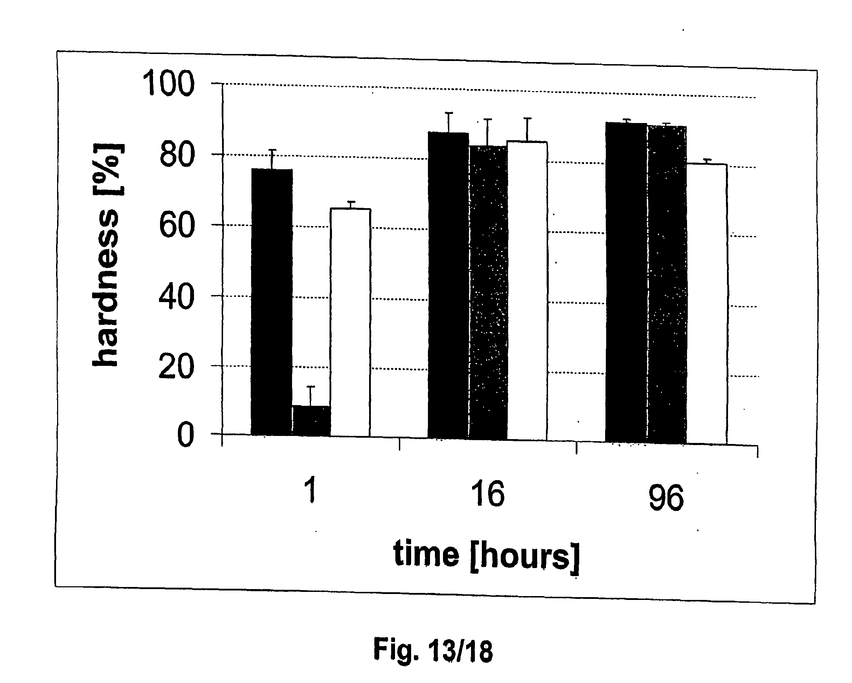

**Figure 13** shows the improved mechanical strength (hardness [%]) of the IFS composition in comparison with other related self-hardening devices over time. The composition of the calcium phosphate cement used is described under Fig. 6.

Thanks to the present invention, an *in situ* hardening paste could be developed with a higher mechanical strength within one hour after hardening of the paste *in situ* compared to a calcium phosphate cement (white bar) or a polymer based system consisting of PLGA and N-methylpyrrolidone (grey bar). In addition, after 96 hours the mechanical strength was higher than the mechanical properties of the calcium phosphate cement.

black: IFS consisting of PLGA RG 503H (22.2 wt%), polyethylene glycol 400 (44.5 wt%) and a calcium phosphate cement (33.3 wt%)
grey: polymer solution consisting of PLGA RG 756 (45.0 wt%) and N-methytpyrrolidone (55.0 wt%)
white: a calcium phosphate cement consisting of alpha-TCP (62.5 wt%), calcium hydrogenphosphate (26.8 wt%), calcium carbonate (8.9 wt%) and hydroxyapatit (1.8 wt%)

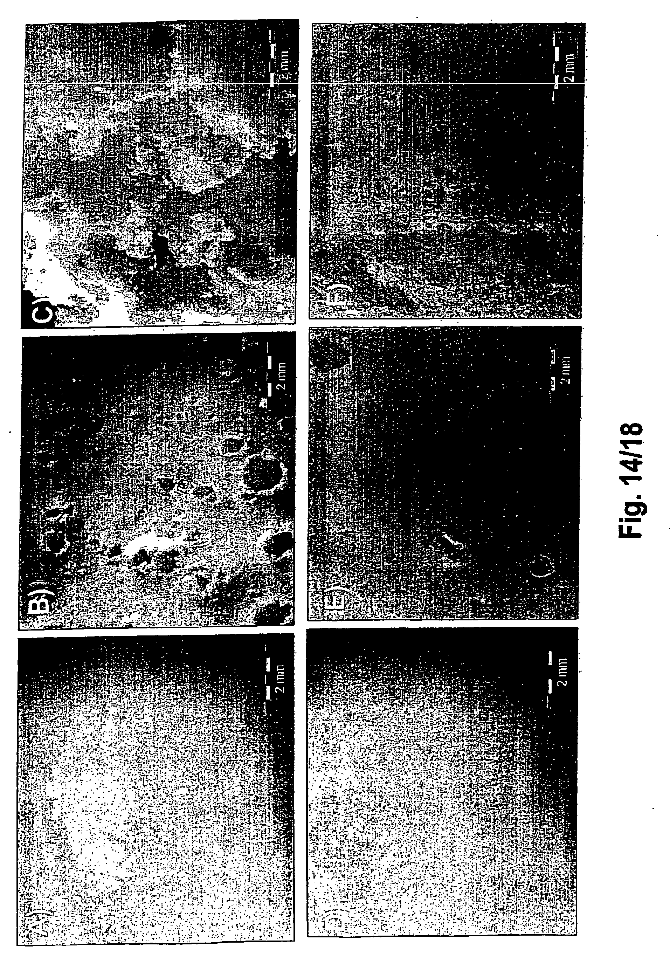

**Figure 14** shows the pore forming capacity of carboxymethylcellulose sodium salt in different concentrations on the pore forming capacity on the outer surface of the IFS. Thanks to the present invention, a skin formation around *the in situ* hardening paste could be prevented by addition of pore forming agents such as carboxymethylcellulose sodium salt, which shows swelling properties in an aqueous ambience. If carboxymethylcellulose sodium salt was introduced in the IFS formulation, a pore forming process occurs on the surface of the IFS composition after application (hardening *in situ*) with pore sizes, which now facilitate cell migration into the internal structures of the IFS. However, a sufficient macroporosity can only be achieved after addition of for example carboxymethylcellulose sodium salt into the composition of the present invention, whereas other compositions such as those comprising NMP are less porous at the outer structure of the *in situ* forming matrix.

A) PLGA RG 503H (22.2 wt%), polyethylene glycol 400 (44.5 wt%), calcium phosphate cement (33.3 wt%)
B) PLGA RG 503H (21.6 wt%), polyethylene glycol 400 (43.4 wt%), calcium phosphate cement (32.5 wt%), carboxymethylcellulose sodium salt (2.5 wt%)
C) PLGA RG 503H (21.1 wt%), polyethylene glycol 400 (42.3 wt%), calcium phosphate cement (31.6 wt%), carboxymethytceitutose sodium salt (5.0 wt%)
D) PLGA RG 756 (45.0 wt%), N-methylpyrrolidon (55.0 wt%)
E) PLGA RG 756 (43.9 wt%), N-methylpyrrolidon (53.6 wt%), carboxymethylcellulose sodium salt (2.5 wt%)
F) PLGA RG 756 (42.8 wt%), N-methylpyrrolidon (52.2 wt%), carboxymethylcellulose sodium salt (5.0 wt%)

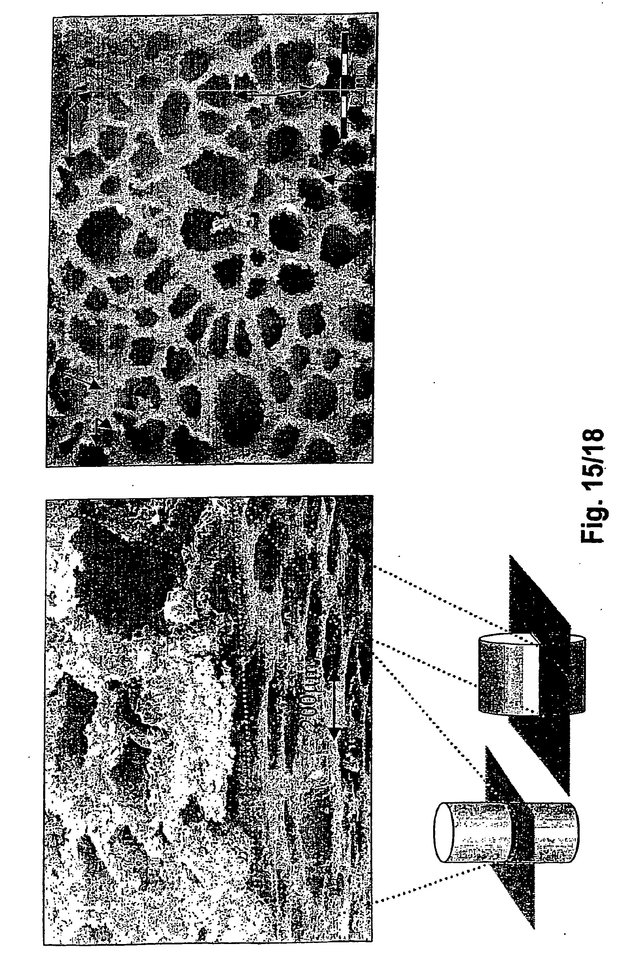

**Figure 15** presents a SEM - microphotography of the IFS showing high macropores of the hardened paste and interconnecting pores.

A shows a SEM - microphotography of the IFS, which was taken according to example 20. The specimen shown is the result of two cross-sections In an angle of 90°. The two main directions in which the pore formation has taken place throughout the scaffold, lead to a three dimensional network of interconnecting pores (micro- and macropores).
composition of the IFS: PLGA RG 503H (22.2 wt%), calcium phosphate cement (33.3 wt%), polyethylene glycol 400 (44.5 wt%)

B shows interconnecting pores of the IFS

The photo made by light microscopy shows the cross-sectional area of an IFS specimen of the following composition: PLGA 503H (22.2 wt%), calcium phosphate cement (33.3 wt%), polyethylene glycol 400 (44.5 wt%).

Thanks to the present invention a macroporous scaffold similar to spongiosa could be generated. Arrows figure out regions, which show the interconnectivity of pores generated within the *in situ* hardening paste of the invention, which are present all over the *in situ* scaffold formed.

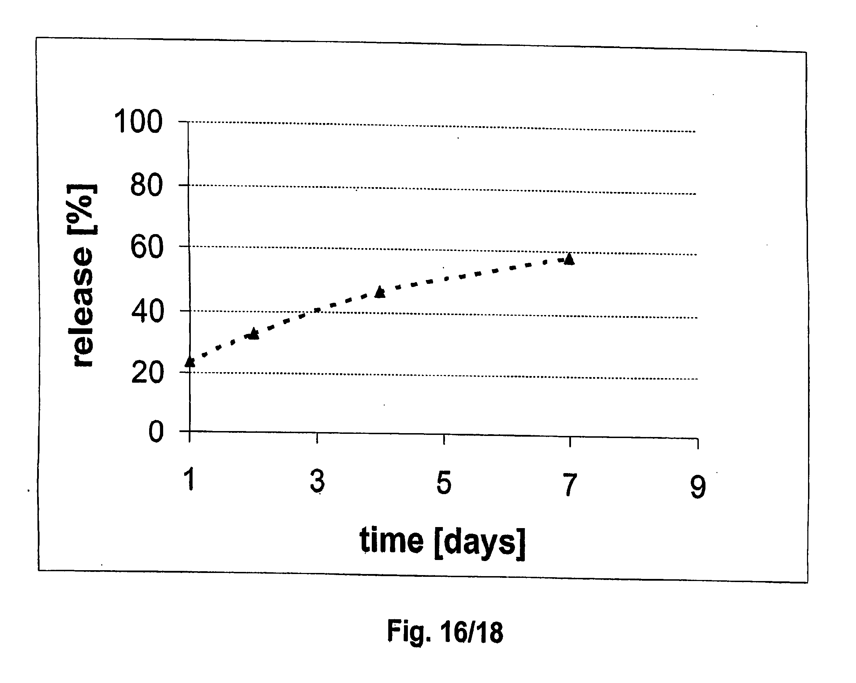

**Figure 16** shows the release of rhGDF-5 from IFS composition over time in days. The used IFS was composed of PLGA RG 503H (22.2 wt%), polyethylene glycol 400 (44.5 wt%); calcium phosphate cement (33.3 wt%) including rhGDF-5 coated α-TCP granules (62.5 wt%); resulting rhGDF-5 concentration: 104 μg/ g IFS. The release of protein was determined as described by Example 15 at 4°C. Thanks to the present invention a sustained release of active agent could be shown over 7 days. After 7 days only 60 % of active agent were delivered.

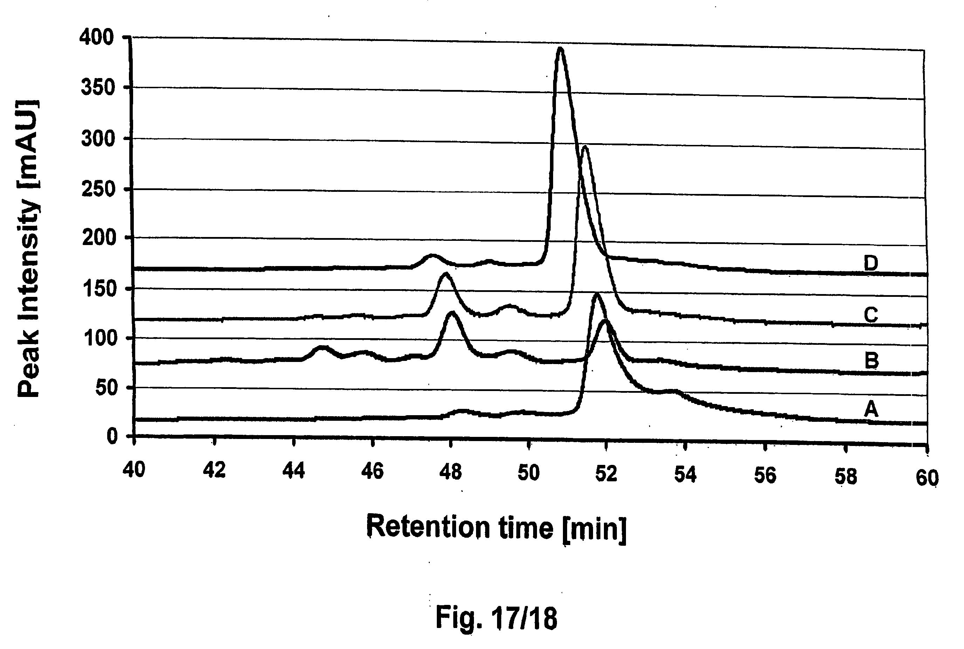

**Figure 17** shows the stability of rhGDF-5 coated α-TCP granules in various organic solvents (A - Aceton, B - NMP, C - PEG 400) determined according to Example 14 under non-sterile conditions. D shows rhGDF-5 coated α-TCP granules without organic solvent treatment as a control. The stability of the protein was determined as relative peak area by the amount of native rhGDF-5 after extraction from the coated α-TCP granules. The highest stability could be achieved for PEG 400 over the other organic solvents analyzed using rhGDF-5 as an exemplary active agent. The stability can be further increased if the manufacturing conditions are further improved such as manufacturing under sterile conditions, inert gas or addition of stabilizers.

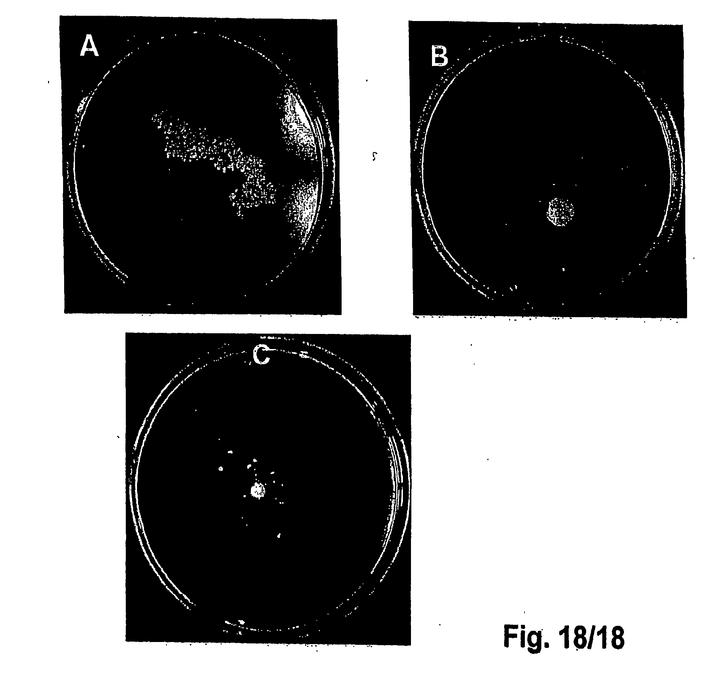

**Figure 18** shows the washout resistance of IFS compared to other state of the art systems such as Ostim® hydroxyapatite paste purchased from Heraeus Kulzer (A) and Calcibon® calcium phosphate cement purchased from Merck (C) with the IFS of the present invention (B) after application of the paste to pure water. The used *in situ* hardening paste was composed of PLGA RG 503H (22.2 wt%), polyethylene glycol 400 (44.5 wt%) and a calcium phosphate cement ad 100 % containing α-TCP (62.5 wt%), calcium hydrogen phosphate (26.8 wt%), calcium carbonate (8.9 wt%) and hydroxyapatite (1.8 wt%). The assay was carried out at ambient temperature. Ostim® was directly injected in the aqueous media. Calcibon® was premixed by merging 1g of cement powder and 0.32 ml of cement liquid. After 5 minutes the resulting pasty cement mixture was placed in the aqueous media. It could be observed, that the IFS showed excellent washout resistance, remained stable, and hardened while immersed in water in contrast to the other specimen analyzed.

Detailed description of the preferred embodiments

For the purpose of promoting an understanding of the principles of the invention, references will be made to certain embodiments thereof and specific language to be used to describe the same. It will nevertheless be understood that no limitation of the scope of the invention is thereby intended, such alterations, further applications and modifications of the principle of the invention as illustrated herein being contemplated as would normally occur to one skilled in the art to which the invention relates.

**A The *in situ* hardening paste**

As indicated above, the present invention generally provides an *in situ* hardening paste composition including at least three components: a pasticizer, which is a water soluble or water miscible biocompatible organic liquid, a water insoluble polymer, which is biocompatible, biodegradable, and/or bioresorbable and soluble in the plasticizer, and a water insoluble solid filler, which is insoluble in the plasticizer, wherein the paste, is injectable and stable in its package and hardens after being placed in the defect. After getting into contact with an aqueous medium or body fluid, the *in situ* hardening paste is capable of hardening *in situ* to form a solid implant. Preferably stability in the package of the premixed paste is at least for several weeks, more preferably several months, most preferably at least one year. Stability can be understood as a consistency and moldabllity of the premixed *in situ* hardening paste without dramatic alterations in the consistency over time. The package is a commonly used waterproof package such as commonly used for parenteral applications in pharmaceutical applications.

The term *in situ* hardening paste, *in situ* forming scaffold (IFS), *in situ* forming paste, IFS, IFS composition, ceramic/polymer composite paste and paste are used interchangeable within the present invention.

The term "*in situ* hardening" as used In the present invention refers to a solid implant being formed after contact with an aqueous medium such as water, a physiological solution or body fluid after dissipation or dissolution of the organic solvent Into the surrounding *ex vivo* as well as in an organism such as a human or an animal body or tissue. Dependent on the indication and use of the paste such a solid implant would also encompass an implant which at least has a higher mechanical strength after getting into contact with a surrounding body fluid than the paste before application, e.g., in the case of an *in situ* hardening paste for periodontal repair.

The term "paste" as used in accordance to the present invention refers to a soft, smooth, thick mixture or material, or paste like entity administrable using a syringe or minimal invasive application, which comprises at least three components, preferably at least four components, most preferably at least five components as set forth above. The paste is suitable for surgical defect filling, tissue regeneration or bone augmentation. In another embodiment the paste is used as a drug delivery system for the controlled release of active substances after implantation.

In a preferred embodiment the paste of the present invention is free of toxic substances. Preferably such toxic substances are already avoided in the production process, as their production requires additional expenditure due to required removal steps during the production process and necessary expensive means for highly sensitive chemical analysis.

The term "toxic substances", in particular, encompasses those toxic organic solvents and additives which are used by the methods described in the art, which are classified by the ICH as class 2 solvents (ICH Topic Q 3 C Impurities: Residual Solvents) e.g. methylene chloride. Said substances may cause systemic or local toxic effects, inflammation and / or other reactions after implantation of devices containing said substances. Said prior art devices are therapeutically less acceptable due to said undesirable side effects, which cannot be avoided by the conventionally coating methods described in the art. Moreover, the international guidance for the development of therapeutic proteins requires that in the manufacturing process harmful and toxic substances should be avoided (for details see: international Conference on Harmonization (ICH), Topic Q3C; www.emea.eu.int/). However, the paste of the present invention or a paste, which is obtainable by the method of the present invention is, advantageously, free of said class 1 classified toxic substances. Moreover the present invention contains only solvents classified as class 3 by the ICH Topic Q 3C and, therefore, therapeutically well acceptable and fulfills the requirements of the regulatory authorities. Preferably the same requirements as for solvents in common are valid for the plasticizer, the water insoluble solid filler and/or the water soluble pore building filler of the *in situ* hardening paste of the present invention.

The term "density" means the density of the pasty formulation prior to the application into an organism and prior to hardening. Its calculated according to example 23. The density of the paste composition of the present invention is equal to or greater than 1,21 mg /ml.

Moreover, in a further preferred embodiment of the paste or the method of the invention said paste is free of infectious material.

Besides toxic substances, infectious material comprised by a prior art device may cause severe infections in a subject into which the device has been transplanted. Potentially infectious gelatine derived from bovine or porcine bones is, however, used as a protecting protein in many state of the art methods (Lind et al., 1996).

The variation of the concentration of the components of the *in situ* hardening paste of the present invention lead to an adaptation to a specific medical application by changes within the consistency of the injectable paste, hardening time *in situ,* porosity and the mechanical properties of the final implant. Additionally the variation of these parameters is a potent means in adapting the release kinetic of the active agent by changed degradation behavior of the water insoluble polymer.

**B The plasticizer**

The term "plasticizer" according to the present invention means a water soluble or water miscible organic liquid or solvent which is pharmaceutically acceptable or a mixture thereof. Dependent on the feature of the active agent the function of the plasticizer is to dissolve the water insoluble biodegradable, biocompatible and/or bioresorbable polymer or dissolve the water insoluble biodegradable, biocompatible and/or bioresorbable polymer and dissolve or suspend the active agent; to suspend the water insoluble solid filler material; or to dissolute the insoluble polymer additionally suspending the water insoluble solid filler. During *in situ* hardening in contact with aqueous medium or body fluid the plasticizer diffuses out of the paste, leaving pores and leading to a form stable composite device or *in situ* implant. Therefore, the plasticizer has to be a water soluble or water miscible solvent, and Is a liquid, preferably a water soluble polymer. Preferably the plasticizer has a low impact on the glass transition temperature of the water insoluble polymer in the *in situ* hardened implant and is compatible with the active agent. Dependent on the water insoluble polymer a plasticizer selected from a group of plasticizers further defined below should be used with the lowest impact on the glass transition temperature of the polymer after setting.

The term "dissolving" means the dissolution or suspension of a substance in a liquid, yields to a homogenous distribution of the substance within the liquid.

Preferably said plasticizer is biocompatible. Said plasticizer is selected from the group consisting of polyethylene glycol (PEG) 400, PEG 200, PEG 300, PEG 600, 1,3 butandiole, castor oil. C2 to C6 alkanols, propylene glycol, solketal, acetone, methyl acetate, ethyl acetate, ethyl lactate, methyl ethyl ketone, dimethylformamide, dimethyl sulfoxide, dimethyl sulfone, tetrahydrofuran, caprotactam, decylmethylsulfoxide, oleic acid, propylene carbonate, triacetin, N,N-dlethyl-m-toluamide, 1-docecylazacycloheptan-2-one or mixtures thereof.

Most preferably, said plasticizer is a polyethylene glycol, because of its relatively low impact on the Tg of the polymeric component (Fig. 8, 9), the accelerated hardening process of polyethylene glycol comprising pastes compared to those, comprising N-methylpyrrolidone (Fig. 13) and the high compatibility of polyethylene glycol with proteins (Fig. 17).

Preferably the *in situ* hardening paste of the present invention contains less than 60 % of the plasticizer, more preferably less than 55%, more preferably less than 50 %, even more preferably equal or less than 45 %, but more than 40 %, most preferably between 40 % and 45 %. In *situ* forming scaffolds of the present invention containing such an amount of PEG exhibit improved mechanical properties (Fig. 4).

The term "biocompatible" means the ability of a material to perform with an appropriate host response in a specific application (Wintermantel E. et al., 2002). Furthermore the term "biocompatible" means, that the material does not exhibit any toxic properties and that it does not induce any immunological or inflammatory reactions after application.

The term "biodegradable" specifies materials for example polymers, which break down due to macromolecular degradation with dispersion *in vivo* but for which no proof exists for the elimination from the body. The decrease in mass of the biodegradable material within the body is the result of a passive process, which is catalyzed by the physicochemical conditions (e.g. humidity, pH value) within the host tissue.

The term "bioresorbable" specifies materials such as polymeric materials, which underwent degradation and further resorption *in vivo;* i.e. polymers, which are eliminated through natural pathways either because of simple filtration of degradation by-products or after their metabolization. Bioresorption is thus a concept, which reflects total elimination of the initial foreign material. In a preferred embodiment of the paste or the method of the invention said bioresorbable polymer is a polymer that undergoes a chain cleavage due to macromolecular degradation in an aqueous environment. It has to be mentioned that the term "resorption" always describes an active process.

## C The water insoluble polymer

The term "water insoluble polymer" means a polymer not soluble in water, i.e. does not form a homogeneous phase when admixed with water, which is soluble in the plasticizer and capable of solidifying in aqueous media to form a solid implant in which the water insoluble solid filler is incorporated upon removal of the plasticizer into the surrounding tissue. Preferably said water insoluble polymer is a "biocompatible", a "biodegradable" and/or a "bioresorbable" polymer. More preferably said water insoluble polymer is an aliphatic polymer preferably with a glass transition temperature above 35°C of the pure polymer substance. The inherent viscosity (viscosity measured at 25 °C, 0.1 % in chloroform) of the polymers of the invention will range from about 0.1 dl/g to 5 dl/g, preferably from about 0,1 dl/g to 1 dl/g.

Alternatively, said water insoluble polymer is selected from the group consisting of polyethylene (PE), polypropylene (PP), polyethylenerephthalate (PET), polyglactine, polyamide (PA), polymethylmethacrylate (PMMA), polyhydroxymethylmethacrylate (PHEMA), polyvinylchloride (PVC), polyvinylalcohole (PVA), polytetrafluorethylene (PTFE), potyetheretherketone (PEEK), polysulfon (PSU), polyurethane, polysiloxane or mixtures thereof. These polymers are at least biocompatible.

More preferably, said polymer is selected from the group consisting of poly(alpha-hydroxy acids), poly (ortho esters), poly(anhydrides), poly(aminoacids), polyglycolid (PGA), polyfactid (PLLA), poly(D,L-lactide) (PDLLA), poly(D, L-lactide-co-glycolide) or poly(L-lactide-co-glycolide) (PLGA), poly(lactic-co-glycolic acid) polyethylene glycol (PLGA-PEG) copolymers, poly(3-hydroxybutyricacid) (P(3-HB)), poly(3-hydroxy valeric acid) (P(3-HV)), poly(p-dioxanone) (PDS), poly(epsilon-caprolactone) (PCL), polyanhydride (PA), copolymers, terpolymers, blockcopolymers, combinations, mixtures thereof. These polymers are at least biocompatible and biodegradable.

The term "polymer content of the composition" means the weight proportion of the polymer, contained in a formulation relating to the total weight of the composition. The *in situ* hardening paste of the present invention preferably has a polymer content equal to or less than 40 wt% of the polymer, more preferably equal to or less than 35 wt% of polymer, even more preferably equal to or less than 33 wt%, most preferably 20 to 25 wt%. These compositions with a selected amount of polymer exhibit the best mechanical properties of the *in situ* hardening paste according to the invention (Fig. 2 to 4).

More preferably, said polymer is PLGA, most preferably PLGA (50:50). By changing the lactic acid: glycolic acid ratio, it is possible to tailor the rate of degradation to that required for the specific application or use by varying the glycolic acid composition within the polymer chain between 0 and 100 mol% (m%) glycolic acid, preferable 50 m% (50: 50) within the polymer chain. Another possible parameter to fine-tune the degradation behavior of the polymer is the selection of the enantiomeric ratio of the lactic acid (d-form or l-form). PLGA (50:50) means a lactic acid: glycolic acid monomer ratio in the polymer chain of about 1:1. According to the present invention PLGA with a higher molecular weight results to implants with a higher mechanical stability over time such as observed for the higher molecular weight polymer RG 503 compared to the lower molecular weight polymer RG 502 as shown in Fig. 10. Therefore preferably polymers with a higher molecular weight are favorable.

In another embodiment of the present invention the water insoluble polymer is an end-capped polymer. The term "end-capped polymer" means that the free carboxylic acid group of the linear polymer chain as is present for example in the polymer RG 503H has been esterified with alcohols. By using end-capped polymers and regulation of the amount of free carboxylic acid groups within the polymer the degradation rate of the polymer can be regulated dependent on the application of the *in situ* hardening paste of the present invention. The inventors found furthermore in extensive degradation studies that the long time mechanical stability of the implant in aqueous media can be significantly enhanced if end-capped polymers are used as shown for a PLGA (50:50) such as RG 503 (Fig. 10). The degradation can for example be demonstrated by investigating the decrease of the glass transition temperature (Tg) of the polymer (Fig. 12). Further analytical experiments show a relation between the glass transition temperature of the polymer and the mechanical stability *in vitro* as determined in the examples. A low glass transition temperature results in a decreased mechanical stability of the *in situ* forming scaffold. In addition these degradation products (e.g., oligomers) can exert a plasticizing effect on the residual polymeric entity reducing the mechanical strength.

In another embodiment of the present invention the water insoluble polymer is a PLGA-PEG copolymer, preferably a PLGA-PEG diblock- or triblock-copolymer. As shown in Fig. 11 suitable *in situ* hardening pastes can also be generated wherein the polymeric components are PLGA-PEG diblock-copolymers. By alteration of the PLGA / PEG ratio within the polymer chain these polymers allow a further adjustment of the degradation behavior. Instead of the conventional PLGA polymers without PEG these materials allow the release of incorporated active agents by diffusion. As was shown according to the invention PLGA-PEG diblock-copolymer formulations exhibit mechanical properties comparable to formulations containing PLGA as polymeric component. Indeed the used PLGA-PEG diblock-copolymer was hardly soluble in polyethylene glycol. In such cases another plasticizer needs to be selected such as dimethylsulphoxide (DMSO).

## D The water insoluble solid filler

The term "water insoluble solid filler" means a compound insoluble in water as well as in the plasticizer i.e. does not form a homogeneous phase when admixed with water or the plasticizer. The water insoluble solid filler serves as matrix in the paste, and as carrier for the active agent within the scaffold formed *in vivo,* once the paste is hardened. Furthermore, the water insoluble solid filler can further increase the biocompatibility (e.g., cell attachment) to stabilize the local pH during degradation of the polymer.

Preferably said water insoluble solid filler is an inorganic or organic compound.

Preferably the inorganic compound is selected from the group of magnesiumoxide, magnesiumhydroxide, magnesium carbonate, silicium dioxide or a calcium compound. Said water insoluble solid filler forms an inorganic matrix within the water insoluble polymer. Said inorganic matrix consists of ceramics and improves in the hardened state the osteoconductive properties as well as the mechanical performance of the scaffold. By using a water insoluble solid filler material it is possible to reduce the amount of the water insoluble polymer without decreasing the overall behavior of the scaffold. More preferably said water insoluble solid filler is a calcium phosphate; calcium sulfate or calcium carbonate, most preferably tricalcium phosphate, beta-tricalcium phosphate ($\beta$-TCP), alpha-tricalcium phosphate ($\alpha$-TCP), apatite, calcium phosphate containing cement or tetracalcium phosphate, or a mixture of the above various different inorganic, preferably calcium containing compounds.

The term "calcium phosphate" encompasses compositions comprising calcium ions ($Ca^{2+}$), phosphate ions ($PO_3^{3-}$), optionally, further ions like hydroxyl ions ($OH^-$), carbonate ($CO_3^{2-}$) or magnesium ($Mg^{2+}$) or other ions which are suitable for the water insoluble solid filler of the present invention. The calcium phosphates as used in accordance with the present invention are crystals having a three dimensional structure suitable for the paste of the present invention as set forth above. Said calcium phosphates are particularly well suited as carriers for the paste of the present invention. Their *in vivo* properties have been described in Hotz, 1994, Gao, 1996, and in WO98/21972. A list of preferred and well-known calcium phosphates is given above.

Calcium phosphate containing cements (CPC) include but are not restricted to for example apatite CPC, brushite CPC, mixtures of tetracalcium phosphate (TTCP) and dicalcium phosphate anhydrous (DCPA), mixtures of alpha-TCP and hydroxyapatite or Calcibon® cement (Biomet Merck Darmstadt), which consists of a mixture of 62.5 % by weight alpha-tricalcium phosphate, 26,8 % by weight dicalcium phosphate anhydrous (DCPA), 8,9 % by weight calcium carbonate ($CaCO_3$), and 1.8 % by weight hydroxyapatite (HA). Further examples of CPC are described supra and infra. Calcium phosphate containing cements can be combined with one ore more inorganic water insoluble fillers.

Preferably the organic compound is selected from chitosan, collagen, calcium alginate, poly(2-hydroxyethyl methacrylate), hyaluronic acid or derivatives thereof, cellulose or derivatives thereof, or starch or derivatives and / or any combinations thereof.

Organic fillers can be added to increase bioadhesion of the implant for example collagen. Such components can further affect the final mechanical properties (e.g., tensile strength, torsion) of the implant comparable to the function of collagen within natural bone such as fiber reinforcement

The *in situ* hardening paste of the present invention is effective for use in organisms such as humans and animals, wherein the composition provides a lasting mineral scaffold to support bone ingrowth. Accordingly a feature of the present invention is the provision of a premixed stable composition useful for example as a void filler or for bone augmentation.

In accordance with the present invention, the composite matrix is preferably based on a calcium phosphate, which is a beta tricalcium phosphate, alpha-tricalcium phosphate, apatite, hydroxyapatite, calcium carbonate, calcium hydrogenphosphate and / or a calcium phosphate containing cement or a mixture thereof.

In such a composite material the calcium phosphate shows excellent local buffering capacity and the permeable composite structure avoids even local pH decrease when the polymer is degraded *in vivo*. Cytotoxic side effects due to degradation of the polymer are, hence, reduced or avoided. This is especially valid, since the ceramic material is chief ingredient of the ceramic/polymer composite material of the present invention, which preferably contains less than 40 wt% of the polymer, more preferably less than 35 wt% of polymer such as PLGA even more preferably equal or less than 33 wt% of PLGA, most preferably 20 to 25 wt%.

In a preferred embodiment of the present invention the content of the water insoluble solid filler in the *in situ* hardening paste of the present invention is less than 50 wt% between 25 wt% and 50 wt%, preferably between 28 wt% and 38 wt%, most preferably between 30 wt% and 36 wt%. Moreover, the content of the water insoluble solid filler is preferably chosen depending from the water insoluble polymer, e.g. with a ratio of water insoluble solid filler to water insoluble polymer of 1,2 : 1 to 4 : 1, preferably 1,2 : 1 to 2 : 1, more preferably 1,2 : 1 to 1,8 : 1, most preferably 1,5 : 1. The present invention is based on the further surprising results that within one day the *in situ* hardening paste according to the invention containing a water insoluble solid filler in the range of equal or more than about 15 wt% and a water insoluble polymer of less than 40 wt% has a higher mechanical strength than other systems such as CPC, CPC and plasticizer or a conventional polymer/organic solvent system (PLGA/PEG) (Fig. 2). Over 24 days the *in situ* hardening paste containing a water insoluble solid filler in the range of more than 20 % and a water insoluble polymer of less than 38 % has a hardness comparable to CPC or CPC and plasticizer and a significantly improved hardness over PLGA/PEG alone as shown in Fig. 2 and 3. The influence of the ratio of calcium phosphate as a function of time on the hardness of various the *in situ* hardening pastes has also been analyzed as shown for example in Fig. 3. The selected examples have shown preferred compositions with an ideal mechanical strength containing a water insoluble solid filler to water insoluble polymer ratio of about 1,5 : 1 (grey bar in Fig. 3).

Further surprisingly the inventors found that calcium phosphate cements such as a calcium phosphate cement consisting of $\alpha$-TCP (62.5 wt%), $CaHPO_4$ (26.8 wt%), $CaCO_3$ (8.9 wt%), hydroxyapatite (1.8 wt%), $\alpha$-TCP with various concentrations of hydroxyapatite or $\alpha$-TCP can further improve the mechanical properties of the *in situ* hardening paste, compared to formulations, containing only $\beta$-TCP as an inorganic filler (Fig. 5, 6). This also includes that the amount of hydroxyapatite as seed crystal can be varied at least between 0 % and 2 % resulting in an IFS with strong mechanical properties.

In a further embodiment the alpha-tricalcium phosphate particle size of the *in situ* hardening paste is equal to or greater than 300 $\mu$m, more preferably equal to or greater than 500 $\mu$m, most preferably between 500 $\mu$m and 1000 $\mu$m.

Unexpected the inventors found that the particle size of the tricalcium phosphate has a further impact on the mechanical performance of the *in situ* hardening paste (Fig 7), where an increase of the particle size of $\alpha$-TCP further improves the mechanical properties of the *in situ* hardening paste. An increase in particle size to 300 $\mu$m or more further increases the mechanical properties.


**E The water soluble pore building filler**

The term "water soluble pore building filler" means a compound which is pharmaceutical acceptable and swellable or soluble in aqueous fluid such as water or body fluid which when added to the *in situ* hardening paste increases the number and size of micro- and macropores within *the in situ* hardening solid implant *ex vivo* and in the organism. The porosity of the solid implant formed will be increased dependent on the amount of water soluble pore building filler used. The water soluble pore building filler used increases the number of pores preferably macropores of a size sufficient for ingrowth of living cells within the interior of the *in situ* hardening solid implant preferably the outer layer of the implant.

In a preferred embodiment the water soluble pore building filler is an anti-skin forming agent such as an agent which reduces or eliminates a skin formation around the hardening paste when the *in situ* hardening paste is being placed into a defect and gets in contact with an aqueous fluid or body fluid. The phrase "water soluble pore building filler" is meant to include the formation of macropores not only in the interior of the *in situ* formed scaffold but also within the outer surface or skin to enable ingrowth of cell and replacement of the material with new bone formation.

Water soluble pore building filler include pharmaceutical acceptable compounds which dissipate from the *in situ* implant and thereby result in pore formation within the implant.

In one embodiment water soluble pore building fillers are swelling agents. In contact with aqueous media these excipients increase their volume and dissolute into the surrounding fluid leaving behind a porous interconnected structure. By using different amounts of water soluble pore building filler the porosity of the *in situ* hardening paste can be adopted and the amount and size of pores be regulated. An advantage of using swelling agents as water soluble pore building fillers in comparison to pore building fillers such as salts (e.g., sodium chloride) is that a reduced amount of these materials is effective since the volume will be increased due to water uptake. Other swelling agents according to the present invention are blasting agent's known to experts in the field from manufacturing of tablets.

Swelling agents of the present invention include such as sodium alginate, amylase, amylopectine, starch, hyaluronic acid, sodium hyaluronate, gelatine, collagen, carboxymethylcellulose, methylcellulose, carboxymethylcellulose calcium salt, carboxymethylcellulose calcium salt, hydroxylprorpyl methylcellulose, hydroxybutylmethylcellulose, hydroxyethylcellulose, hydroxyethylcellulose, or methylhydroxyethylcellulose.

In another embodiment water soluble pore building fillers are surfactants, preferably block copolymers of ethylene oxide/sorbitan and propylene oxide such as Pluronics® or Tween® 80 (e.g., Polysorbate 80; Montanox® 80; Polyoxyethylene sorbitan monooleate). These pore building fillers introduce a porosity by dissolution into aqueous media analogous to sodium chloride (salt leeching effect) but can also stabilize the forming porous structure. Theis effect is related to their amphipathic (amphiphilic) nature and surface activity and these materials therefore are widely used as froth-stabilizing agents.

In another embodiment suitable water soluble pore building filler that can be used in the present invention include porogenic substances such as sugars or salts of crystal size, which will provide pores when dissolved in the implant *in situ*.

Other pore forming agents form $CO_2$ gas bubbles and thereby leaving pores when moved from the implant.

The skilled person knows which parameters can be set to realize pore sizes of more than 100 $\mu$m, preferably 100 to 500 $\mu$m with the above pore building fillers. These pore sizes are not only present in the interconnecting pores, but also on the surface of the *in situ* hardening paste to allow ingrowths of cells of cellular surroundings at the site of the implant (Fig. 15).

Preferably the weight percentage of the water soluble pore building filler is less than 10 wt%, more preferably less than 5 wt%, even more preferably less than 2,5 wt%, most preferably less than 1 wt%.

More preferably the water soluble pore building filler is a carboxymethylcellulose salt, most preferably a carboxymethylcellulose sodium salt, optimally with a particle size less than 1000 $\mu$m, more preferably with a particle size 25 to 1000 $\mu$m. Preferably the weight percentage of the carboxymethylcellulose sodium salt is less than 10 wt%, more preferably less than 5 wt%, even more preferably less than 2,5 wt%, most preferably equal or less than 1 wt%.

The term "particle size" according to the present invention means an average distribution of the size diameter of the material such as tricalcium phosphate and carboxymethylcellulose in microns ($\mu$m), which can be determined by laser diffraction. A specific particle size range of material can for example be achieved by sieving.

It was further unexpected that carboxymethylcellulose overcomes the hurdles of an non-macroporous outer skin formation, preferably carboxymethylcellulose with a particle size diameter between 25 and 1000 $\mu$m significantly increased the number of macropores greater than 200 $\mu$m within the outer surface of the in situ hardened paste after contact with an aqueous medium as shown in Table 3. A further unexpected observation in terms of porosity in the skin relates to the organic solvent used in combination with the water soluble pore building filler. The data presented in Fig. 14 demonstrate an advantageous porosity in the skin when using PEG such as PEG 400 as a plasticizer compared to NMP.

By using different concentrations of the pore building filler the number of macropores can be adjusted according to the application of the *in situ* hardening paste.

In another embodiment the *in situ* hardening paste has interconnecting pores with a diameter of equal or more than 100 $\mu$m.

The term "interconnecting pores" means a network of pores and pore channels with micro-and macropores throughout the implant most preferably macropores and macrochannels creating a porosity with a pore size sufficient for cell infiltration such as bone cell or precursor cells. According to the present invention, an *in situ* hardening paste could be developed with a network of such interconnecting pores supporting the ingrowth of living cells for new bone formation (Fig. 15).

**F The active agent**

The term "active agent" comprises a polypeptide or a small molecule drug, which is immobilized on the water insoluble solid filler and/or dissolved or suspended in the plasticizer. Preferably, said polypeptide or drug is homogeneously distributed on the calcium phosphate containing carrier and / or within the polymer.

The term "coated" of the present invention means that the surface of the water insoluble solid filler is entirely coated with the active agent, whereby essential identical amounts of protein are present in each and every area of the surface of said carrier. A homogeneously coated carrier in accordance with this invention, preferably, exhibits a maximum covering with the osteoinductive protein on its surface. It is to be understood that the osteoinductive polypeptides are not aggregated and partially or entirely inactivated due to precipitation or micro-precipitation.

The term "homogeneously coated" or "homogeneously distributed" means that the active agent is homogeneously distributed within the respective phase coincides to the description of the methods of embodiments (32) and (33). This can be achieved by the characteristic of the active agent that either dissolves in the water soluble polymer or adsorbes to the water insoluble filler leading to a suspension of the coated water insoluble filler in the water soluble polymer. Homogenous distribution is a prerequisite for efficient release and activity of the active agent into the tissue surrounding at the site of implantation. Moreover, it is to be understood that the active agent is not aggregated and partially or entirely inactivated due to precipitation or micro-precipitation, rather attachment of biologically active, non-aggregated proteins is to be achieved by homogenous coating.

The homogenous coating of the carrier with said active agent and the simultaneous and / or additional distribution of the carrier within the water insoluble polymer achieves a structure which acts in two manners: as a protection of the active agent and as a diffusion barrier to slow down the dissolution of the protein or peptide to achieve a sustained release. The described methods (see (32) and (33)) allow the homogenous distribution and immobilization of the osteoinductive active agent into and / or on the carrier and the sustained release of the active agent due to the polymeric component as shown in Fig. 17.

The efficacy of the coating process is, furthermore, supported by the carrier due to capillary forces resulting from the presence of numerous, preferably interconnected macro- and micropores which due to their size are capable of soaking the solutions into the pores.

Moreover, in contrast to other methods described in the art, e.g., in WO98/21972, the active agent is - according to the methods of the present invention - applied by attachment to the carriers from the soluble state to achieve a homogeneous coating.

The findings underlying the present invention demonstrate that the aggregation of the proteins can be avoided in a tri-component-system by the use of suitable additives as described herein. An important precondition is the knowledge of the solubility of the osteoinductive active agent dependent on the nature of the solvent, i.e. aqueous and / or organic solvent, pH value, ionic strength and surfaces present. For method (33), the active agent is homogeneously onto the carrier. This homogeneous distribution is performed as described in e.g. in WO03/043673 using an aqueous solution and a buffer. The term "aqueous solution" specifies any solution comprising water. The slowing down of the pH increase caused by the contact of the coating solution with the calcium phosphates in the carrier reacting in an alkaline manner, in particular, plays an important role during the coating.

The methods (32) and (33) of the present invention, distribute the active agent homogeneously across the inner surface of the carrier material and allow binding to the surface before a precipitation of the said protein takes place within the coating solution. For method B, it could be demonstrated that the pH increase taking place during the coating of calcium phosphates is decelerated sufficiently by the use of a weak acid, such as acetic acid. Furthermore, the addition of organic compounds such as ethanol or sucrose proves to be additional advantageous. Furthermore, a low ionic strength is an important precondition for successful coating of the protein or peptide onto the calcium phosphate. Moreover, our tests show that the volume of the coating solutions (solution containing active agent and / or polymer), too, has a considerable effect on the quality of both coatings.

The term "osteoconductive" refers to substrates that provide a favourable porous scaffolding for vascular ingress cellular infiltration and attachment, cartilage formation, and calcified tissue deposition. Osteoconductive materials may support osseous generation via the scaffolding effect (Kenley et al., 1993)

The term "osteoinductive" refers to the capability of the transformation of mesenchymal stem cells into osteoblasts and chondrocytes. A prerequisite for osteoinduction is a signal, which is distributed by the paste into the surrounding tissues where the aforementioned osteoblast precursors become activated. Osteoinduction as used herein encompasses the differentiation of mesenchymal cells into the bone precursor cells, the osteblasts. Moreover, osteoinduction also comprises the differentiation of said osteoblasts into osteocytes, the mature cells of the bone. Moreover, also encompassed by osteoinduction is the differentiation of mesenchymal cells into chondrocytes. In particular in the long bones, the chondroblasts and the chondrocytes residing in the perichondrium of the bone can also differentiate into osteocytes. Thus, osteoinduction requires differentiation of undifferentiated or less-differentiated cells into osteocytes, which are capable of forming the bone. Thus, a prerequisite for osteoinduction is a signal, which is distributed by the paste into the surrounding tissues where the aforementioned osteocyte precursors usually reside. As has been described above, the osteoinductive proteins or peptides used in accordance with the present invention are sustained released from the paste, once said paste has *in situ* formed a hard scaffold after implantation and said osteoinductive proteins or peptides are subsequently distributed efficiently in the surrounding tissues as shown in Fig. 17.

The term "osteogenic" describes the synthesis of new bone by osteoblasts. In accordance with the present invention, preexisting bone in the surrounding at the side of implantation of the paste grows into the hardened paste using the structure of the hardened paste, especially formed during the hardening process, as a matrix onto which cells (e.g., bone cells) can adhere.

The proteins and peptides encompassed in the *in situ* hardening paste of the present invention have osteoinductive properties *in vivo.* For example, it is well known in the art that the Transforming Growth Factor-β (TGF-β) superfamily encompasses members, which have osteoinductive properties. Individual members of said TGF-β superfamily, which have particular well osteoinductive properties are listed infra. In conclusion, the osteoinductive proteins or peptides of the paste of the present invention after having been released from the carrier serve as an osteoinductive signal for the osteocyte precursors of the tissue surrounding the side of implantation of the paste.

The term "osteoinductive polypeptide" refers to polypeptides, such as the members of the Transforming Growth Factor-β (TGF-β) superfamily, which have osteoinductive properties.

In a further preferred embodiment of the paste or the method of the invention said osteoinductive protein is a member of the TGF-β family.

The TGF-β family of growth and differentiation factors has been shown to be involved in numerous biological processes comprising bone formation. All members of said family are secreted polypeptides comprising a characteristic domain structure. On the very N-terminus, the TGF-β family members comprise a signal peptide or secretion leader. This sequence is followed at the C-terminus by the prodomain and by the sequence of the mature polypeptide. The sequence of the mature polypeptide comprises seven conserved cysteins, six of which are required for the formation of intramolecular disulfide bonds whereas one is required for dimerization of two polypeptides. The biologically active TGF-β family member is a dimer, preferably composed of two mature polypeptides. The TGF-β family members are usually secreted as proteins comprising in addition to the mature sequence the prodomain. The prodomains are extracellularly cleaved off and are not part of the signaling molecule.

In the context of the present invention, the term "TGF-β family member" or the proteins of said family referred to below encompass all biologically active variants of the said proteins or members and all variants as well as their inactive precursors. Thus, proteins comprising merely the mature sequence as well as proteins comprising the mature protein and the prodomain or the mature protein, the prodomain and the leader sequence are within the scope of the invention as well as biologically active fragments thereof. Whether a fragment of a TGF-β member has the biological activity can be easily determined by biological assays described, e.g. in: Katagiri T, Yamaguchi A, Ikeda T, Yoshiki S, Wozney JM, Rosen V, Wang EA, Tanka H, Omura S, Suda T, (1990): The non-osteogenic mouse pluripotent cell line, C3H10T1/2, is induced to differentiate into osteoblastic cells by recombinant human bone morphogenetic protein-2. Biochem. Biophys. Res. Commun. 172: 295-299 or Nishitoh H, Ichijo H, Kimura M, Matsumoto T, Makishima F, Yamaguchi A, Yamashita H, Enomoto S, Miyazono K (1996): Identification of type serine/ threonine kinase receptors for growth/ differentiation factor-5. J. Biol. Chem. 271: 21345-21352).

Preferably, *in vivo* models as described in the accompanied examples can determine the biological activity according to the invention. Furthermore, encompassed by the present invention are variants of the TGF-β members which have an amino acid sequences being at least 75 %, at least 80 %, at least 90 %, at least 95 %, at least 96 %, at least 97 %, at least 98 % or at least 99 to identical to the amino acid sequences of the members of the TGF-β family.

An overview of the members of the TGF-β superfamily is given in: Wozney JM, Rosen V (1998): Bone morphogenetic protein and bone morphogenetic protein gene family in bone formation and repair. Clin Orthop 346: 26-37. The amino acid sequences of the members of the TGF-β family can be obtained from the well known databases such as Swiss-Prot via the internet (http://www.expasy.ch/sprot/sprot-top.html) Amino acid sequences for BMP-2, BMP-7 and GDF-5, members of the TGF-family with a particularly high osteoinductive potential, are also shown in SEQ ID No: 1 to 3, respectively. Amino acid sequences for BMP-2, BMP-7 and GDF-5, members of the TGF-β family with a particularly high osteogenic potential, are also shown in SEQ ID No:1 to 3, respectively.

More preferably, said member of the TGF-β superfamily is a member of the BMP subfamily. The members of the Bone Morphogenetic Protein (BMP) subfamily have been shown to be involved, inter alia, in the induction and re-modeling of bone tissue. BMPs were originally isolated from bone matrix. These proteins are characterized by their ability to induce new bone formation at ectopic sites. Various *in vivo* studies demonstrated the promotion of osteogenesis and chondrogenesis of precursor cells by BMPs and raise the possibility that each BMP molecule has distinct role during the skeletal development. More details about the molecular and biological properties of the BMPs are described in: Wozney JM, Rosen V (1998): Bone morphogenetic protein and bone morphogenetic protein gene family in bone formation and repair. Clin Orthop 346: 26-27, Schmitt J, Hwang K, Winn, SR, Hollinger J (1999): Bone morphogenetic proteins: an update on basic biology and clinical relevance. J Orthop Res 17: 269-278 and Lind M (1996): Growth factors: possible new clinical tools. A review. Acta Orthop Scand 67: 407-17.

The osteoinductive polypeptide of the present invention is preferably selected from the group consisting of BMP-1, BMP-2, BMP-3, BMP-4, BMP-5, BMP-6, BMP-7, BMP-8, BMP-9, BMP-10, BMP-11, BMP-12, BMP-13, BMP-14, BMP-15 and BMP-16. Most preferably, said member of the BMP family is BMP-2 or BMP-7.

The amino acid sequence for the preproform of BMP-2 is deposited under Swiss-Prot Accession number P12643 and is shown below. Amino acids 1 to 23 correspond to the signal sequence, amino acids 24 to 282 correspond to the propeptide and amino acids 283 to 396 correspond to the mature protein. The amino acid sequence for the preproform of BMP-7 is deposited under Swiss-Prot Accession number P18075 or shown in SEQ ID No: 2. Amino acids 1 to 29 correspond to the leader sequence, amino acids 30 to 292 correspond to the proform and amino acids 293 to 431 correspond to the mature protein. Preferably, BMP-2 or BMP-7 refers to the preproform, to the proform or to the mature BMP-2 or BMP-7 peptide, respectively. Moreover also encompassed are fragments of said proteins having essentially the same biological activity, preferably osteoinductive properties. More sequence information for BMP-2 and BMP-7 is provided below.

Alternatively, the osteoinductive polypeptide of the present invention is selected from another TGF-β family, i.e. the GDF family.

Growth and Differentiation Factor (GDF) have been also shown to be involved, inter alia, in the induction and re-modeling of bone tissue. Growth Differentiation Factor 5 (GDF-5), also known as cartilage-derived morphogenetic protein 1 (CDMP-1) is a member of a subgroup of the BMP family, which also includes other related proteins, preferably, GDF-6 and GDF-7. The mature form of the protein is a 27 kDa homodimer. Various *in vivo* and *in vitro* studies demonstrate the role of GDF-5 during the formation of different morphological features in the mammalian skeleton. Mutations of GDF-5 are responsible for skeletal abnormalities including decrease of the length of long bones of limbs, abnormal joint development in the limb and sternum (Storm & Kingsley (1999), Development Biology, 209, 11-27). The amino acid sequence between mouse and human Is highly conserved.

Preferably, the osteoinductive polypeptide of the present invention is selected from the group consisting of GDF-1, GDF-2, GDF-3, GDF-4, GDF-5, GDF-6, GDF-7, GDF-8, GDF-9, GDF-10 and GDF-11. Most preferably, said member of the GDF subfamily is GDF5.

The amino acid sequence for the preproform of GDF-5 is deposited under Swiss-Prot Accession number P 43026 or shown in SEQ ID No: 3. Amino acids 1 to 27 correspond to the leader sequence, amino acids 28 to 381 correspond to the proform and amino acids 382 to 501 correspond to the mature protein. Preferably, GDF-5 refers to the preproform, to the proform or to the mature GDF-5 peptide. Moreover also encompassed are fragments of GDF-5 having essentially the same biological activity, prefrably osteoinductive properties. Most preferably, said fragment comprises amino acids 383 to 501 of the sequence shown in SEQ ID No: 3.

The following tables show amino acid sequences for BMP-2, BMP-7 and GDF-5:

Human BMP-2 (Swiss-Prot Prim. Accession Number P12643); SEQ ID No. 1:

```
Key
From      To
Length


SIGNAL
1         23
23


PROPEP
24        282
259


hBMP2
283       396
114
```

```
            10          20          30          40          50          60
             |           |           |           |           |           |
        MVAGTRCLLA  LLLPQVLLGG  AAGLVPELGR  RKFAAASSGR  PSSQPSDEVL  SEFELRLLSM

            70          80          90         100         110         120
             |           |           |           |           |           |
        FGLKQRPTPS  RDAVVPPYML  DLYRRHSGQP  GSPAPDHRLE  RAASRANTVR  SFHHEESLEE

           130         140         150         160         170         180
             |           |           |           |           |           |
        LPETSGKTTR  RFFFNLSSIP  TEEFITSAEL  QVFREQMQDA  LGNNSSFHHR  INIYEIIKPA

           190         200         210         220         230         240
             |           |           |           |           |           |
        TANSKFPVTR  LLDTRLVNQN  ASRWESFDVT  PAVMRWTAQG  HANHGFVVEV  AHLEEKQGVS

           250         260         270         280         290         300
             |           |           |           |           |           |
        KRHVRISRSL  HQDEHSWSQI  RPLLVTFGHD  GKGHPLHKRE  KRQAKHKQRK  RLKSSCKRHP

           310         320         330         340         350         360
             |           |           |           |           |           |
        LYVDFSDVGW  NDWIVAPPGY  HAFYCHGECP  FPLADHLNST  NHAIVQTLVN  SVNSKIPKAC

           370         380         390
             |           |           |

        CVPTELSAIS  MLYLDENEKV  VLKNYQDMVV  EGCGCR
```

References

[1]
SEQUENCE FROM NUCLEIC ACID.
MEDLINE=89072730; PubMed=3201241;
Wozney J.M., Rosen V., Celeste A.J., Mitsock L.M., Whitters M.J., Kriz R.W., Hewick R.M., Wang E.A.;"Novel regulators of bone formation: molecular clones and activities.";Science 242:1528-1534(1988).

[2]
X-RAY CRYSTALLOGRAPHY (2.7 ANGSTROMS) OF 292-396.
MEDLINE=99175323; PubMed=10074410;
Scheufler C., Sebald W., Huelsmeyer M.;"Crystal structure of human bone morphogenetic protein-2 at 2.7 A resolution.";J. Mol. Biol. 287:103-115(1999).

Human BMP-7(Swiss-Prot Prim. Accession. Number: P18075); SEQ ID No. 2:

```
Key
From          To
Length


SIGNAL
1        29
29


PROPEP
30       292
263


hBMP-7
293      431
139
```

EP 1 763 374 B1

```
         10          20          30          40          50          60
          |           |           |           |           |           |
    MHVRSLRAAA  PHSFVALWAP  LFLLRSALAD  FSLDNEVHSS  FIHRRLRSQE  RREMQREILS

         70          80          90         100         110         120
          |           |           |           |           |           |
    ILGLPHRPRP  HLQGKHNSAP  MFMLDLYNAM  AVEEGGGPGG  QGFSYPYKAV  FSTQGPPLAS

        130         140         150         160         170         180
          |           |           |           |           |           |
    LQDSHFLTDA  DMVMSFVNLV  EHDKEFFHPR  YHHREFRFDL  SKIPEGEAVT  AAEFRIYKDY

        190         200         210         220         230         240
          |           |           |           |           |           |
    IRERFDNETF  RISVYQVLQE  HLGRESDLFL  LDSRTLWASE  EGWLVFDITA  TSNHWVVNPR

        250         260         270         280         290         300
          |           |           |           |           |           |
    HNLGLQLSVE  TLDGQSINPK  LAGLIGRHGP  QNKQPFMVAF  FKATEVHFRS  IRSTGSKQRS

        310         320         330         340         350         360
          |           |           |           |           |           |
    QNRSKTPKNQ  EALRMANVAE  NSSSDQRQAC  KKHELYVSFR  DLGWQDWIIA  PEGYAAYYCE

        370         380         390         400         410         420
          |           |           |           |           |           |
    GECAFPLNSY  MNATNHAIVQ  TLVHFINPET  VPKPCCAPTQ  LNAISVLYFD  DSSNVILKKY

        430
          |
    RNMVVRACGC  H
```

References

[1]
SEQUENCE FROM NUCLEIC ACID, AND PARTIAL SEQUENCE.
TISSUE=Placenta;
MEDLINE=90291971; PubMed=2357959;
Oezkaynak E., Rueger D.C., Drier E.A., Corbett C., Ridge R.J., Sampath T.K., Oppermann H.;"OP-1 cDNA encodes an osteogenic protein in the TGF-beta family."; EMBO J. 9:2085-2093(1990).

[2]
SEQUENCE FROM NUCLEIC ACID.
MEDLINE=91088608; PubMed=2263636;
Celeste A.J., Iannazzi J.A., Taylor R.C., Hewick R.M., Rosen v., Wang E.A., Wozney J.M.;"Identification of transforming growth factor beta family members present in bone-inductive protein purified from bovine bone.";Proc. Natl. Acad. Sci. U.S.A. 87:9843-9847(1990).

[3]
X-RAY CRYSTALLOGRAPHY (2.8 ANGSTROMS) OF 293-431.
MEDLINE=96149402; Pubmed=8570652;
Griffith D.L., Keck P.C., Sampath T.K., Rueger D.C., Carlson W.D.; "Three-dimensional structure of recombinant human osteogenic protein 1: structural paradigm for the transforming growth factor beta superfamily.";Proc. Natl. Acad. Sci. U.S.A. 93:878-883(1996).

Human GDF-5 (Swiss-Prot Prim. Accession Number: P 43026); SEQ ID No. 3:

```
Key
From            To
Length


SIGNAL
1         27
27


PROPEP
28        381
354


hGDF-5
382       501
120
```

```
          10          20          30          40          50          60
          |           |           |           |           |           |
MRLPKLLTFL LWYLAWLDLE FICTVLGAPD LGQRPQGSRP GLAKAEAKER PPLARNVFRP

          70          80          90         100         110         120
          |           |           |           |           |           |
GGHSYGGGAT NANARAKGGT GQTGGLTQPK KDEPKKLPPR PGGPEPKPGH PPQTRQATAR

         130         140         150         160         170         180
          |           |           |           |           |           |
TVTPKGQLPG GKAPPKAGSV PSSFLLKKAR EPGPPREPKE PFRPPPITPH EYMLSLYRTL

         190         200         210         220         230         240
          |           |           |           |           |           |
SDADRKGGNS SVKLEAGLAN TITSFIDKGQ DDRGPVVRKQ RYVFDISALE KDGLLGAELR

         250         260         270         280         290         300
          |           |           |           |           |           |
ILRKKPSDTA KPAVPRSRRA AQLKLSSCPS GRQPAALLDV RSVPGLDGSG WEVFDIWKLF

         310         320         330         340         350         360
          |           |           |           |           |           |
RNFKNSAQLC LELEAWERGR TVDLRGLGFD RAARQVHEKA LFLVFGRTKK RDLFFNEIKA

         370         380         390         400         410         420
          |           |           |           |           |           |
RSGQDDKTVY EYLFSQRRKR RAPLATRQGK RPSKNLKARC SRKALHVNFK DMGWDDWIIA

         430         440         450         460         470         480
          |           |           |           |           |           |
PLEYEAFHCE GLCEFPLRSH LEPTNHAVIQ TLMNSMDPES TPPTCCVPTR LSPISILFID

         490         500
          |           |
SANNVVYKQY EDMVVESCGC R
```

References

[1]
SEQUENCE FROM NUCLEIC ACID.
TISSUE=Placenta;
MEDLINE=95071375; Pubmed-7980526;
Hoetten G., Neidhardt H., Jacobowsky B., Pohl J.;"clowning and expression of recombinant human growth/differentiation factor 5.";Biochem. Biophys. Res. Commun. 204:646-652(1994).

[2]
SEQUENCE FROM NUCLEIC ACID. TISSUE=Articular cartilage:
MEDLINE=95050609: PubMed=7961761;
Chang S., Hoang B., Thomas J.T., Vukicevic S.. Luyten F.P., Ryba N.J.P., Kozak C.A., Reddi A.H., Moos M.;"Cartilage-derived morphogenetic proteins. New members of the transforming growth factor-beta superfamily predominantly expressed in long bones during human embryonic development.";J. Biol. Chem. 269:28227-28234(1994).

Also encompassed by the present invention are embodiments, wherein said active agent is selected from hormones, cytokines, growth factors, antibiotics and other natural and/or synthesized drug substances like steroids, prostaglandins etc.

Preferably, said active agent is parathyroid hormone (PTH) and/or PTH 1-34 peptide.

In another embodiment of the invention, the active agent is a "cartilage inductive" or "cartilage regenerating" protein. A preferred cartilage inductive protein is MIA/CD-RAP (MIA, melanoma inhibitory activity, cartilage derived-retinoic acid-sensitive protein), more preferably human MIA/CD-RAP.

The amino acid sequence for the preproform of MIAICD-RAP is deposited under Swiss-Prot Accession number Q 16674 or shown in SEQ ID No: 4. Amino acids 1 to 24 correspond to the signal sequence, amino acids 25 to 131 correspond to the coding sequence.

The following table shows the amino acid sequences for MIAICD-RAP:
Human MIA/CD-RAP (Swiss-Prot Prim. Accession Number: Q 16674); SEQ ID No. 4:

```
Key
From          To
Length


SIGNAL
1        24
24


h MIA/CD-RAP
25      131
107
```

MARSLVCLGVIILLSAFSGPGVRGGPMPKLADRKLCADQECSHPISMAVALQDYMAPDCR

FLTIHRGQVVYVFSKLKGRGRLFWGGSVQGDYYGDLAARLGYFPSSIVREDQTLKPGKVD

VKTDKWDFYCQ

**G Specific preparations and optional ingredients**

The paste of the present invention optionally, may comprise additional excipients. These excipients serve the stabilization of the protein or peptide, e.g., saccharides, amino acids, polyots, detergents or maintenance of the pH, e.g., buffer substances. Other ingredients of the *in situ* forming paste could be hardening promoters such as sodium phosphate, $Na_2HPO_4$ or sodium citrate and those described further above.

The methods (31) to (34) of the present invention allow not to use toxic organic solvents (see below), e.g., the use of dimethyl sulfoxide, anisol or glacial acid. Toxic solvents, however, are routinely used in the methods described in the art. Normally they damage the protein during contacting and/or especially during drying but such damage is surprisingly avoided by using the special drying technique of the present invention, because the active agent is adsorbed / or attached onto the inorganic solid carrier.

In a preferred embodiment of the method (33) of the invention said buffer has a buffer concentration of 10 mmol/l to achieve a sufficient solubility of the active agent during the adsorption process and to avoid any modification of the monophasic calcium ceramic beta-TCP. The pH value of the solution shifts controlled during coating and drying process from pH 3 to pH 7. The pH shift causes a reduction of the solubility of the bone growth factor homogenous, defined on the TCP.

The term " viscous liquid" means a liquid with a decreased flowability compared to water. For example a viscous liquid according to the present invention is related to a honey-like consistency at ambient temperature allowing simple administration.

In a further preferred embodiment of the method of the invention said buffer containing solution for method (33) comprises a polyol and/or alcohol. Suitable alcohols or polyols are well known in the art and are described in standard textbooks, such as Römpp, dictionary of chemistry. More preferably, said alcohol is ethanol and said polyol is mannitol.

In a more preferred embodiment the concentration of the polyol and or alcohol is between 0 -and 10 % (w/v).

The term "saccharides" encompasses mono-, di- and polysaccharides. The structure and composition of mono-, di-, and polysaccharides are well known in the art and are described in standard textbooks, such as Römpp, dictionary of chemistry.

More preferably, said saccharide is a disaccharide. Most preferably, said dissaccharide is sucrose or trehalose.

Further means and methods for controlling homogeneous distribution, quantification and characterization of the active agent are described in the accompanied examples.

Surprisingly, active agents, in particular surprisingly proteins, when adsorbed on the surface of ceramic fillers / carriers are much more resistant against degradation caused by organic solvents than proteins freely dissolved or suspended in organic solutions or integrated in biphasic emulsion systems. Thus, this aspect of the invention opens a new possibility to produce polymer based drug delivery systems for proteins without denaturation and / or modification of polypeptides in singular or multiphase organic systems.

Suitable for one of the two methods of the present invention as active agents are all proteins, polypeptides and small molecule drugs. Especially such active, agents with low or no affinity for inorganic carrier matrices can be immobilized in the polymer - calcium phosphate composite material. Preferably, the binding of said active agent to the carrier is reversible.

Thereby, dissolution of said active agent is allowed once the paste has been brought into a suitable *in vivo* surrounding, such as a bone cavity or into the intramuscular or subcutaneous aqueous environment. The *in situ* hardening paste hardens by diffusion of the plasticizer out of the water insoluble polymer while aqueous body fluids diffuse into the water insoluble polymer. In consequence the polymer solidifies due to its insolubility in water (Shively et al., 1995).

More preferably, said dissolution of the immobilized compounds is a sustained release allowing diffusion of the active agent into the tissue, which surrounds the hardened paste. Thus, the hardened paste is suitable to serve as an *in vivo* source for e.g. osteoinductive proteins, peptides, antibiotics or small molecule drugs, which are slowly released and which can be thereby efficiently distributed into the surrounding tissues or have an effect in the immobilized form.

Water insoluble polymer and filler together with the plasticizer form the carrier/polymer composite paste material of the present invention, which binds an active agent to result in the paste of the present invention, which allows the sustained release of said active agent *in vivo,* once the paste is hardened within minutes to hours by diffusion out of the water soluble polymer (plasticizer) while aqueous body fluids diffuse into the polymer. In consequence the polymer solidifies due to its insolubility in water (Shively et al., 1995). The time until the full mechanical stability is reached can be controlled by the variation of the ingredients of the implant material.

The term "buffer" which assists in keeping the active agent dissolved in aqueous solutions for a time sufficient to allow "homogenous coating refers to a component allowing the osteoinductive active agent to be efficiently dissolved and kept in the solution. This buffer is capable of avoiding and or balancing the increase of pH caused by contacting the solutions with the calcium phosphate carrier so that the protein does not immediately precipitate, e.g., due to a pH increase. Said buffer can be determined by the person skilled in the art considering the solubility of the osteoinductive protein (which depends on the pH and the ionic strength) and the influence of the carrier on said parameters after contacting the carrier with said buffer containing solution. In accordance with the present invention it has been found that a suitable buffer is needed for the homogeneous distribution of the active agent onto the surface of the carrier, e.g. calcium phosphate, said buffer comprising preferably a weak acid, an alcohol and a saccharide.

**H Pharmaceutical applications**

The invention encompasses a pharmaceutical composition comprising the paste of the invention or a paste, which is obtainable by the method of the invention.

The product of the present invention can be formulated as a pharmaceutical composition or a medical device.

The composition of said product may comprise additional compounds like stabilizers, buffer substances and other excipients. The amount of the product of the present invention applied to the patient will be determined by the attending physician and other clinical factors; preferably in accordance with any of the above described methods. As it is well known in the medical arts, the amount applied to a patient depends upon many factors, including the patient's size, body surface area, age, sex, time and route of administration, general health conditions, and other drugs being administered concurrently. Progress can be monitored by periodic assessment. Thanks to the present invention it's possible to have a system, which allows to care for dental or orthopedic defects by optimum adaptation of the paste-like material to the implantation site and at the same time a controlled release of a active agent.

Preferably the application method is minimal invasive even more preferable the application is applied by injection.

Thanks to the present invention, it is possible to treat various bone defects including large cavities in a new manner. In particular, large cavities could not or only under use of autogenous bone material be efficiently treated. However, due to the reliable and efficient osteoinductive and/or the osteoconductive properties of the paste of the present invention or the device which can be obtained by the method of the invention, treatment of bone defects which requires extensive bone augmentation or repair has now become possible without a second surgery.

The invention also encompasses the use of the paste of the invention or a paste which is obtainable by the method of the invention for the preparation of a pharmaceutical composition for bone augmentation.

The term "bone augmentation" refers to the induced formation of bone, which is indicated in order to treat bone defects, cavities in bones, or to treat diseases and disorders accompanied with loss of bone tissue or to prepare the subsequent setting of an implant. The diseases and disorders described in the following are well known in the art and are described in detail in standard medical textbooks such as Pschyrembel or Stedman.

Preferably, said bone augmentation follows traumatic, malignant or artificial defects.

Another embodiment of the present invention relates to the use of the paste of the invention or the preparation of a pharmaceutical composition for treating bone defects.

More preferably, said bone defects includes fractures such as radius fracture and tibia head fracture, non-fracture repair, spinal fusion, long bone defects or bone defects following apicoectomy, extirpation of cysts or tumors, tooth extraction, or surgical removal of retained teeth.

The invention also relates to the use of the paste of the invention for filing of cavities and support guided tissue regeneration in periodontology.

Another embodiment of the present invention relates to the use of the paste of the invention for the preparation of a pharmaceutical composition for sinus floor elevation, augmentation of the atrophied maxillary and mandibulary ridge and stabilisation of immediate implants.

Also within the scope of the present invention is a method for treating one or more of the diseases referred to in accordance with the uses of the present invention, wherein said method comprises at least the step of administering the paste of the invention in a pharmaceutically acceptable form to a subject. Preferably, said subject is a human.

Finally, the invention relates to a kit comprising the paste of the invention, Ingredients of the kit are described further above.

The parts of the kit of the invention can be packaged Individually in vials or other appropriate means depending on the respective ingredient or in combination in suitable containers or multicontainer units. Manufacture of the kit follows preferably standard procedures, which are known to the person skilled in the art.

**I) Benefits of the in situ hardening paste of the present invention**

According to the present invention an *in situ* hardening paste could be developed with excellent washout resistance due to rapid setting, which allows the premixed paste such as a CPC containing paste to be form stable In a wet environment (e.g. indications with severe bleeding) before hardening is complete. These premixed pastes according to the present invention have the advantage that there is unlimited time for application and modulation and the polymer containing the water Insoluble solid filler begins to harden after it becomes exposed to water or body fluid from surrounding tissue.

Thanks to the present invention, a moldable preferably injectable, self hardening and resorbable *in situ* hardening paste could be generated avoiding the disadvantages such as brittleness of CPC compositions with a better mechanical property than conventional CPC compositions.

Preferably with the selection of the plasticizer, such as PEG, 400, PEg 200, PEg 300 PEg600 or glycerol the mechanical strength could be further improved after dissipation of the plasticizer. The inventors found that dependent on the polymer used the plasticizer has an impact on the glass transition temperature of the polymer in the hardened implant, which influences the mechanical strength of the *in situ* hardening paste. This impact is different for different plasticizers used. Surprisingly, PEG was the plasticizer with the lowest impact on the polymer used (Fig. 9). DMSO, which is a preferred plasticizer for dissolving block copolymers due to there solubility characteristics is comparable to PEG concerning the impact on the lowering of the glass transition temperature of the polymer and therefore the mechanical strength of the *in situ* hardening paste.

Furthermore, the ceramic/polymer composite paste of the present invention shows improved mechanical stability compared to conventional flowing systems using polymeric matrices for retarded release.

Preferably the *in situ* hardening paste has a hardness of 20 % within one hour determined by the method well known to the expert in the field and further described in Example 12, more preferably 30 % after one hour, most preferably 40 % within one hour after contact with an aqueous medium.

According to the present invention the *in situ* hardening paste is superior over conventional flowing systems such as CPC (Fig. 13 white bar) or calcium phosphate free polymer systems (Fig. 13 grey bar) concerning the initial mechanical strength within one hour after having been in contact with an aqueous media or body fluid as shown in Fig. 13.

The ceramic/polymer composite carrier material of the present invention shows improved mechanical stability compared to conventional systems using polymeric matrices for retarded release. In porous embodiments the composite matrix allows for improved osteocoriductive properties compared to prior art systems due to the porous system, in particular free of interconnected pores.

The ceramic/polymer composite carrier material of the present invention is suitable to replace conventional encapsulating polymeric granules important for retarded release. Due to the content of the ceramic carrier of the system (ceramic carrier plus polymer coating), the amount of polymer can be significantly reduced compared with totally polymer based conventional flowing systems using polymeric matrices. The reduced amount of polymer leads to a reduced risk of cytotoxicity. A further aspect of the invention is the increased mechanical stability of the composite material compared with totally polymer based scaffolds.

The invention will now be described by reference to the following examples which are merely illustrative and which shall not limit the scope of the present invention.

## Examples

### Example 1: Coating of beta-TCP powder with rhGDF-5

333 mg beta-TCP and 147 μl rhGDF-5 in 10 mM HCl (3,4 mg/ml) was pipetted on the beta-TCP and absorbed. The damp powder was incubated for ca. 1 hour at 25°C and dried in a subsequent lyophilization step.

### Example 2: Preparation of an IFS (rhGDF-5/ beta-TCP / PLGA/ PEG 400)

167 μl PEG 400 and 83 mg PLGA were dissolved by gentle warming to get a viscous solution. After cooling at room temperature 333 mg coated rhGDF-5 beta-TCP powder was added under continuous stirring to get a homogenous paste.

### Example 3: Preparation of the IFS (beta-TCP / PLGA/PEG 400)

1 ml PEG 400 and 500 mg PLGA are dissolved by gentle warming to get a viscous solution. After cooling at room temperature the 2000 mg beta-TCP powder was added under continuous stirring to get a homogenous paste.

### Example 4: Extraction of the immobilised protein

### (step A)

Extraction of 417 mg hardened test sample (1h at 4 °C) in 1 ml chloroform solution to dissolve the polymer. After removing the chloroform-polymer solution the residual chloroform was removed in a desiccator under vacuum.

### (step B)

For the extraction of the immobilised protein from the beta-TCP the resulting sample from step A (coated and polymer free granules) were immersed in 8M Urea, 10mM Tris, 100mM EDTA for 1 h at 4 °C.

In the subsequent step the solution was centrifuged (9300* g, 3 min). The protein content and the degradation products in the supernatant were quantified by RP-HPLC.

### Example 5: Preparation of test samples

The hardening of the test samples takes place by replacement of the PEG with water from the body fluid. Under simulated physiological conditions the body fluid was represented by phosphate buffered saline (PBS). After casting the paste into a hollow mould subsequently the paste was hardened by incubating the hollow mould in PBS during gentle agitation of the PBS to support the diffusion of PEG away from the test sample. The final hardness was reached already after three hours at room temperature.

**Example 6: Improved mechanical properties by variation of beta -TCP-grain size**

Different grain sizes were used:

- beta-TCP approximately 10-100 $\mu$m
- beta-TCP approximately 100-300 $\mu$m
- beta-TCP approximately 10 $\mu$m

**Example 7: Improved mechanical properties by variation of the polymer content**

The different pastes are prepared by variation of the method described in example 3. To manufacture test samples the paste was hardened according example 5. The mechanical stability war determined as described in example 9.

Table 1: composition of the paste:

| 14 % PLGA | 29 % PEG | 57 % beta-TCP |
|-----------|----------|---------------|
| 18 % PLGA | 29 % PEG | 53 % beta-TCP |
| 21 % PLGA | 29 % PEG | 50 % beta-TCP |
| 25 % PLGA | 29 % PEG | 46 % beta-TCP |

Table 2: composition of hardened test samples:

| 20 % % PLGA | 80 % beta-TCP |
|-------------|---------------|
| 25 % PLGA | 75 % beta-TCP |
| 30 % PLGA | 60 % beta-TCP |
| 35 % PLGA | 55 % beta-TCP |

**Example 8: Improved mechanical properties by variation of the polymer content**

Different PLGA qualities were used to manufacture the test bodies

| PLGA type | molecular weight [g/mol] |
|-----------|--------------------------|
| 20% PLGA (Resomer® RG 503 H) | 34000 |
| 20 % PLGA (Resomer® RG 502 H) | 18600 |

**Example 9: Determination of the pore size and pore orientation**

During hardening of the test samples according example 5, the structured porosity is formed. The pore size and the pore orientation were determined by the composition of the paste by the content of the water soluble plasticizer. Various formulations were tested and analyzed. The pore size and structure were analyzed by light microscopy an by raster electron microscopy of various cross sections through the test samples.

The structured interconnecting pores were formed in the direction to surrounding tissue. The pore size was determined. The structured pores enable a leaded cell rapid movement into the core of the scaffold to support the replacement and accelerated vascularization with new tissue.

**Example 10: Selection different analysed compositions of the IFS**

| polymer | [% (wt)] | organic solvent | [% (wt)] | Inorganic ' filler | [% (wt)] | pore forming agent | [% (wt)] |
|---|---|---|---|---|---|---|---|
| PLGA RG 503H | 11:0 | PEG 400 | 44.5 | CPC | 44.5 | - | - |
| | 16.0 | | 60.0 | | 24.0 | | |
| | 19.4 | | 44.5 | | 36.1 | | |
| | 20.0 | | 50.0 | | 30.0 | | |
| | 22.2 | | 44.5 | β-TCP | 33.3 | | |
| | | | 44.5 | | 33.3 | | |
| | 22.0 | | 44.0 | α-TCP | 33.0 | CMC | 1.0 |
| | | | 44.5 | CPC | 33.3 | - | - |
| | 22.0 | | 44.1 | | 33.0 | PEG 4000 powder | 0.9 |
| | 22.0 | | 44.1 | | 33.0 | PEG 4000 solved | 0.9 |
| | 21.5 | | 43.2 | | 32.3 | PEG 4000 solved | 3.0 |
| | 22.0 | | 44.1 | | 33.0 | PEG 10000 powder | 0.9 |
| | 22.0 | | 44.1 | | 33.0 | PEG 10000 solved | 0.9 |
| | 21.5 | | 43.2 | | 32.3 | PEG 10000 solved | 3.0 |
| | 22.0 | | 44.1 | | 33.1 | NMP | 0.8 |
| | 21.1 | | 42.3 | | 31.6 | CaSO4 | 5.0 |
| | 18.4 | | 37.0 | | 27.6 | NaCl | 17.0 |
| | 18.4 | | 37.0 | | 27.6 | sucrose | 17.0 |
| | 22.0 | | 44.0 | | 33.0 | Chitosan | 1.0 |

(continued)

| polymer | [% (wt)] | organic solvent | [% (wt)] | Inorganic ' filler | [% (wt)] | pore forming agent | [% (wt)] |
|---|---|---|---|---|---|---|---|
| | 21.6 | | 43.4 | | 32.5 | | 2.5 |
| | 21.1 | | 42.3 | | 31.6 | | 5.0 |
| | 22.0 | | 44.0 | | 33.0 | PVP powder | 1.0. |
| | 21.6 | | 43.4 | | 32.5 | PVP powder | 2.5 |
| | 21.1 | | 42.3 | | 31.6 | PVP powder | 5.0 |
| | 22.0 | | 44.0 | | 33.0 | Pluronic F68 | 1.0 |
| | 22.2 | | 44.4 | | 33.3 | Tween 80 | 0.1 |
| | 22.0 | | 44.0 | | 33.0 | | 1.0 |
| | 21.8 | | 43.7 | | 32.7 | CMC | 1.8 |
| | 21.6 | | 43.4 | | 32.5 | | 2.5 |
| | 21.1 | | 42.3 | | 31.6 | | 5.0 |
| | 25.0 | | | | 30.5 | - | - |
| | 38.8 | | | | 16.7 | | |
| polymer | [% (wt)] | organic solvent | [% (wt)] | Inorganic filler | [% (wt)] | pore forming agent | [%(wt)] |
| PLGA RG 503H | 21.5 | PEG 400 | 43.2 | | 32.3 | NaHCO$_3$/ NaH$_2$PO$_4$ | 1.0/2.0 |
| | 21.3 | | 42.7 | | 32.0 | | 2.0/2.0 |
| | 21.1 | | 42.3 | | 31.6 | | 2.0/3.0 |
| | 20.9 | | 41.8 | | 31.3 | | 3.0/3.0 |
| | 20.9 | | 41.8 | | 31.3 | | 4.0/2.0 |
| | 20.4 | | 40.9 | | 30.7 | | 5.0/3.0 |
| PLGA RG 503 | 22.2 | PEG 400 | | CPC | 33.3 | - | - |
| PLGA RG 502 | | | | | | | |
| PLGA RG 756 | | | 4.4.5 | | | | |
| PLGA-PEG diblock copolymer | 3.8 | DMSO | | | 44.1 | | |
| | 7.6 | | | | 47.9 | | |
| | 11.4 | | | | 51.7 | | |
| PLGA RG 503H | 22.2 | NMP | 44.5 | | 33.3 | - | - |

RG503H; PLGA; polymer composition: 48-52 mol% D,L-Lactide and 48-52 mol% Glycolide; inherent viscosity: 0,32-0,44 dl/g, 25 °C, 0,1 % in CHCl$_3$; (Boehringer, Ingelheim)

RG502H; PLGA; polymer composition: 48-52 mol% D,L-Lactide and 48-52 mol% Glycolide; inherent viscosity: 0,16-0,24 dl/g, 25 °C, 0,1 % in CHCl$_3$. (Boehringer, Ingelheim) RG503; PLGA; polymer composition: 48-52 mol% D,L-Lactide and 48-52 mol% Glycolide; inherent viscosity: 0,32-0,44 dl/g, 25 °C, 0,1 % in CHCl$_3$; (Boehringer, Ingelheim)

RG502; PLGA; polymer composition: 48-52 mol% D,L-Lactide and 48-52 mol% Glycolide; inherent viscosity: 0,16-0,24 dl/g, 25 °C, 0,1 % in CHCl$_3$; (Boehringer, Ingelheim)

## Example 11: Preparation of the IFS

Initially the obligate amount of organic solvent was weight in a porcelain crucible. In a second step the polymer was added. These two components were homogenized and were heated at a temperature of approximately 60°C until the polymer was completely solved in the organic solvent Subsequently the inorganic filler (e.g. TCP or a calcium phosphate cement for example which consisted of 62.5 % by weight alpha-tricalcium phosphate, 26.8 % by weight dicalcium phosphate anhydrous (DCPA), 8.9 % by weight calcium carbonate ($CaCO_3$), and 1.8 % by weight hydroxyapatite (HA) and optionally other excipients (e.g. pore forming agents like carboxymethylcellulose sodium salt) were dispersed in the polymeric solution.

**Example 12: Mechanical testing of the IFS (and related systems)**

The IFS in its pasty shape, prepared as described in example 11, was transferred in a syringe. Thereby the filling-up of the formulation into the wells of a 96-well plate was facilitated. Subsequently the 96-well plate, containing the pasty IFS specimens (150 - 200 mg per well), was transferred in an incubation bath, which was constantly remained at 37°C to simulate physiological conditions, whereas PBS-buffer serves as incubation media. At pure-defined times the 96-well plate was removed from the incubation bath to carry out the mechanical testing. Hardness of the specimens was tested by using a TH 2730 (Fa Thuemler). Substantially this machine consists of a metallic punching tool, which enables to apply compressive forces on the specimens and a LVDT-transducer, which serves to control and to measure the applied force and to determine the distance, covered during the measurement. Prior to testing the different specimens, the height ($h_1$) of a well, which does not contain any specimen, has to be defined. Therefore the starting point of the punching tool for the following measurements was fixed. The actual determination of hardness of the specimens encompasses two steps. In a first measurement the height of the particular specimen ($h_2$) has to be ascertained, whereas the crosshead velocity of the punching tool was 40 mm per minute and the applied force was limited to 0.2N. A second measurement was carried out to determine the distance (d), covered by the punching tool within the specimen during a period of 30 seconds, whereby the applied force was kept constant at 20N. Hardness of the specimen was calculated in the following manner.

$$hardness\ [\%] = (h_2 - d)/\ h_2 * 100\%$$

The described method was based on the determination of hardness according to Shore (DIN 53505).

**Example 13: Preparation of the IFS (witch an active agent, for in-vitro tests, which require sterile conditions)**

The raw materials have to be sterilized in an appropriate way. Initially 500 mg $\alpha$-TCP (100 - 350 $\mu$m granule size) were placed in a dry form in a 2R-glass. The stock solution of rhGDF-5 (3.4 mg/ml in 10 mM HCl) was diluted to 0.54 $\mu$g/ml with the means of the corresponding coating buffer. 475 $\mu$l of the rhGDF-5 solution obtained in that manner were pipetted on the $\alpha$-TCP and absorbed. The damp granulate was incubated for 1 hour at 25°C and then lyophilized. If $\alpha$-TCP alone was used as inorganic filler the coated granules were directly and accurately dispersed in the solution of the biodegradable polymer (e.g. PLGA RG 503H). Otherwise the coated granules where merged with other calcium compounds to form a CPC, which on its part was accurately dispersed in the solution of the biodegradable polymer.

**Example 14: Stability testing of rhGDF-5 and rhBMP-2 coated granules in different organic solvents**

Solvents such as polyethylene glycol 400, N-methylpyrrolidone (NMP) and acetone were used. The samples as well as the references were prepared by coating 500 mg of $\beta$-TCP with the respective protein (rhGDF-5 or rhBMP-2) to achieve a final concentration of 500 $\mu$g/g $\beta$-TCP. Afterwards 425 $\mu$l of the respective solvent were added to each sample, while the references were left untreated. After incubation for 24 hours at a temperature of 25°C both samples and references were extracted at 4°C for one hour with 3ml of an extraction buffer, consisting of urea (8M), Tris (10mM) and EDTA (100mM), whose pH level was adjusted to 6.7 with hydrochloric acid. After this extraction step all samples and references were centrifuged for 3 minutes with 4500 rpm. Subsequently the supernatant was diluted with solvent A (0.15 % trifluoroacetic acid and 20 % acetonitrile in water) in a ratio of 1:1. Solvent B was composed of 0.15 % trifluoroacetic acid and 84 % acetonitrile in water. The characterization of the proteins was carried out, using a Vydac C18, 2.1 x 250mm at a flow rate of 0.3 ml/min. The elution profile was recorded by measuring the absorbance at 220 nm. The amounts of rhGDF-5, rhBMP-2 and their degradation products were calculated from the peak area at 220 nm.

**Example 15: Release studies of rhGDF-5**

Pre-incubated specimens (preparation described in example 11), whose weight was well defined (150-300 mg), were transferred in a 50 ml tube, which contains 48 ml of medium ($\alpha$-MEM with 10 % FCS). The release studies were carried out at a temperature of 4°C. The pre-incubation step was necessary to obtain specimens of a constant shape to eliminate any influences of altering surfaces on the rate of rhGDP-5 release out of the formulations.

**Example 16: Quantification of rhGDF-5 release**

The rhGDF-5 release was quantified by means of ELISA. Infially antibody aMP-5 for rhGDF-5 was fixed on the surface of a microtiter plate. After having saturated free binding sites the plate was incubated with the samples containing rhGDF-5. Subsequently the bonded rhGDF-5 was incubated antibody aMP4, which was quantified by means of immune reaction with streptavidin POD.

**Example 17: Extraction of the immobilized rhGDF-5**

The immobilized rhGDF-5 was extracted by the means of an extraction buffer, consisting of urea (8M), Tris (10mM) and EDTA (100mM). The pH level was adjusted to pH6.7 with hydrochloric acid. 500 mg coated TCP-granules (500 mg rhGDF-5/ g TCP) were transferred in a 15 ml tube and were suspended in 3 ml of extraction buffer. Subsequently the granules were incubated for 60 minutes at 4°C. Finally the supernatant was centrifuged and diluted with solvent A in a ratio of 1:1. This solution serves for the characterization and quantification of rhGDF-5 via RP-HPLC.

**Example 18: Quantification of rhGDF-5 in solution by RP-HPLC**

The rhGDF-5 content was determined by reversed phase (RP-HPLC)-analysis. Aliquots of the sample were analyzed using a Porous 10 R1 C4 column (self-packed). 0.045 % trifluoroacetic acid in water (solvent A) and 0.025 % trifluoroacetic acid in 84 % acetonitrile (solvent B) were used as solvents at a flow rate of 0.4 ml/min. The elution profile was recorded by measuring the absorbance at 220 nm. The amounts of rhGDF-5 were calculated form the peak area at 220 nm using a standard curve.

**Example 19: Characterization of rhGDF-5 in solution by RP-HPLC**

The characterization of rhGDF-5 and its potential modifications was carried out by the means of RP-HPLC. Aliquots of the sample were analyzed using a Vydac C18, 21 x 250mm. 0.1 % trifluoroacetic acid in water (solvent A) and 0.15 % trifluoroacetic acid acetonitrile (solvent B) were used as solvents at a flow rate of 0.4 ml/min. The elution profile was recorded by measuring the absorbance at 220 nm. The relative content of rhGDF-5 was calculated from the peak area at 220 nm.

**Example 20: Analyzing of the IFS by scanning electron microscopy (SEM)**

The hardened and vacuum dried specimens were sputtered with carbon. Thereby a vacuum of approximately $10^{-4}$ mbar was applied. The target structures for these analyses were the surface and the core of the particular IFS specimens and especially the porosity exhibited by these structures.

**Example 21: Analyzing the IFS by differential scanning calorimetry (DSC)**

The specimens were vacuum dried. 10-20 mg of the specimens were accurately weight and analyzed, whereas the heating and cooling rates were between 10 and 30 K/min. The cooling was carried out by the means of liquid nitrogen.

**Example 22: Analyzing the IFS by light microscopy**

The specimens were prepared by incubating the pasty IFS-formulation in PBS buffer for at least 48 hours. To achieve a reproducible shape of the hardened IFS specimens, 1.5g (per specimen) of the pasty formulation were dosed in a well of a 24-well plate. After having finished the incubation the specimens were removed from the particular wells and were vacuum dried. The target structures for these analyses were the surface and the core of the particular IFS specimens and especially the porosity exhibited by these structures. The number of pores was counted. The pore size of the counted pores was measured by the means of so-called Soft Imaging Systems® software. These date served for the calculation of the average pore size, the related standard deviation and for the determination of pore size distribution.

**Table 3** shows the impact of different particle sizes of carboxymethylcellulose sodium salt (NaCMC) on the pore size distribution at the surface and in the core of the IFS

| analyzed structure: | surface | core | surface | core | surface | core | surface | core |
|---|---|---|---|---|---|---|---|---|
| Concentration NaCMC [wt%]: | 0 | 0 | 1 | | 1 | 1 | 1 | 1 |
| Particle size NaCMC: | - | | 25 - 1000 μm | | 25 -71 μm | | 900 -1000 μm | |
| Analysed area [mm²] | 59.0 | 29.5 | 59.0 | 29.5 | 59.0 | 29.5 | 59.0 | 29.5 |
| Number of pores: | 6 | 68 | 31 | 47 | 41 | 95 | 13 | 59 |
| Average pore size [μm]: | 104 | 641 | 313 | 523 | 83 | 292 | 233 | 348 |
| Minimal pore size [μm]: | 20 | 174 | 81 | 168 | 13 | 34 | 34 | 87 |
| Maximal pore size [μm]: | 228 | 1497 | 980 | 3960 | 705 | 1550 | 738 | 3369 |
| Standard deviation [μm]: | 72 | 206 | 200 | 538 | 146 | 177 | 236 | 423 |
| <200 μm: | 5 | 5 1 | 11 | 3 | 37 | 28 | 7 | 19 |
| 201-400μm: | 1 | 6 | 10 | 17 | 2 | 52 | 3 | 27 |
| 401- 600μm: | 0 | 19 | 10 | 17 | 1 | 13 | 1 | 11 |
| 601- 800μm: | 0 | 29 | 0 | 8 | 1 | 1 | 2 | 2 0 |
| 801-1000μm: | 0 | 9 | 0 | 1 | 0 | 0 | 0 | 1 |
| >1000 μm: | 0 | 4 | 0 | 1 | 0 | 1 | 0 | 1 |

IFS- composition (without NaCMC): PLGA RG 503H (22.2 wt%), calcium phosphate cement (33.3 wt%), polyethylene glycol 400 (44.5 wt%), IFS- composition (with NaCMC): PLGA RG 503H (22 wt%) calcium phosphate cement (33 wt%), polyethylene glycol 400 (44 wt%), NaCMC (1 wt%)

**Example 23: Determination of the density of the IFS and related systems**

A hollow mould with a defined volume was filled up with the particular formulations. The mass of the formulation fracture, which was required, therefore was determined. Hence the density was calculated as ratio of mass and volume (g/ml). The data are shown in Table 3. By adding an inorganic filler to the *in situ* hardening paste the amount of water insoluble polymer related to the defect volume can be considerably reduced about one third in contrast to conventional polymer based implants. This influences the local pH value within the tissue or defect site and avoids adverse or toxic site reactions due to the decrease of the pH during degradation of the polymer within the body.

Table 4 shows the Determination of PLGA content in a defined defect volume

| | IFS | IFS (CMC) | CaP free polymer solution | CaP free polymer solution (CMC) |
|---|---|---|---|---|
| density [g/ml] | 1.38 | 1.46 | 1.07 | 1.17 |
| content PLGA* wt% | 22.2 | 22.0 | 45.0 | 44.6 |

(continued)

|  | IFS | IFS (CMC) | CaP free polymer solution | CaP free polymer solution (CMC) |
|---|---|---|---|---|
| mass PLGA in a 1ml defect** [g] | 0.31 | 0.32 | 0.48 | 0.52 |

1. IFS: PLGA RG 503H (22.2 wt%), calcium phosphate cement (33.3 wt%), polyethylene glycol 400 (44.5 wt%)
2. IFS (CMC): PLGA 503H (22.0 wt%), calcium phosphate cement (33.0 wt%), polyethylene glycol 400 (44.0 wt%), carboxymethyl cellulose sodium salt (1.0 wt%)
3. CaP free polymer solution: PLGA RG 756 (45.0 wt%), N-methylpyrrolidone (55.0 wt%)
4. CaP free polymer solution (CMC): PLGA RG 756 (44.6 wt%), N-methylpyrrolidone (54.4 wt%), carboxymethylcellulose sodium salt (1.0 wt%), * in the formulation before application, ** after hardening

**References:**

Agrawal, C. M. et al. (1997); "Technique to Control pH in Vicinity of Biodegrading PLA-PGA implants"; J. Biomed. Mater. Res. (Appl. Biomater.) 38: 105-114.

Bohner, M. (2001); Eur Spine J 10: S114-121.

Bohner, M; Baroud, G. (2004); Biomaterials 26: 1553-1563.

Breitenbach, J. (2002); "Melt extrusion: from process to drug delivery technology", Eur. J. Pharm. Biopharm. 54: 107-117.

Celeste A.J. et al. (1990) "Identification of transforming growth factor.." Proc. Natl. Acad. Sci. U.S.A. 87: 9843-9847.

Chang, S. et al. (1994); "Cartilage-derived morphogenetic proteins..." J. Biol. Chem. 269:. 28227-28234.

Cherng, A. et al. (1997); J Biomed Mater Res 35: 273-277.

Chow, L.C. (2000); Mater Res Symp Proc 599: 27-37.

Del Real, R.P. et al (2002); Biomaterials 23: 3673-3680.

Draenert, K. et al (2001); Trauma Berufskrankh. 3: 293-300.

Driessens, F.C.M. et al. (2002); "The Ca/P range of nanoapatitic calcium phosphate cements." Biomaterials 23: 4011-4017.

Dunn et al. US patent 5,702,716.

Durucan, C. et al. (2000); "Calcium-deficient hydroxyapatite-PLGA composites: mechanical and microstructural investigation." J. Biomed. Mater. Res. 51: 726-724.

EMEA, ICH Topic Q 3 C, Impurities: Residual Solvents.

Friess, W. et al. (1998); Pharm. Dev. Technol. 4: 387-396.

Friess, W. (1999); "Collagen - biomaterial for drug delivery." Eur J Pharm Biopharm 45: 113-136.

Gao, T. et al. (1996); Int. Orthopaedics 20: 321- 325.

Gombotz, W. et al. (1996) in Formulation, characterization and stability of protein drugs, Plenum Press, New York, USA, pp 219 - 245.

Griffith, D.L. et al. (1996); "Three-dimensional structure of recombinant human..." Proc. Nati. Acad. Sci. U.S.A. 93: 878-883.

Herbert, P. et al., (1998); "A large scale process to produce microencapsulated Proteins." Pharm Res. 15: 357-361.

Hoetten, G. et al. (1994); "Coning and expression of recombinant human growth/differentiation factor 5." Biochem. Biophys. Res. Commun. 204: 646-652.

Hollinger, J.O. et al. (1996); "Poly($\alpha$-hydroxy acids): carriers for bone morphogenetic proteins." Biomaterials 17: 187-194.

Hotz, G. et al. (1994); Int. J. Oral Maxillofac. Surg. 23: 413-417.

Ignjatovic, N.L. et al. (1999); Biomaterials 20: 809-816.

Katagiri, T. et al. (1990); Biochem. Biophys. Res. Commun. 172: 295-299.

Kenley, R.A., et al. (1993); Pharm. Res. 10: 1393-1401.

Li, S.M., et al. (1990); "Structure-property relationship in the case of the degradation of massive poly(alpha-hydroxy acids) in aqueous media part. Part 1: poly(DL-lactic acid), J. Mater. Sci. Mater. Med., 1, 123-130

Lind, M. et al. (1996); J. Orthopaedic Res. 14: 343-350.

Lind, M. (1996); Acta. Orthop. Scand. 67: 407-417.

Middleton, J.C. et al. (2000); "Synthetic biopolymers as orthopedic devices." Biomaterials 21: 2335-2346.

Nishitoh. H. et al (1996); J Biol. Chem. 271: 21345-21352.

Oezkayanak. E. et al. (1990); "OP-1 cDNA encodes an osteogenic protein in the TGF-beta family." EMBO J. 9: 2085-2093.

Rueger, J.M. et al (1996); "Knochenersatzmittel"; Unfallchir. 99: 228-236.

Ruhe, P.Q., et al. (2003) ; J Bone Joint surgery 85-A(3): 75-81.

Seehermann, H., et al. (2002); "Bone morphogenentic protein delivery systems; Spine 15(16 Suppl 1): S16-23. Review.

Seehermann, H., et al. (2003); "A Review of Preclinical Program Development for Evaluation Injectable Carriers for Osteogenic Factors." J Bone Joint surgery 85-A(3): 96-108.

Scheufler, C. et al. (1990); "Crystal structure of human bone morphogenetic protein-2 at 2.7 A resolution." J Mol Biol. 287:103-115.

Schiller, C. et al. (2003); "Carbonated calcium phosphates are suitable pH-stabilising fillers for biodegradable polyesters." Biomaterials 24: 2037-2043.

Schmidmaler, G. et al. (2000); "Local liberation of IGF-I and TGF-beta 1 from a biodegradable poly(D,L-lactide) coating of implants accelerates fracture healing." Chirurg 71: 1016-1022.

Schmitt, J. et al. (1999); J. Orthop. Res. 17: 269-278.

Seeherman, H. (2003); "A review of preclinical program development for evaluating in injectable carriers for osteogenic factors." J. Bone. Joint Surg. Am. 85-A: Suppl. 3, 96-108. Shively M.L. et al. (1995); "Physico-chemical characterization of a polymeric injectable implant delivery system." J. Controlled Rel. 33: 237-243.

Shore, E.M. et al. (1997); "Human bone morphogenetic protein-2 (BMP-2) genomic DNA sequence".

Storm & Kingsley (1999); Development Biology, 209: 11-27:

Takagi, S. et al. (2003); J Biomed Mater Res Part B: Appl Biomater 67B: 689-696.

Terheyden, H. et al. (1997); Mund Kiefer Gesichtschir 1: 272-275.

Tormälä, P. et al. (1995); "Biodegradable polymers in orthopaedics: experimental and clinical." Mat. Clin. Appl. 639-651.

Tormälä, P. et al. (1992); "Biodegradable self-reinforced composite materials; manufacturing structure and mechanical properties." Clin. Mater. 10: 29-34.

Vert, M. (1989); "Bioresorbable polymers for temporary therapeutic applications." Angew. Makromol. Chem. 166/167: 155-168.

Wang, E.A. et al. (1990); "Identification of transforming growth factor beta family members present in bone-inductive protein purified from bovine bone." Proc. Natl. Acad. Sci. U.S.A. 87: 9843-9847.

Wang, Z.G. et al. (2000). Polymer 41: 621-628.

Wintermantel, E. et al., Medizintechnik mit biokompatiblen Werkstoffen und Verfahren, page 7, 511, 3. Auflage, Springer 2002, ISBN 3-540-41261-1.

Wozney, J.M. et al. (1998); Clin. Orthop. 346: 26-37.

Wozney, J.M. et al. (1988); Science 242:1528-1534.

Xu, H.H.R.; Quinn, J.B. (2001); J Biomed Mater Res. 57: 457-466.

Xu. H.H.R. et al. (2002); J Dent Res. 81: 219-224.

## Claims

1. An *in situ* hardening paste comprising:

   (a) a plasticizer, which is a water soluble or water miscible biocompatible organic liquid,
   (b) a water insoluble polymer, which is biocompatible, biodegradable, and/or bioresorbable and soluble in the plasticizer,
   (c) a water insoluble solid filler, which is insoluble in the plasticizer,

   wherein the paste, which is injectable and stable in its package, is capable of hardening *in situ* to form a solid implant upon contact with the aqueous medium or body fluid,
   wherein said plasticizer is polyethylene glycol (PEG) 400, PEG 200, PEG 300, PEG 600, 1,3-butandiol, castor oil, N-ethyl-2-pyrrolidone, 2-pyrrolidone, C2 to C6 alkanols, propylene glycol, solketal, acetone, methyl acetate, ethyl acetate, ethyl lactate, methyl ethyl ketone, dimethylformamide, dimethyl sulfone, tetrahydrofuran, caprolactam, docylmethylsuffoxide, oleic acid, propylene carbonate, triacetin, N,N-diethyl-m-toluamide; 1-doderytazacycloheptan-2-one or mixtures thereof.

2. The *in situ* hardening paste according to claim 1, further comprising an effective amount of a water soluble pore building filler.

3. The *in situ* hardening paste according to claim 1, wherein the implant has Interconnecting pores with a diameter of equal or more than 100 μm.

4. The *in situ* hardening paste according to claim 1, further comprising an active agent.

5. The *in situ* hardening paste according to claim 1, comprising an active agent coated onto the water insoluble solid filler.

6. The *in situ* hardening paste according to claim 1, wherein the water insoluble solid filler is alpha-tricalclum phosphate having a particle size that is equal to or greater than 300 μm.

7. The *in situ* hardening paste according to claim 1, wherein the ratio of the water insoluble solid filler and the water insoluble polymer is between 1: 1 and 5: 1.

8. The *in situ* hardening paste according to claim 1, wherein the water insoluble polymer is PLGA.

9. The *in situ* hardening paste according to claim 8, wherein the water insoluble polymer is an end-capped polymer.

10. The *in situ* hardening paste according to claim 1, wherein the water insoluble polymer content of the composition is equal or smaller than 40 wt%.

11. The *in situ* hardening paste according to claim 1, wherein the density of the paste composition is equal to or greater than 1,21 g /ml.

12. The *in situ* hardening paste according to claim 1, wherein said plasticizer is polyethylene glycol (PEG), 400.

13. The *in situ* hardening paste according to claim 1, wherein the plasticizer content is 40 - 95 wt%.

14. The *in situ* hardening paste according to claim 2, wherein the water soluble solid pore building filler is carboxymethylcellulose sodium salt.

15. The *in situ* hardening paste according to claim 2, wherein the water soluble solid pore building filler content of the composition is less than 10 wt%.

16. The *in situ* hardening paste according to claim 2, wherein the water soluble solid pore building filler has a particle size of 25 to 1000 μm.

17. The *in situ* hardening paste of claim 4 wherein said active agent is an osteoinductive or cartilage inductive protein.

18. A method for the production of the *in situ hardening paste* of claim 1, comprising the steps of:

   (a) mixing a component A comprising the plasticizer and a component B comprising the water insoluble polymer by dissolving component B in component A to provide a viscous liquid; and
   (b) mixing said viscous liquid obtained in (a) with the water insoluble solid filler to prepare the paste.

19. The method for the production of the *in situ hardening paste* of claim 4 comprising the steps of:

   (a) mixing a component A comprising the plasticizer and a component B comprising the water insoluble polymer by dissolving component B in component A to provide a viscous liquid;
   (b) dissolving the active agent in said viscous liquid; and
   (c) mixing said viscous liquid obtained in (b) with the water insoluble solid filler to prepare the paste.

20. The method for the production of the *in situ hardening paste* of claim 4 comprising the steps of:

   (a) mixing a component A comprising the plasticizer and a component B comprising the water insoluble polymer by dissolving component B in component A to provide a viscous liquid;
   (b) mixing said viscous liquid obtained in (a) with the water insoluble solid filler to prepare the paste, wherein said water insoluble solid filler comprises the active agent which is coated on said solid filler.

21. A method for preparation of a pharmaceutical composition comprising combining the *in situ hardening paste* of claim 1 with an active agent.

22. The *in situ* hardening paste of any of claims 1 to 17 for use in bone augmentation.

**Patentansprüche**

1. Eine *in situ aus*härtende Paste umfassend:

   (a) einen Weichmacher, welche eine wasserlösliche oder wassermischbare biokompatible organische Flüssigkeit ist,
   (b) ein wasserunlösliches Polymer, welches biokompatibel, bioabbaubar und/oder bioresorbierbar ist und löslich ist in dem Weichmacher,
   (c) ein wasserunlöslicher fester Füllstoff, der unlöslich ist in dem Weichmacher,

   wobei die Paste, die in injizierbar ist und stabil in ihrer Verpackung ist, in der Lage ist, *in situ* auszuhärten, um ein festes Implantat nach Kontakt mit dem wässrigen Medium oder der Körperflüssigkeit auszubilden,
   wobei besagter Weichmacher Polyethylenglykol (PEG) 400, PEG 200, PEG 300, PEG 600, 1,3-butandiol, Castoröl, N-methyl-2-pyrrolidon, 2-pyrrolidon, C2 bis C6 Alkanole, Propyleneglykol, Solketal, Aceton, Methylazetat, Ethylazetat, Ethyllazetat, Methylethylketon, Dimethylformamid, Dimethylsulfon, Tetrahydrofuran, Caprolactam, Decylmethylsulfoxid, Ölsäure, Propylencarbonat, Triazetin, N,N-diethyl-m-toluamid; 1-dodecylazacycloheptan-2-on ist oder Mischungen davon.

2. Die *in situ aus*härtende Paste gemäß Anspruch 1, des Weiteren umfassend eine effektive Menge eines wasserlöslichen porenbildenden Füllstoffes.

3. Die *in situ aus*härtende Paste gemäß Anspruch 1, wobei das Implantat verbrückende Poren mit einem Durchmesser von gleich oder von größer oder gleich 100 $\mu$m aufweist.

4. Die *in situ aus*härtende Paste gemäß Anspruch 1, des Weiteren umfassend ein aktives Agens.

5. Die *in situ aus*härtende Paste gemäß Anspruch 1, umfassend ein aktives Agens überzogen auf dem wasserunlöslichen festen Füllstoff.

6. Die *in situ aus*härtende Paste gemäß Anspruch 1, wobei der wasserunlösliche feste Füllstoff Alpha-Trikalziumphosphat ist mit einer Partikelgröße, die größer oder gleich 300 $\mu$m ist.

7. Die *in situ aus*härtende Paste gemäß Anspruch 1, wobei das Verhältnis des wasserunlöslichen festen Füllstoffes zum wasserunlöslichen Polymer zwischen 1:1 und 5:1 ist.

8. Die *in situ aus*härtende Paste gemäß Anspruch 1, wobei das wasserunlösliche Polymer PLGA ist.

9. Die *in situ aus*härtende Paste gemäß Anspruch 8, wobei das wasserunlösliche Polymer ein end-verkapptes Polymer ist.

10. Die *in situ aus*härtende Paste gemäß Anspruch 1, wobei der Gehalt des wassersunlöslichen Polymers der Zusammensetzung kleiner oder gleich 40 Gew.-% ist.

11. Die *in situ aus*härtende Paste gemäß Anspruch 1, wobei die Dichte der Pasten-Zusammensetzung größer oder gleich 1,21 g/ml ist.

12. Die *in situ aus*härtende Paste gemäß Anspruch 1, wobei besagter Weichmacher Polyethylenglykol (PEG) 400 ist.

13. Die *in situ aus*härtende Paste gemäß Anspruch 1, wobei der Gehalt des Weichmachers 40-95 Gew.-% ist.

14. Die *in situ aus*härtende Paste gemäß Anspruch 2, wobei der wasserlösliche feste porenbildende Füllstoff Carboxymethylcellulose in Form des Natriumsalzes ist.

**15.** Die *in situ aush*ärtende Paste gemäß Anspruch 2, wobei der Gehalt des wasserlöslichen festen porenbildenden Füllstoffes der Zusammensetzung weniger als 10 Gew.-% beträgt.

**16.** Die *in situ aush*ärtende Paste gemäß Anspruch 2, wobei der wasserlösliche feste porenbildende Füllstoff eine Partikelgröße von 25-1000 μm aufweist.

**17.** Die *in situ aush*ärtende Paste gemäß Anspruch 4, wobei besagtes aktives Agens ein osteoinduktives oder Knorpel-induzierendes Protein ist.

**18.** Ein Verfahren zum Herstellen einer *in situ aush*ärtenden Paste gemäß Anspruch 1, umfassend die Schritte von:

(a) Vermischen einer Komponente A, umfassend den Weichmacher, und einer Komponente B, umfassend das wasserunlösliche Polymer, durch Auflösen der Komponente B in Komponente A, um eine viskose Flüssigkeit bereitzustellen; und
(b) Vermischen von besagter viskoser Flüssigkeit, erhalten in (a) mit dem wasserunlöslichen festen Füllstoff, um die Paste herzustellen.

**19.** Das Verfahren für die Herstellung der *in situ* aushärtenden Paste gemäß Anspruch 4, umfassend die Schritte von:

(a) Vermischen einer Komponente A umfassend den Weichmacher, und einer Komponente B umfassend das wasserunlösliche Polymer, durch Auflösen der Komponente B in Komponente A, um eine viskose Flüssigkeit bereitzustellen;
(b) Auflösen des aktiven Agens in besagter viskoser Flüssigkeit; und
(c) Vermischen von besagter viskoser Flüssigkeit, erhalten in (b) mit dem wasserunlöslichen festen Füllstoff, um die Paste herzustellen.

**20.** Das Verfahren zur Herstellung der *in situ* aushärtenden Paste von Anspruch 4, umfassend die Schritte von:

(a) Vermischen einer Komponente A, umfassend den Weichmacher, und einer Komponente B, umfassend das wasserunlösliche Polymer, durch Auflösen der Komponente B in Komponente A, um eine viskose Flüssigkeit bereitzustellen;
(b) Vermischen von besagter viskoser Flüssigkeit, erhalten in (a) mit dem wasserunlöslichen festen Füllstoff, um die Paste herzustellen, wobei besagter wasserunlöslicher fester Füllstoff das aktive Agens umfasst, welches auf besagten festen Füllstoff als Überzug aufgebracht ist.

**21.** Ein Verfahren zur Herstellung einer pharmazeutischen Zusammensetzung, umfassend das Vereinigen der *in situ aush*ärtenden Paste von Anspruch 1 mit einem aktiven Agens.

**22.** *In situ aush*ärtende Paste gemäß irgendeinen der Ansprüche 1 bis 17 zur Verwendung im Knochenaufbau.

**Revendications**

**1.** Pâte à durcissement *in situ* comprenant :

(a) un plastifiant, qui est un liquide organique biocompatible soluble dans l'eau ou miscible à l'eau,
(b) un polymère insoluble dans l'eau, qui est biocompatible, biodégradable, et/ou biorésorbable et soluble dans le plastifiant,
(c) une charge solide insoluble dans l'eau, qui est insoluble dans le plastifiant,

où la pâte, qui est injectable et stable dans son conditionnement, est capable de durcir *in situ* pour former un implant solide après contact avec le milieu aqueux ou un fluide corporel,
où ledit plastifiant est le polyéthylèneglycol (PEG) 400, le PEG 200, le PEG 300, le PEG 600, le 1,3-butanediol, l'huile de ricin, la N-méthyl-2-pyrrolidone, la 2-pyrrolidone, les alcanols en C2 à C6, le propylèneglycol, le Solketal, l'acétone, l'acétate de méthyle, l'acétate d'éthyle, le lactate d'éthyle, la méthyléthylcétone, le diméthylformamide, la diméthylsulfone, le tétrahydrofurane, le caprolactame, le décylméthylsulfoxyde, l'acide oléique, le carbonate de propylène, la triacétine, le N,N-diéthyl-m-toluamide ; la 1-dodécylazacycloheptan-2-one ou les mélanges de ceux-ci.

**2.** Pâte à durcissement *in situ* selon la revendication 1, comprenant en outre une quantité efficace d'une charge de construction de pores soluble dans l'eau.

**3.** Pâte à durcissement *in situ* selon la revendication 1, dans laquelle l'implant comporte des pores interconnectés ayant un diamètre supérieur ou égal à 100 μm.

**4.** Pâte à durcissement *in situ* selon la revendication 1, comprenant en outre un agent actif.

**5.** Pâte à durcissement *in situ* selon la revendication 1, comprenant un agent actif appliqué en revêtement sur la charge solide insoluble dans l'eau.

**6.** Pâte à durcissement *in situ* selon la revendication 1, dans laquelle la charge solide insoluble dans l'eau est le phosphate tricalcique alpha ayant une taille de particule supérieure ou égale à 300 μm.

**7.** Pâte à durcissement *in situ* selon la revendication 1, dans laquelle le rapport de la charge solide insoluble dans l'eau et du polymère insoluble dans l'eau est compris entre 1:1 et 5:1.

**8.** Pâte à durcissement *in situ* selon la revendication 1, dans laquelle le polymère insoluble dans l'eau est le PLGA.

**9.** Pâte à durcissement *in situ* selon la revendication 8, dans laquelle le polymère insoluble dans l'eau est un polymère terminalement coiffé.

**10.** Pâte à durcissement *in situ* selon la revendication 1, dans laquelle la teneur en polymère insoluble dans l'eau de la composition est inférieure ou égale à 40 % en poids.

**11.** Pâte à durcissement *in situ* selon la revendication 1, dans laquelle la masse volumique de la composition de pâte est supérieure ou égale à 1,21 g/ml.

**12.** Pâte à durcissement *in situ* selon la revendication 1, dans laquelle ledit plastifiant est le polyéthylèneglycol (PEG) 400.

**13.** Pâte à durcissement *in situ* selon la revendication 1, dans laquelle la teneur en plastifiant est de 40-95 % en poids.

**14.** Pâte à durcissement *in situ* selon la revendication 2, dans laquelle la charge de construction de pores soluble dans l'eau est le sel de sodium de la carboxyméthylcellulose.

**15.** Pâte à durcissement *in situ* selon la revendication 2, dans laquelle la teneur en charge de construction de pores solide soluble dans l'eau de la composition est inférieure à 10 % en poids.

**16.** Pâte à durcissement *in situ* selon la revendication 2, dans laquelle la charge de construction de pores solide soluble dans l'eau a une taille de particule de 25 à 1000 μm.

**17.** Pâte à durcissement *in situ* selon la revendication 4 dans laquelle ledit agent actif est une protéine ostéoinductrice ou inductrice de cartilage.

**18.** Procédé de production de la pâte à durcissement *in situ* selon la revendication 1, comprenant les étapes consistant à :

(a) mélanger un composant A comprenant le plastifiant et un composant B comprenant le polymère insoluble dans l'eau en dissolvant le composant B dans le composant A pour obtenir un liquide visqueux ; et
(b) mélanger ledit liquide visqueux obtenu en (a) avec la charge solide insoluble dans l'eau pour préparer la pâte.

**19.** Procédé de production de la pâte à durcissement *in situ* selon la revendication 4 comprenant les étapes consistant à :

(a) mélanger un composant A comprenant le plastifiant et un composant B comprenant le polymère insoluble dans l'eau en dissolvant le composant B dans le composant A pour obtenir un liquide visqueux ;
(b) dissoudre l'agent actif dans ledit liquide visqueux ; et
(c) mélanger ledit liquide visqueux obtenu en (b) avec la charge solide insoluble dans l'eau pour préparer la pâte.

**20.** Procédé de production de la pâte à durcissement *in situ* selon la revendication 4 comprenant les étapes consistant à :

(a) mélanger un composant A comprenant le plastifiant et un composant B comprenant le polymère insoluble dans l'eau en dissolvant le composant B dans le composant A pour obtenir un liquide visqueux ;

(b) mélanger ledit liquide visqueux obtenu en (a) avec la charge solide insoluble dans l'eau pour préparer la pâte, ladite charge solide insoluble dans l'eau comprenant l'agent actif qui appliqué en revêtement sur ladite charge solide.

21. Procédé de préparation d'une composition pharmaceutique comprenant la combinaison de la pâte à durcissement *in situ* selon la revendication 1 avec un agent actif.

22. Pâte à durcissement *in situ* selon n'importe lesquelles des revendications 1 à 17 destinée à être utilisée dans l'augmentation osseuse.

Fig. 1/18

Fig. 2/18

Fig. 3/18

**Fig. 4/18**

Fig. 5/18

EP 1 763 374 B1

Fig. 6/18

**Fig. 7/18**

EP 1 763 374 B1

Fig. 8/18

EP 1 763 374 B1

Tg [°C]

51.7

36.3

21.7

36.8

1d

time [days]

60 50 40 30 20 10 0

Fig. 9/18

Fig. 10/18

**Fig. 11/18**

EP 1 763 374 B1

Fig. 12/18

Fig. 13/18

EP 1 763 374 B1

Fig. 14/18

Fig. 15/18

Fig. 16/18

Fig. 17/18

Fig. 18/18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6206957 B
- WO 9821972 A
- WO 03043673 A
- WO 02070029 A
- US 6726860 B
- EP 0436667 A
- EP 0537559 A
- EP 0539751 A
- EP 0754032 A
- EP 0862416 A
- EP 1126822 A
- EP 1147781 A
- EP 1404294 A
- US 6461631 B
- US 5780044 A
- US 5278202 A
- US 2004034434 A1
- WO 2004073563 A2
- US 5702716 A, Dunn
- EP 04013668 A

### Non-patent literature cited in the description

- **KATAGIRI T ; YAMAGUCHI A ; IKEDA T ; YOSHIKI S ; WOZNEY JM ; ROSEN V ; WANG EA ; TANKA H ; OMURA S ; SUDA T.** The non-osteogenic mouse pluripotent cell line, C3H10T1/2, is induced to differentiate into osteoblastic cells by recombinant human bone morphogenetic protein-2. *Biochem. Biophys. Res. Commun.,* 1990, vol. 172, 295-299
- **NISHITOH H ; ICHIJO H ; KIMURA M ; MATSUMOTO T ; MAKISHIMA F ; YAMAGUCHI A ; YAMASHITA H ; ENOMOTO S ; MIYAZONO K.** Identification of type serine/ threonine kinase receptors for growth/differentiation factor-5. *J. Biol. Chem.,* 1996, vol. 271, 21345-21352
- **WOZNEY JM ; ROSEN V.** Bone morphogenetic protein and bone morphogenetic protein gene family in bone formation and repair. *Clin Orthop,* 1998, vol. 346, 26-37
- **WOZNEY JM ; ROSEN V.** Bone morphogenetic protein and bone morphogenetic protein gene family in bone formation and repair. *Clin Orthop,* 1998, vol. 346, 26-27
- **SCHMITT J ; HWANG K ; WINN, SR ; HOLLINGER J.** Bone morphogenetic proteins: an update on basic biology and clinical relevance. *J Orthop Res,* 1999, vol. 17, 269-278
- **LIND M.** Growth factors: possible new clinical tools. *A review. Acta Orthop Scand,* 1996, vol. 67, 407-17
- **STORM ; KINGSLEY.** *Development Biology,* 1999, vol. 209, 11-27
- **WOZNEY J.M. ; ROSEN V. ; CELESTE A.J. ; MITSOCK L.M. ; WHITTERS M.J. ; KRIZ R.W. ; HEWICK R.M. ; WANG E.A.** Novel regulators of bone formation: molecular clones and activities. *Science,* 1988, vol. 242, 1528-1534
- **SCHEUFLER C. ; SEBALD W. ; HUELSMEYER M.** Crystal structure of human bone morphogenetic protein-2 at 2.7 A resolution. *J. Mol. Biol.,* 1999, vol. 287, 103-115
- **OEZKAYNAK E. ; RUEGER D.C. ; DRIER E.A. ; CORBETT C. ; RIDGE R.J. ; SAMPATH T.K. ; OPPERMANN H.** OP-1 cDNA encodes an osteogenic protein in the TGF-beta family. *EMBO J.,* 1990, vol. 9, 2085-2093
- **CELESTE A.J. ; IANNAZZI J.A. ; TAYLOR R.C. ; HEWICK R.M. ; ROSEN V. ; WANG E.A. ; WOZNEY J.M.** Identification of transforming growth factor beta family members present in bone-inductive protein purified from bovine bone. *Proc. Natl. Acad. Sci. U.S.A.,* 1990, vol. 87, 9843-9847
- **GRIFFITH D.L. ; KECK P.C. ; SAMPATH T.K. ; RUEGER D.C. ; CARLSON W.D.** Three-dimensional structure of recombinant human osteogenic protein 1: structural paradigm for the transforming growth factor beta superfamily. *Proc. Natl. Acad. Sci. U.S.A.,* 1996, vol. 93, 878-883
- **HOETTEN G. ; NEIDHARDT H. ; JACOBOWSKY B. ; POHL J.** clowning and expression of recombinant human growth/differentiation factor 5. *Biochem. Biophys. Res. Commun.,* 1994, vol. 204, 646-652

- **CHANG S. ; HOANG B. ; THOMAS J.T. ; VU-KICEVIC S. ; LUYTEN F.P. ; RYBA N.J.P. ; KOZAK C.A. ; REDDI A.H. ; MOOS M.** Cartilage-derived morphogenetic proteins. New members of the transforming growth factor-beta superfamily predominantly expressed in long bones during human embryonic development. *J. Biol. Chem.,* 1994, vol. 269, 28227-28234
- **AGRAWAL, C. M. et al.** Technique to Control pH in Vicinity of Biodegrading PLA-PGA implants. *J. Biomed. Mater. Res. (Appl. Biomater.,* 1997, vol. 38, 105-114
- **BOHNER, M.** *Eur Spine J,* 2001, vol. 10, 114-121
- **BOHNER, M ; BAROUD, G.** *Biomaterials,* 2004, vol. 26, 1553-1563
- **BREITENBACH, J.** Melt extrusion: from process to drug delivery technology. *Eur. J. Pharm. Biopharm.,* 2002, vol. 54, 107-117
- **CELESTE A.J. et al.** Identification of transforming growth factor. *Proc. Natl. Acad. Sci. U.S.A.,* 1990, vol. 87, 9843-9847
- **CHANG, S. et al.** Cartilage-derived morphogenetic proteins... *J. Biol. Chem.,* 1994, vol. 269, 28227-28234
- **CHERNG, A. et al.** *J Biomed Mater Res,* 1997, vol. 35, 273-277
- **CHOW, L.C.** *Mater Res Symp Proc,* 2000, vol. 599, 27-37
- **DEL REAL, R.P. et al.** *Biomaterials,* 2002, vol. 23, 3673-3680
- **DRAENERT, K. et al.** *Trauma Berufskrankh.,* 2001, vol. 3, 293-300
- **DRIESSENS, F.C.M. et al.** The Ca/P range of nanoapatitic calcium phosphate cements. *Biomaterials,* 2002, vol. 23, 4011-4017
- **DURUCAN, C. et al.** Calcium-deficient hydroxyapatite-PLGA composites: mechanical and microstructural investigation. *J. Biomed. Mater. Res.,* 2000, vol. 51, 726-724
- **FRIESS, W. et al.** *Pharm. Dev. Technol.,* 1998, vol. 4, 387-396
- **FRIESS, W.** Collagen - biomaterial for drug delivery. *Eur J Pharm Biopharm,* 1999, vol. 45, 113-136
- **GAO, T. et al.** *Int. Orthopaedics,* 1996, vol. 20, 321-325
- **GOMBOTZ, W. et al.** Formulation, characterization and stability of protein drugs. Plenum Press, 1996, 219-245
- **GRIFFITH, D.L. et al.** Three-dimensional structure of recombinant human... *Proc. Nati. Acad. Sci. U.S.A.,* 1996, vol. 93, 878-883
- **HERBERT, P. et al.** A large scale process to produce microencapsulated Proteins. *Pharm Res.,* 1998, vol. 15, 357-361
- **HOETTEN, G. et al.** Coning and expression of recombinant human growth/differentiation factor 5. *Biochem. Biophys. Res. Commun.,* 1994, vol. 204, 646-652
- **HOLLINGER, J.O. et al.** Poly($\alpha$-hydroxy acids): carriers for bone morphogenetic proteins. *Biomaterials,* 1996, vol. 17, 187-194
- **HOTZ, G. et al.** *Int. J. Oral Maxillofac. Surg.,* 1994, vol. 23, 413-417
- **IGNJATOVIC, N.L. et al.** *Biomaterials,* 1999, vol. 20, 809-816
- **KATAGIRI, T. et al.** *Biochem. Biophys. Res. Commun.,* 1990, vol. 172, 295-299
- **KENLEY, R.A. et al.** *Pharm. Res.,* 1993, vol. 10, 1393-1401
- **LI, S.M. et al.** Structure-property relationship in the case of the degradation of massive poly(alpha-hydroxy acids) in aqueous media part. Part 1: poly(DL-lactic acid. *J. Mater. Sci. Mater. Med.,* 1990, vol. 1, 123-130
- **LIND, M. et al.** *J. Orthopaedic Res.,* 1996, vol. 14, 343-350
- **LIND, M.** *Acta. Orthop. Scand.,* 1996, vol. 67, 407-417
- **MIDDLETON, J.C. et al.** Synthetic biopolymers as orthopedic devices. *Biomaterials,* 2000, vol. 21, 2335-2346
- **NISHITOH. H. et al.** *J Biol. Chem.,* 1996, vol. 271, 21345-21352
- **OEZKAYANAK. E. et al.** OP-1 cDNA encodes an osteogenic protein in the TGF-beta family. *EMBO J.,* 1990, vol. 9, 2085-2093
- **RUEGER, J.M. et al.** Knochenersatzmittel. *Unfallchir.,* 1996, vol. 99, 228-236
- **RUHE, P.Q. et al.** *J Bone Joint surgery,* 2003, vol. 85-A (3), 75-81
- **SEEHERMANN, H. et al.** Bone morphogenentic protein delivery systems. *Spine,* 2002, vol. 15 (16), 16-23
- **SEEHERMANN, H. et al.** A Review of Preclinical Program Development for Evaluation Injectable Carriers for Osteogenic Factors. *J Bone Joint surgery,* 2003, vol. 85-A (3), 6-108
- **SCHEUFLER, C. et al.** Crystal structure of human bone morphogenetic protein-2 at 2.7 A resolution. *J Mol Biol.,* 1990, vol. 287, 103-115
- **SCHILLER, C. et al.** Carbonated calcium phosphates are suitable pH-stabilising fillers for biodegradable polyesters. *Biomaterials,* 2003, vol. 24, 2037-2043
- **SCHMIDMALER, G. et al.** Local liberation of IGF-I and TGF-beta 1 from a biodegradable poly(D,L-lactide) coating of implants accelerates fracture healing. *Chirurg,* 2000, vol. 71, 1016-1022
- **SCHMITT, J. et al.** *J. Orthop. Res.,* 1999, vol. 17, 269-278

- **SEEHERMAN, H.** A review of preclinical program development for evaluating in injectable carriers for osteogenic factors. *J. Bone. Joint Surg. Am.,* 2003, vol. 85-A (3), 96-108
- **SHIVELY M.L. et al.** Physico-chemical characterization of a polymeric injectable implant delivery system. *J. Controlled Rel.,* 1995, vol. 33, 237-243
- **SHORE, E.M. et al.** *Human bone morphogenetic protein-2 (BMP-2) genomic DNA sequence,* 1997
- **TAKAGI, S. et al.** *J Biomed Mater Res Part B: Appl Biomater,* 2003, vol. 67B, 689-696
- **TERHEYDEN, H. et al.** *Mund Kiefer Gesichtschir,* 1997, vol. 1, 272-275
- **TORMÄLÄ, P. et al.** Biodegradable polymers in orthopaedics: experimental and clinical. *Mat. Clin. Appl.,* 1995, 639-651
- **TORMÄLÄ, P. et al.** Biodegradable self-reinforced composite materials; manufacturing structure and mechanical properties. *Clin. Mater.,* 1992, vol. 10, 29-34
- **VERT, M.** Bioresorbable polymers for temporary therapeutic applications. *Angew. Makromol. Chem.,* 1989, vol. 166/167, 155-168
- **WANG, E.A. et al.** Identification of transforming growth factor beta family members present in bone-inductive protein purified from bovine bone. *Proc. Natl. Acad. Sci. U.S.A.,* 1990, vol. 87, 9843-9847
- **WANG, Z.G. et al.** *Polymer,* 2000, vol. 41, 621-628
- **WINTERMANTEL, E. et al.** Medizintechnik mit biokompatiblen Werkstoffen und Verfahren. Springer, 2002
- **WOZNEY, J.M. et al.** *Clin. Orthop.,* 1998, vol. 346, 26-37
- **WOZNEY, J.M. et al.** *Science,* 1988, vol. 242, 1528-1534
- **XU, H.H.R. ; QUINN, J.B.** *J Biomed Mater Res.,* 2001, vol. 57, 457-466
- **XU. H.H.R. et al.** *J Dent Res.,* 2002, vol. 81, 219-224

SEQUENCE LISTING

<110> Scil Technology GmbH

<120> In situ hardening paste, its manufacturing and use

<130> EP31498EP

<150> EP04013668.1
<151> 2004-06-09

<160> 4

<170> PatentIn version 3.1

<210> 1
<211> 396
<212> PRT
<213> Homo sapiens

<400> 1

```
Met Val Ala Gly Thr Arg Cys Leu Leu Ala Leu Leu Leu Pro Gln Val
1               5               10                      15


Leu Leu Gly Gly Ala Ala Gly Leu Val Pro Glu Leu Gly Arg Arg Lys
            20              25                      30


Phe Ala Ala Ala Ser Ser Gly Arg Pro Ser Ser Gln Pro Ser Asp Glu
        35              40                  45


Val Leu Ser Glu Phe Glu Leu Arg Leu Leu Ser Met Phe Gly Leu Lys
    50              55                  60


Gln Arg Pro Thr Pro Ser Arg Asp Ala Val Val Pro Pro Tyr Met Leu
65              70              75                      80


Asp Leu Tyr Arg Arg His Ser Gly Gln Pro Gly Ser Pro Ala Pro Asp
            85                  90                      95


His Arg Leu Glu Arg Ala Ala Ser Arg Ala Asn Thr Val Arg Ser Phe
            100             105                 110


His His Glu Glu Ser Leu Glu Glu Leu Pro Glu Thr Ser Gly Lys Thr
        115             120                 125


Thr Arg Arg Phe Phe Phe Asn Leu Ser Ser Ile Pro Thr Glu Glu Phe
    130                 135                 140


Ile Thr Ser Ala Glu Leu Gln Val Phe Arg Glu Gln Met Gln Asp Ala
145                 150                 155                 160


Leu Gly Asn Asn Ser Ser Phe His His Arg Ile Asn Ile Tyr Glu Ile
```

40

                        165                       170                       175

Ile Lys Pro Ala Thr Ala Asn Ser Lys Phe Pro Val Thr Arg Leu Leu
            180                   185                   190

Asp Thr Arg Leu Val Asn Gln Asn Ala Ser Arg Trp Glu Ser Phe Asp
            195                   200                   205

Val Thr Pro Ala Val Met Arg Trp Thr Ala Gln Gly His Ala Asn His
    210                   215                   220

Gly Phe Val Val Glu Val Ala His Leu Glu Glu Lys Gln Gly Val Ser
225                   230                   235                   240

Lys Arg His Val Arg Ile Ser Arg Ser Leu His Gln Asp Glu His Ser
                245                   250                   255

Trp Ser Gln Ile Arg Pro Leu Leu Val Thr Phe Gly His Asp Gly Lys
            260                   265                   270

Gly His Pro Leu His Lys Arg Glu Lys Arg Gln Ala Lys His Lys Gln
            275                   280                   285

Arg Lys Arg Leu Lys Ser Ser Cys Lys Arg His Pro Leu Tyr Val Asp
    290                   295                   300

Phe Ser Asp Val Gly Trp Asn Asp Trp Ile Val Ala Pro Pro Gly Tyr
305                   310                   315                   320

His Ala Phe Tyr Cys His Gly Glu Cys Pro Phe Pro Leu Ala Asp His
                325                   330                   335

Leu Asn Ser Thr Asn His Ala Ile Val Gln Thr Leu Val Asn Ser Val
            340                   345                   350

Asn Ser Lys Ile Pro Lys Ala Cys Cys Val Pro Thr Glu Leu Ser Ala
            355                   360                   365

Ile Ser Met Leu Tyr Leu Asp Glu Asn Glu Lys Val Val Leu Lys Asn
    370                   375                   380

Tyr Gln Asp Met Val Val Glu Gly Cys Gly Cys Arg
385                   390                   395

<210> 2
<211> 431
<212> PRT

<213> Homo sapiens

<400> 2

```
Met His Val Arg Ser Leu Arg Ala Ala Ala Pro His Ser Phe Val Ala
1              5                   10                  15

Leu Trp Ala Pro Leu Phe Leu Leu Arg Ser Ala Leu Ala Asp Phe Ser
             20                  25                  30

Leu Asp Asn Glu Val His Ser Ser Phe Ile His Arg Arg Leu Arg Ser
         35                  40                  45

Gln Glu Arg Arg Glu Met Gln Arg Glu Ile Leu Ser Ile Leu Gly Leu
     50                  55                  60

Pro His Arg Pro Arg Pro His Leu Gln Gly Lys His Asn Ser Ala Pro
65              70                  75                  80

Met Phe Met Leu Asp Leu Tyr Asn Ala Met Ala Val Glu Glu Gly Gly
             85                  90                  95

Gly Pro Gly Gly Gln Gly Phe Ser Tyr Pro Tyr Lys Ala Val Phe Ser
         100                 105                 110

Thr Gln Gly Pro Pro Leu Ala Ser Leu Gln Asp Ser His Phe Leu Thr
     115                 120                 125

Asp Ala Asp Met Val Met Ser Phe Val Asn Leu Val Glu His Asp Lys
     130                 135                 140

Glu Phe Phe His Pro Arg Tyr His His Arg Glu Phe Arg Phe Asp Leu
145             150                 155                 160

Ser Lys Ile Pro Glu Gly Glu Ala Val Thr Ala Ala Glu Phe Arg Ile
             165                 170                 175

Tyr Lys Asp Tyr Ile Arg Glu Arg Phe Asp Asn Glu Thr Phe Arg Ile
         180                 185                 190

Ser Val Tyr Gln Val Leu Gln Glu His Leu Gly Arg Glu Ser Asp Leu
         195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240
```

His Asn Leu Gly Leu Gln Leu Ser Val Glu Thr Leu Asp Gly Gln Ser
                    245                 250                 255

Ile Asn Pro Lys Leu Ala Gly Leu Ile Gly Arg His Gly Pro Gln Asn
        260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
            355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
                420                 425                 430

<210> 3
<211> 501
<212> PRT
<213> Homo sapiens

<400> 3

44

Met Arg Leu Pro Lys Leu Leu Thr Phe Leu Leu Trp Tyr Leu Ala Trp
1               5                   10              15

Leu Asp Leu Glu Phe Ile Cys Thr Val Leu Gly Ala Pro Asp Leu Gly
                20                  25                  30

```
Gln Arg Pro Gln Gly Ser Arg Pro Gly Leu Ala Lys Ala Glu Ala Lys
          35                  40                  45

Glu Arg Pro Pro Leu Ala Arg Asn Val Phe Arg Pro Gly Gly His Ser
          50                  55                  60

Tyr Gly Gly Gly Ala Thr Asn Ala Asn Ala Arg Ala Lys Gly Gly Thr
65                  70                  75                  80

Gly Gln Thr Gly Gly Leu Thr Gln Pro Lys Lys Asp Glu Pro Lys Lys
              85                  90                  95

Leu Pro Pro Arg Pro Gly Gly Pro Glu Pro Lys Pro Gly His Pro Pro
          100                 105                 110

Gln Thr Arg Gln Ala Thr Ala Arg Thr Val Thr Pro Lys Gly Gln Leu
          115                 120                 125

Pro Gly Gly Lys Ala Pro Pro Lys Ala Gly Ser Val Pro Ser Ser Phe
    130                 135                 140

Leu Leu Lys Lys Ala Arg Glu Pro Gly Pro Pro Arg Glu Pro Lys Glu
145                 150                 155                 160

Pro Phe Arg Pro Pro Pro Ile Thr Pro His Glu Tyr Met Leu Ser Leu
              165                 170                 175

Tyr Arg Thr Leu Ser Asp Ala Asp Arg Lys Gly Gly Asn Ser Ser Val
          180                 185                 190

Lys Leu Glu Ala Gly Leu Ala Asn Thr Ile Thr Ser Phe Ile Asp Lys
    195                 200                 205

Gly Gln Asp Asp Arg Gly Pro Val Val Arg Lys Gln Arg Tyr Val Phe
    210                 215                 220

Asp Ile Ser Ala Leu Glu Lys Asp Gly Leu Leu Gly Ala Glu Leu Arg
225                 230                 235                 240

Ile Leu Arg Lys Lys Pro Ser Asp Thr Ala Lys Pro Ala Val Pro Arg
              245                 250                 255

Ser Arg Arg Ala Ala Gln Leu Lys Leu Ser Ser Cys Pro Ser Gly Arg
          260                 265                 270

Gln Pro Ala Ala Leu Leu Asp Val Arg Ser Val Pro Gly Leu Asp Gly
    275                 280                 285
```

46

Ser Gly Trp Glu Val Phe Asp Ile Trp Lys Leu Phe Arg Asn Phe Lys
290                    295              300

Asn Ser Ala Gln Leu Cys Leu Glu Leu Glu Ala Trp Glu Arg Gly Arg
305              310              315                    320

Thr Val Asp Leu Arg Gly Leu Gly Phe Asp Arg Ala Ala Arg Gln Val
                325              330                    335

His Glu Lys Ala Leu Phe Leu Val Phe Gly Arg Thr Lys Lys Arg Asp
            340              345              350

Leu Phe Phe Asn Glu Ile Lys Ala Arg Ser Gly Gln Asp Asp Lys Thr
        355              360              365

Val Tyr Glu Tyr Leu Phe Ser Gln Arg Arg Lys Arg Arg Ala Pro Leu
370              375              380

Ala Thr Arg Gln Gly Lys Arg Pro Ser Lys Asn Leu Lys Ala Arg Cys
385              390              395              400

Ser Arg Lys Ala Leu His Val Asn Phe Lys Asp Met Gly Trp Asp Asp
            405              410              415

Trp Ile Ile Ala Pro Leu Glu Tyr Glu Ala Phe His Cys Glu Gly Leu
            420              425              430

Cys Glu Phe Pro Leu Arg Ser His Leu Glu Pro Thr Asn His Ala Val
        435              440              445

Ile Gln Thr Leu Met Asn Ser Met Asp Pro Glu Ser Thr Pro Pro Thr
    450              455              460

Cys Cys Val Pro Thr Arg Leu Ser Pro Ile Ser Ile Leu Phe Ile Asp
465              470              475              480

Ser Ala Asn Asn Val Val Tyr Lys Gln Tyr Glu Asp Met Val Val Glu
            485              490              495

Ser Cys Gly Cys Arg
        500

<210> 4
<211> 131
<212> PRT
<213> Homo sapiens

<400> 4

```
Met Ala Arg Ser Leu Val Cys Leu Gly Val Ile Ile Leu Leu Ser Ala
1               5               10              15

Phe Ser Gly Pro Gly Val Arg Gly Gly Pro Met Pro Lys Leu Ala Asp
            20              25              30

Arg Lys Leu Cys Ala Asp Gln Glu Cys Ser His Pro Ile Ser Met Ala
        35              40              45

Val Ala Leu Gln Asp Tyr Met Ala Pro Asp Cys Arg Phe Leu Thr Ile
    50              55              60

His Arg Gly Gln Val Val Tyr Val Phe Ser Lys Leu Lys Gly Arg Gly
65              70              75              80

Arg Leu Phe Trp Gly Gly Ser Val Gln Gly Asp Tyr Tyr Gly Asp Leu
            85              90              95

Ala Ala Arg Leu Gly Tyr Phe Pro Ser Ser Ile Val Arg Glu Asp Gln
        100             105             110

Thr Leu Lys Pro Gly Lys Val Asp Val Lys Thr Asp Lys Trp Asp Phe
        115             120             125

Tyr Cys Gln
    130
```